# EUROPEAN PATENT SPECIFICATION

(11) **EP 1 631 677 B1**
(45) Date of publication and mention of the grant of the patent: **17.11.2010**
(21) Application number: 04776079.8
(22) Date of filing: 20.05.2004
(51) Int. Cl.: C12Q 1/68

(54) **SYSTEM FOR DETECTING POLYNUCLEOTIDES**
SYSTEM ZUM NACHWEIS VON POLYNUKLEOTIDEN
SYSTEME DE DETECTION DE POLYNUCLEOTIDES

(30) Priority: 20.05.2003 US 471827 P
(43) Date of publication of application: 08.03.2006
(73) Proprietor: Investigen, Inc., Hercules, California 94547 (US)
(72) Inventor: KOSHINSKY, Heather, El Cerrito, CA 94530 (US); ZWICK, Michael, S., Vacaville, CA 95688 (US); CHOI, K., Yeon, Alameda, CA 94501 (US)
(74) Representative: Hallybone, Huw George
(86) International application number: PCT/US2004/016118
(87) International publication number: WO 2005/017181

(56) References cited:
- WO-A-2004/074447
- US-A1- 2007 231 821
- US-B1- 6 355 421
- US-B1- 6 391 558
- US-B2- 6 280 946
- WILHELMSSON L MARCUS ET AL: "Genetic screening using the colour change of a PNA-DNA hybrid-binding cyanine dye." NUCLEIC ACIDS RESEARCH 15 JAN 2002, vol. 30, no. 2, 15 January 2002 (2002-01-15), page E3, XP002423595 ISSN: 1362-4962
- NORDEN B ET AL: "OPTICAL STUDIES ON COMPLEXES BETWEEN DNA AND PSEUDO ISO CYANINE" BIOPHYSICAL CHEMISTRY, vol. 6, no. 1, 1976, pages 31-45, XP002423597 ISSN: 0301-4622
- WANG MIAOMIAO ET AL: "Colorimetric detection of PNA-DNA hybridization using cyanine dyes." METHODS IN MOLECULAR BIOLOGY (CLIFTON, N.J.) 2002, vol. 208, 2002, pages 131-142, XP009080135 ISSN: 1064-3745
- KOMIYAMA MAKOTO ET AL: "PNA for one-base differentiating protection of DNA from nuclease and its use for SNPs detection." JOURNAL OF THE AMERICAN CHEMICAL SOCIETY, vol. 125, no. 13, 2 April 2003 (2003-04-02), pages 3758-3762, XP002423596 ISSN: 0002-7863
- ANIKOVSKY, M.Y., TATIKOLOV, A.S., SHVEDOVA, L.A., KUZMIN, V.A.: "Photochemical investigation of the triplet state of 3,3'-diethylthiacarbocyanine iodide in the presence of DNA" RUSSIAN CHEMICAL BULLETIN, INTL. EDITION, vol. 50, no. 7, July 2001 (2001-07), pages 119-110, XP009080179 ISSN: 1608-3148
- TOMLINSON ET AL: "A structural model for cyanine dyes templated into the minor groove of DNA" CHEMICAL PHYSICS, NORTH-HOLLAND, vol. 325, no. 1, 9 June 2006 (2006-06-09), pages 36-47, XP005479106 ISSN: 0301-0104
- SMITH J O ET AL: "Molecular recognition of PNA-containing hybrids: Spontaneous assembly of helical cyanine dye aggregates on PNA templates" JOURNAL OF THE AMERICAN CHEMICAL SOCIETY 31 MAR 1999 UNITED STATES, vol. 121, no. 12, 31 March 1999 (1999-03-31), pages 2686-2695, XP002423593 ISSN: 0002-7863

### SEQUENCE LISTING

<110> Investigen, Inc.
   Koshinsky, Heather
   Zwick, Michael S.
   Choi, K. Yeon
<120> SYSTEM FOR DETECTING POLYNUCLEOTIDES
<130> 429572000740
<150> 60/471,827
   <151> 2003-05-20
<160> 27
<170> FastSEQ for Windows Version 4.0
<210> 1
   <211> 19
   <212> DNA
   <213> bacteria
<220>
   <221> misc_feature
   <222> 19
   <223> Lysine is attached to g
<400> 1
   cactgctgcc tccccgtag 19
<210> 2
   <211> 18
   <212> DNA
   <213> bacteria
<400> 2
   ctacgggagg cagcagtg 18
<210> 3
   <211> 22
   <212> DNA
   <213> bacteria
<400> 3
   gaassmycya acacytagca ct 22
<210> 4
   <211> 24
   <212> DNA
   <213> bacteria
<400> 4
   tacaamgagy ygcwagacsg ygas 24
<210> 5
   <211> 21
   <212> DNA
   <213> gram positive bacteria
<400> 5
   gcagywaacg cattaagcac t 21
<210> 6
   <211> 20
   <212> DNA
   <213> gram positive bacteria
<400> 6
   acgacacgag ctgacgacaa 20
<210> 7
   <211> 23
   <212> DNA
   <213> gram negative bacteria
<400> 7
   tctagctggt ctgagaggat gac 23
<210> 8
   <211> 21
   <212> DNA
   <213> gram negative bacteria
<400> 8
   gagttagccg gtgcttcttc t 21
<210> 9
   <211> 21
   <212> DNA
   <213> fungi
<400> 9
   cctgcggctt aatttgactc a 21
<210> 10
   <211> 19
   <212> DNA
   <213> fungi
<400> 10
   tagcgacggg cggtgtgta 19
<210> 11
   <211> 13
   <212> DNA
   <213> cauliflower mosaic virus
<220>
   <221> misc_feature
   <222> 13
   <223> Lysine is attached to
<400> 11
   cccacccacg agg 13
<210> 12
   <211> 19
   <212> DNA
   <213> cauliflower mosaic virus
<400> 12
   gctcctacaa atgccatca 19
<210> 13
   <211> 20
   <212> DNA
   <213> cauliflower mosaic virus
<400> 13
   gatagtggga ttgtgcgtca 20
<210> 14
   <211> 19
   <212> DNA
   <213> cauliflower mosaic virus
<400> 14
   cccacccacg aggaacatc 19
<210> 15
   <211> 18
   <212> RNA
   <213> bacteria
<400> 15
   cuacgggagg cagcagug 18
<210> 16
   <211> 18
   <212> DNA
   <213> cauliflower mosaic virus
<400> 16
   gatagtggga ttgtgcgt 18
<210> 17
   <211> 15
   <212> DNA
   <213> cauliflower mosaic virus
<220>
   <221> misc_feature
   <222> 15
   <223> Lysine is attached to g
<400> 17
   tcttcttttt ccacg 15
<210> 18
   <211> 15
   <212> DNA
   <213> cauliflower mosaic virus
<220>
   <221> misc_feature
   <222> 15
   <223> Lysine is attached to g
<400> 18
   tcacatcaat ccact 15
<210> 19
   <211> 17
   <212> DNA
   <213> homo sapiens
<400> 19
   tgagtgtgtg gctttcg 17
<210> 20
   <211> 17
   <212> DNA
   <213> bacteria
<400> 20
   actgctgcct cccgtag 17
<210> 21
   <211> 12
   <212> DNA
   <213> bacteria
<400> 21
   tgcctcccgt ag 12
<210> 22
   <211> 11
   <212> DNA
   <213> bacteria
<400> 22
   tgcctcccgt a 11
<210> 23
   <211> 12
   <212> DNA
   <213> human immunodeficiency virus
<400> 23
   ctcattgatg gt 12
<210> 24
   <211> 12
   <212> DNA
   <213> hepatitis C virus
<400> 24
   cgcagaccac ta 12
<210> 25
   <211> 12
   <212> DNA
   <213> bacteria
<220>
   <221> misc_feature
   <222> 2,3,5,6,7
   <223> Modified
<400> 25
   tgcctcccgt ag 12
<210> 26
   <211> 12
   <212> DNA
   <213> bacteria
<220>
   <221> misc_feature
   <222> 3,5,7
   <223> Modified
<400> 26
   tgcctcccgt ag 12
<210> 27
   <211> 12
   <212> DNA
   <213> bacteria
<220>
   <221> misc_feature
   <222> 7
   <223> Modified
<400> 27
   tgcctcccgt ag 12

## Description

### BACKGROUND OF THE INVENTION

There is a great need to identify and quantify polynucleotides. Current methods of identifying target polynucleotides such as those associated with pathogens, pathogen infection, human genes associated with diseases and disorders, genetically modified organisms (GMOs), biowarfare agents, veterinary applications, and agricultural applications resently rely on methods such as the polymerase chain reaction (PCR), NASBA, TMA, or bDNA. The prior art includes document Wilhelmsson L.M. et al. (2002), Nucleic Acids Res. 30, E3, which teaches melting curve assays wherein the color of a cyanine dye changes upon heating in the presence of a DNA/PNA target-probe hybrid. These methods require skilled personnel and specialized equipment. Accordingly, there is a great need for convenient and economical methods of detection, identification, and quantification of target polynucleotides. This invention meets this and other needs.

### SUMMARY OF THE INVENTION

In one aspect, the present invention relates to methods for detecting the presence or amount of a target polynucleotide in a sample. In a further aspect, one method includes the following steps:

A nucleic acid analog that binds a target nucleic acid sequence of the target polynucleotide in a sequence specific manner, and a dye for which the rate of change in an optical property is different in the presence and absence of a target polynucleotide/nucleic acid analog hybrid, are combined to produce a mixture. The rate of change in the optical property of the dye in the mixture is compared to a reference value characteristic of the rate of change in the optical property of the dye in a similar mixture containing a known amount of a target polynucleotide/nucleic acid analog hybrid to determine a relative rate of change in the optical property. The relative rate of change in the optical property of dye in the mixture is correlated with the presence or amount of the specified target polynucleotide in a sample.

In another aspect, a method includes the following steps: peptide nucleic acid (PNA) that binds a target nucleic acid sequence of the target polynucleotide, in a sequence specific manner, and a cyanine dye for which the rate of change in an optical property selected from color, absorbance, and fluorescence when exposed to light stimulus, is different in the presence and absence of a target polynucleotide/PNA hybrid, are combined to produce a mixture. The rate of change in said optical property of the dye in the mixture is compared to a reference value characteristic of the rate of change in the optical property of the dye in a similar mixture containing a known amount of target polynucleotide/PNA hybrid to determine a relative rate of change in the optical property. The relative rate of change in the optical property of dye in the mixture is correlated with the presence or amount of the specified target polynucleotide in a sample.

In other aspects, the nucleic acid analog may be a locked nucleic acid (LNA), threose nucleic acid (TNA), or metal linked nucleic acid.

The rate of change in the optical property of the dye may be different in the presence and absence of a target polynucleotide/nucleic acid analog hybrid and when the mixture is provided with a light stimulus.

The sample may be a tissue, collection of cells, cell lysate, purified polynucleotide, or isolated polynucleotide, virus, environmental sample, industrial sample, medical sample, food sample, agricultural sample, veterinary sample, agro-livestock sample, water sample, soil sample, air sample, sample associated with bio-warfare agent, or sample associated with agro-warfare agent.

In one aspect, the target polynucleotide may be DNA. In some variations, the target polynucleotide may be obtained from total cellular DNA, nuclear DNA, mitochondrial DNA, ribosomal DNA, chloroplast DNA, or viral DNA, plasmid DNA, artificial DNA, epigenomic DNA, epigenetic DNA, in vitro amplified DNA, or chimeric DNA.

In another aspect, the target polynucleotide may be RNA. In some variations, the RNA may be obtained from ribosomal RNA (rRNA), messenger RNA (mRNA), transfer RNA (tRNA), armored RNA, viral RNA, , micro RNA, siRNA artifical RNA, or chimeric RNA.

The target polynucleotide may be obtained from an organism. In one embodiment, the organism may be a human. In another embodiment, the organism may be a pathogen. In a further embodiment, the target polynucleotide may be from a pathogen, such as a virus. bacterium, or fungus. Non-limiting examples of such viruses or bacteria include *Bacillus anthracis, Clostridium botulinum, Brucellae, Vibrio cholera, Clostridium perfringes,* Ebola virus, *Yersinia pesits, Coxiella burnetii,* Smallpox virus, hepatitis C virus, hepatitis B virus, and human immunodeficiency virus.

The nucleic acid analog may be partially complementary to the target nucleic acid sequence. Alternatively, the nucleic acid analog may be exactly complementary to the target nucleic acid sequence.

The nucleic acid analog may be greater than 4 nucleic acid bases in length and/or less than 24 nucleic acid bases in length. In a further variation, the nucleic acid analog may also be 12 nucleic acid bases in length.

The nucleic acid analog or target polynucleotide may be immobilized on a solid substrate. Immobilization may be via a non-covalent interaction, such as between biotin and streptavidin. In a further variation, the nucleic acid analog may be covalently linked to biotin. In still a further variation, non-covalent interaction may be an antigen/antibody interaction. The nucleic acid analog or target polynucleotide may also be covalently bonded to the solid substrate.

In a further aspect, the dye is a cyanine dye. Examples of cyanine dyes are a 3',3'-diethylthiacarbocyanine iodide, 3',3'-diethylthiadicarbocyanine iodide, and 3',3'-diethylthiatricarbocyanine iodide.

The dye may have a higher rate of change in the optical property in the presence of nucleic acid analog/target polynucleotide hybrid than in the absence of a nucleic acid analog/target polynucleotide hybrid.

The rate of change in the optical property of the dye may be determined by measuring an optical property of the dye. Examples of such optical properties include absorbance, fluorescence, reflectance, or chemiluminescence. The optical property may be determined at one or more times.

In another aspect, the optical property of the dye is measured at multiple times. In a further aspect, the optical property is measured at a single time.

In a further aspect, the invention is directed to a method of detecting an organism in a sample by detecting the presence or amount of a target polynucleotide in the sample wherein the presence or amount of the target polynucleotide identifies the presence or amount of the organism.

In a further aspect, the invention is directed to a method of detecting a class of organisms in a sample by detecting the presence or amount of a target polynucleotide in the sample wherein the presence or amount of the target polynucleotide identifies the presence or amount of the class of organism.

In another aspect, the invention is directed to a method of detecting a strain of an organism in a sample by detecting the presence or amount of a target polynucleotide in the sample, wherein the presence or amount of the target polynucleotide identifies the presence or amount of the strain.

In a further aspect, the invention is directed to a method of detecting a genetically modified organism (GMO) in a sample by detecting the presence or amount of a target polynucleotide in the sample, wherein the presence or amount of the target polynucleotide identifies the presence or amount of the genetically modified organism.

The present invention is also directed to a method of detecting the presence of a disease state in a subject by detecting the presence or amount of a target polynucleotide, wherein the presence or amount of the target polynucleotide identifies the disease state.

The present invention is also directed to a method of detecting the presence of genetic variation in a subject by detecting the presence or amount of a target polynucleotide, wherein the presence or amount of the target polynucleotide identifies the genetic variation.

In another aspect, the present invention is directed to detecting infection of a host by a pathogen, where the presence or amount of a target polynucleotide, wherein the target nucleic acid is a ribonucleic acid (RNA), and wherein the presence or amount of the target polynucleotide identifies infection of the host by the pathogen.

In another aspect, the invention is directed to a method of detecting a single nucleotide polymorphism (SNP) in a sample by detecting the presence or amount of a target polynucleotide in the sample, wherein the presence or amount of the target polynucleotide identifies the presence or amount of the SNP.

In another aspect, the invention is directed to a method of detecting a genetic sequence in a sample by detecting the presence or amount of a target polynucleotide in the sample, wherein the presence or amount of the target polynucleotide identifies the presence or amount of the genetic sequence.

In another aspect, the invention is directed to a method of detecting an in vitro amplified sequence in a sample by detecting the presence or amount of a target polynucleotide in the sample, wherein the presence or amount of the target polynucleotide identifies the presence or amount of the in vitro amplified sequence.

In another aspect, the invention is directed to a method of detecting base pair changes in a sample by detecting the presence or amount of a target polynucleotide in the sample, wherein the presence or amount of the target polynucleotide identifies the presence or amount of the base pair changes.

The invention is also directed to a method of detecting a target polynucleotide in two or more samples by detecting the target polynucleotide in a first sample at a first site of a multi-site device using a first nucleic acid analog molecule, and detecting the target polynucleotide in a second sample at a second site of a multi-site device using a second nucleic acid analog molecule. In one variation, the nucleic acid analog molecules are immobilized on a solid substrate. The method includes detecting two or more target polynucleotide sequences in two or more samples. Alternatively, the samples may be immobilized on a solid substrate.

The present invention is also directed to kits for detecting a target polynucleotide. The kit may include one or more of a sample that includes a target polynucleotide, one or more nucleic acid analogs at least partially complementary to a target nucleic acid sequence of the target polynucleotide, and one or more dyes. Optionally, the kit may include a source of stimulus. The kit may include instructions for using the kit.

### BRIEF DESCRIPTION OF THE DRAWINGS

Figure 1 depicts a schematic of a version of the assay system using a PNA.

Figure 2 shows the structural difference between a DNA molecule and a sample PNA molecule.

Figure 3 is a schematic representation of a light activated PNA-based assay system.

Figure 4 depicts a light activated assay reaction.

Figure 5 depicts the time course of 3,3'-diethylthiacarbocyanine iodide dye emission after exposure to PNA and light stimulus for different concentrations of target polynucleotide.

Figure 6 depicts the percent change in 3,3'-diethylthiacarbocyanine iodide dye emission for different DNA concentrations.

Figure 7 compares the percent change in 3,3'-diethylthiacarbocyanine iodide emission in a test sample in which different wavelengths of light stimulus were used for stimulation.

Figure 8 compares the percent change in dye emission in a sample of DNA from GMO soy and DNA from non-GMO soy.

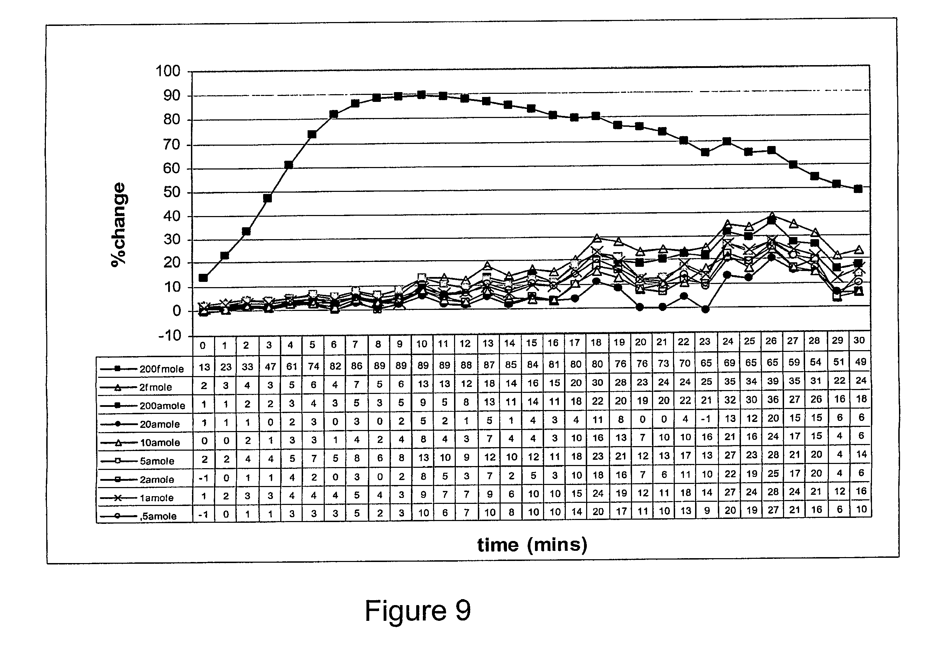

Figure 9 depicts assay sensitivity with varying test DNA concentrations using a mixed wavelength light source. Each line depicts the percent change in emission of the dye after exposure to a light stimulus at varying DNA concentrations. The PNA was N' CACTGCTGCCTCCCCGTAG-Lys. (SEQ ID NO: 1) The polynucleotide sequence was 5' CTACGGGAGGCAGCAGTG 3'. (SEQ ID NO: 2)

Figure 10 depicts the time at which a 20% change in 3,3'-diethylthiacarbocyanine iodide emission occurs at different target polynucleotide concentrations.

Figure 11 depicts the percent change in dye emission in a sample of DNA and samples with varying concentrations ofRNA.

Figure 12 compares the percent change in emission over time for 3,3'-diethylthiacarbocyanine iodide in a sample containing PNA and either DNA in the absence of a deli wash background or in the presence of deli wash background.

Figure 13 depicts the addition of PNA "wedges".

Figures 14A-D compares the emission after application of different wavelengths for a series of samples.

Figure 15 depicts immobilized reactions over time using a universal bacteria PNA probe.

Figure 16 depicts human SRY detection.

Figure 17 depicts a schematic representation of light activated, surface immobilized detection of nucleic acid analogs.

Figures 18A-D depict different schemes for introducing polynucleotide/nucleic acid analog hybrids.

Figure 19 depicts the effect of different wavelengths of light stimulus.

Figure 20 depicts the detection of different concentrations of soy DNA.

Figure 21 depicts the percent change in optical property versus the number of genomes of GMO positive soy.

Figure 22 depicts the effect of different concentrations of tween20.

Figure 23 depicts the percent change in emission for different concentrations of tween20.

Figure 24 compares a reaction using PNA probes to a reaction using LNA probes in a liquid format with different concentrations of tween20.

Figure 25 depicts the percent change in emission using PNAs versus LNAs.

Figure 26 depicts the detection of hepatitis C virus using different quantities of viral RNA.

Figure 27 depicts the emission of different copies of plasma one minute after exposure to light stimulus.

Figure 28 depicts the percent change in emission using bacteria and nucleic acid analogs that are either HCV or bacterial specific.

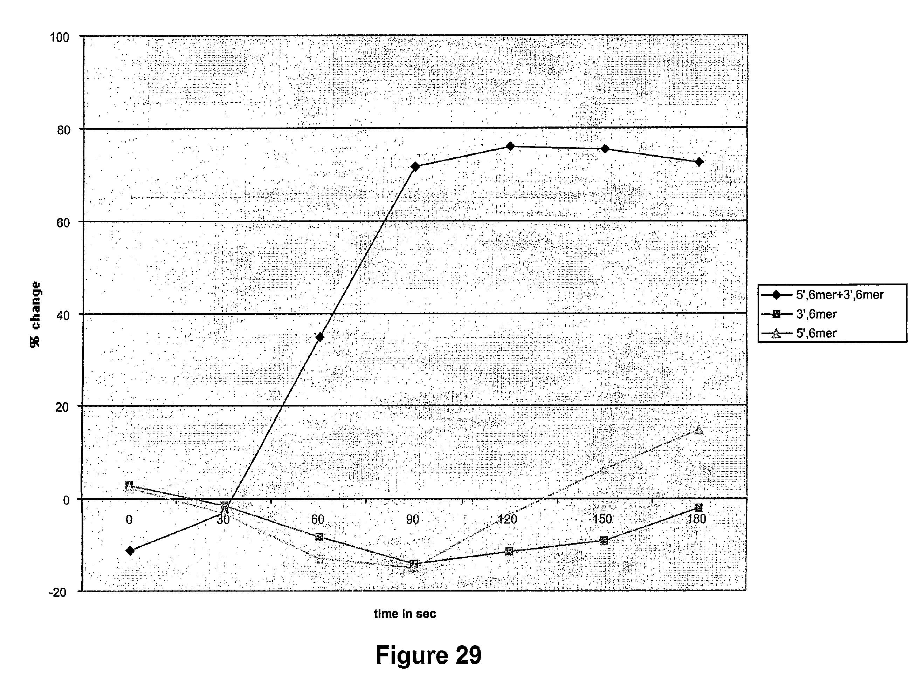

Figure 29 depicts the percent change in emission using short nucleic acid analogs or short nucleic acid analogs put together.

### DETAILED DESCRIPTION OF THE INVENTION

The present invention provides methods, compositions and assay systems for detecting a polynucleotide having a target nucleic acid sequence using nucleic acid analogs.

### I. General Techniques

The practice of the present invention employs, unless otherwise indicated, conventional techniques of molecular biology (including recombinant techniques), microbiology, cell biology, biochemistry, immunology, protein kinetics, and mass spectroscopy, which are within the skill of the art. Such techniques are explained fully in the literature, such as, Molecular Cloning: A Laboratory Manual, third edition (Sambrook et al., 2000) Cold Spring Harbor Press; Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al., eds., 1987 including supplements through May 1, 2004); PCR: The Polymerase Chain Reaction, (Mullis et al., eds., 1994); Current Protocols in Immunology (J.E. Coligan et al., eds., 1991); and Short Protocols in Molecular Biology (Wiley and Sons, 1999). Furthermore, procedures employing commercially available assay kits and reagents typically are used according to manufacturer-defined protocols unless otherwise noted.

### II. Definitions

The term "target nucleic acid sequence" generally refers to a nucleic acid sequence detected using the methods, compositions and assay systems of the invention. All or part of the target nucleic acid sequence may bind with a nucleic acid analog molecule by sequence specific hybridization. The target nucleic acid sequence may be of any length, but is typically less than 1 Kb in length, less than 500 bases in length, less than 24 bases in length, or less than 12 bases in length. In a further embodiment, the target nucleic acid sequence can be 10, 12, 14, or 18 bases in length. In other embodiments, the target nucleic acid can be at least 4, at least 5, at least 6, at least 7, at least 8, at least 9, at least 10, at least 12, at least 14, at least 15, at least 18, at least 20, at least 25 , at least 30, at least 35, at least 40, at least 45, or at least 50 bases in length. The target nucleic acid sequence of the invention may include protein coding sequence and/or non-coding sequences (e.g., regulatory sequences, introns, etc).

The term "polynucleotide" refers to a polymeric form of nucleotides of any length, either deoxyribonucleotides or ribonucleotides, or analogs thereof. The following are non-limiting examples of polynucleotides: a gene or gene fragment, exons, introns, messenger RNA (mRNA), transfer RNA, ribosomal RNA, ribozymes, cDNA, recombinant polynucleotides, branched polynucleotides, plasmids, vectors, nucleic acid probes, primers, and amplified DNA. A polynucleotide may contain modified nucleotides, such as methylated nucleotides and nucleotide analogs. The sequence of nucleotides may be interrupted by non-nucleotide components. A polynucleotide may be further modified before or after polymerization, such as by conjugation with a labeling component. The polynucleotide may be an amplified region of a longer polynucleotide.

The term "target polynucleotide" refers to a polynucleotide that includes a target nucleic acid sequence.

The term "nucleic acid analog" includes any nucleic acid analog having one or more bases that differ from guanine, thymidine, adenosine, cytosine, or uracil, and/or having one or more differences in a phosphoribose of an RNA backbone or phosphodeoxyribose of a DNA backbone. "Nucleic acid analogs" include, but are not limited to, peptide nucleic acids (PNAs), locked nucleic acids (LNA), such as disclosed in Trends in Biotechnology 21:74-81, 2003, metal-linked nucleic acids, modified polynucleotides such as anthraquinone-modified (as disclosed in Yamana et al, 2003), threose nucleic acids (TNAs) such as those disclosed in Chaput et al., Journal of the American Chemical Society, 125, 856 - 857, (2003), and chimeric nucleic acids.

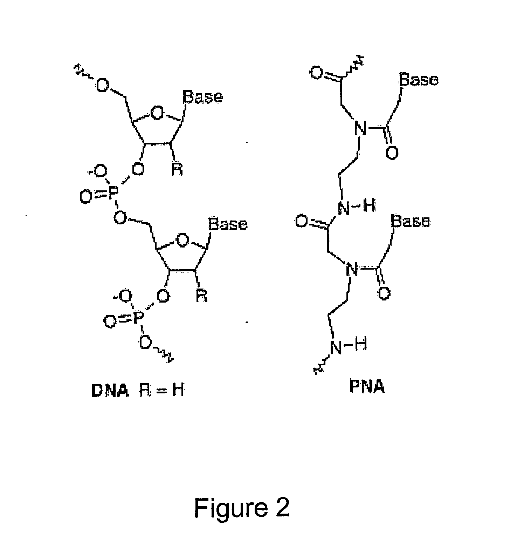

The term "peptide nucleic acid," or "PNA," includes any nucleic acid analog in which the deoxyribose phosphate backbone of a nucleic acid has been replaced by a synthetic peptide-like backbone, including, for example, n-(2-amino-ethyl)-glycine units, such as, without limitations, that depicted in Figure 2 (Nielsen et al., 1991), and those disclosed in U.S. Patent Nos. 5,786,461; 6,357,163; 6,107,470; 5,773,571; 6,441,130, 6,451,968; 6,228,982; 5,641,625; 5,766,855; 5,736,336; 5,719,262; 5,714,331; 5,719,262; and 6,414,112. The purine and pyrimidine bases may be attached by any covalent linkage, including, for example, methylene carbonyl linkages. As used herein, PNA molecules can have additional atoms between the PNA backbone and nucleobase. These analogs include, for example, D-lysine chains, cyclic structures such as cyclopentane or pyrrolidine rings, and/or chiral substitutents, including PNA molecules described in U. S. Patent No. 6,403,763, U.S. Patent Application No. US 2003/0162699, and U.S. Patent Application No. US 2003/0157500. The PNA backbone may include substitutions or extensions in the peptide backbone. PNAs may include peptide-based nucleic acid mimics (PENAMS), such as those disclosed, for example, in U.S. Patent No. 5,705,333, atoms having unusual chiral centers such as D-chiral centers and quasi-chiral centers, and atom substitutions in the PNA backbone.

The terms "nucleic acid analog/polynucleotide hybrid" and "polynucleotide/ nucleic acid analog hybrid" are synonymous and refer to a nucleic acid analog and polynucleotide hybridized in a sequence specific manner.

The terms "PNA/polynucleotide hybrid" and "polynucleotide/PNA hybrid" are synonymous and refer to a PNA and polynucleotide hybridized in a sequence specific manner.

By "complementary" it is meant that the single-stranded nucleic acid analog molecule has the ability to bind polynucleotides in a base-specific manner. The nucleic acid analog molecule may be synthesized to bind a target polynucleotide, such as a full-length polynucleotide strand or a part thereof. A nucleic acid analog molecule that is "complementary" may have one or more single base pair mismatches, additions, and/or deletions, but is still capable of binding the target polynucleotide under the selected hybridization conditions. In one embodiment, complementary sequences may hybridize through Watson-Crick (A-T or A-U and C-G). In a further embodiment, complementary sequences may hybridize through Hoogstein base pairing between the nucleic acid analog and polynucleotide nucleobases.

By "exactly complementary" it is meant that the single-stranded nucleic acid analog molecule has the ability to bind a target nucleic acid sequence and contains no mis-matches. A nucleic acid analog molecule is not exactly complementary to a target polynucleotide if there is a single base pair mismatch between the nucleic acid analog and the target polynucleotide.

The term "rate" refers to a change (e.g., of a property of a composition or compound). A rate may be described in terms of a specific rate constant. A rate may be determined by making measurements over a period of time. A rate may be described by making measurements, determined by measurements at two different time points in a process (e.g., before and after a specific stimulus, addition of a component, etc), or by making measurements at least three, at least four, or at least five, timepoints. A rate may be expressed in quantitative or qualitative terms (e.g. a change is "fast" or "slow"). A rate may be determined by comparing a property or compound to a reference value, or by other methods.

As used herein, the term "relative rate" refers to the rate of one process compared to the rate of another process. A "relative rate" may be approximate (e.g. the rate of one process may be "faster" or "slower" than the rate of another process) or qualitative (e.g. comparing measured rate constants of two processes).

As used herein, the term "dye" refers to a compound that has a measurable optical property or that may be converted to a compound with a measurable optical property. Measurable optical properties include, but are not limited to color, absorbance, fluorescence, reflectance, and chemiluminescence. The dye may exhibit the optical property under certain conditions, such as binding a polynucleotide/nucleic acid analog hybrid, or failing to bind a polynucleotide/nucleic acid analog hybrid.

"Armored RNA™" refers to an RNA that is ribonuclease resistant due to the encapsidation of the RNA by bacteriophage proteins. "Armored RNA™" is further described, for example, in U.S. Pat. Nos. 6,399,307; 6,214.982; 5,939,262; 5,919,625; and 5,677124.

"Non-specific carrier polynucleotide" as used herein refers to non-target polynucleotide molecules that increase the binding affinity of nucleic acid analogs with specific target polynucleotides and/or enhances the sensitivity of assay system.

As used herein, the term "nucleic acid analog binding site" refers to the point of attachment of one or more nucleic acid analog molecules to a solid support.

As used herein, the term "PNA binding site" refers to the point of attachment of one or more PNA molecules to a solid support.

"Sample" refers to a liquid sample of any type (e.g. blood, serum, water, or urine), and/or a solid sample of any type (e.g. cells, food, water, air, dirt, or grain), and/or an airborne sample of any type.

The term "subject" refers to an animal, such as a vertebrate, preferably a mammal, more preferably a human. The term "subject" also refers to a plant.

The term "pathogen" refers to any agent causing a disease, disorder and/or pathological condition and/or symptoms. By way of example, the pathogen may be an organism (or its associated toxin) found in nature, or created in a laboratory, that causes disease in or development of a pathological condition or symptom in, incapacitates, debilitates and/or kills an organism. Pathogens include, but are not limited to, virus, bacteria, fungi, eukaryotes, and/or prokaryotes, as well as biological weapons agents, infectious diseases, water borne pathogens, and food pathogens.

The term "biological weapons agent" refers to any organism (or its associated toxin) found in nature or created in the laboratory that is used for the primary purpose of causing disease in, incapacitating, or killing another living organism. Examples of biological weapons agents include, but are not limited to, pathogenic bacteria, fungi, protozoa, rickettsiae, and viruses.

As used herein, the term "infection" refers to the presence of a pathogen in a host. The infection may be dormant or virulent. In one embodiment, the presence of the pathogen is indicated by an alteration in host polynucleotide and/ or polypeptide expression. Infection may occur through such routes including, but not limited to, airborne droplets, direct contact, animal or insect vectors, and contaminated food or drink.

As used herein, the term "host response polynucleotide" refers to a polynucleotide that is altered, or a polynucleotide for which the expression is altered, in a host in response to a stimulus, such as infection, and/or contact by a pathogen.

The term "host" as used herein refers to animals and plants. The animal may be a mammal. Examples of mammals include humans, non-human primates, farm animals, sport animals, mice, and rats. Examples of plants include, but are not limited to, agricultural crops.

### III. Methods of Detecting Polynucleotides

The present application provides methods, compositions and assay systems for detecting a polynucleotide having a target nucleic acid sequence using nucleic acid analogs. In one embodiment, (i) a sample containing, believed to contain, or expected not to contain, a target polynucleotide, (ii) a nucleic acid analog that binds a target nucleic acid sequence of the polynucleotide in a sequence specific manner, and (iii) a dye for which the rate of change in an optical property is different in the presence and absence of a polynucleotide/nucleic acid analog hybrid, are combined to produce a mixture. The mixture may further include a non-specific carrier polynucleotide such as, but not limited to, non-specific plant DNA, yeast DNA, salmon sperm DNA or tRNA. The rate of change in the optical property of the dye in the mixture is compared to a reference value characteristic of the rate of change in the optical property of the dye in a similar mixture containing a known amount (including a zero amount) of a polynucleotide/nucleic acid analog hybrid to determine a relative rate of change in the optical property. The relative rate of change in the optical property of dye in the mixture is correlated with the presence or amount of the target polynucleotide in a sample to determine the presence or amount of target polynucleotide in the sample.

In one aspect, the present application provides methods, compositions and assay systems for detecting a polynucleotide having a target nucleic acid sequence using peptide nucleic acid (PNA) molecules. In one embodiment, (i) a sample containing, believed to contain, or expected not to contain, a target polynucleotide, (ii) a peptide nucleic acid (PNA) that binds a target nucleic acid sequence of the polynucleotide in a sequence specific manner, and (iii) a dye for which the rate of change in an optical property is different in the presence and absence of a polynucleotide/PNA hybrid, are combined to produce a mixture. The mixture may further include a non-specific carrier polynucleotide such as, but not limited to, non-specific plant DNA, yeast DNA, salmon sperm DNA or tRNA. The rate of change in the optical property of the dye in the mixture is compared to a reference value characteristic of the rate of change in the optical property of the dye in a similar mixture containing a known amount (including a zero amount) of a polynucleotide/PNA hybrid to determine a relative rate of change in the optical property. The relative rate of change in the optical property of dye in the mixture is correlated with the presence or amount of the specified polynucleotide in a sample to determine the presence or amount of target polynucleotide in the sample.

A reference value can be a value characteristic of a property of a composition or compound having a known characteristic. For example, in various embodiments, a reference value can be determined using a mixture that does not contain a polynucleotide/nucleic acid hybrid; contains some amount (e.g., a known amount) of a polynucleotide/nucleic acid hybrid; contains a zero amount of a polynucleotide/nucleic acid hybrid; or is a reaction mixture from which one or more components (e.g., a nucleic acid analog, a target polynucleotide, or a dye) has been omitted. Further nonlimiting examples of reference values include a value characteristic of an optical property of a mixture that has not been exposed to light stimulus, or, in an alternative embodiment, an optical property of a mixture that has been exposed to light stimulus. The aforementioned examples are for illustration and are not intended to limit the invention, and other examples will be apparent to the practitioner guided by this disclosure. It will be appreciated that a reference value may be, but need not necessarily be, empirically determined (for example, if it is known that the optical properties of composition containing a dye do not change, or change minimally, in the absence of target polynucleotide/nucleic acid analog hybrid, the reference value may be calculated or inferred and not measured). The reference value may be a constant. Although in some cases it may be convenient to assay a "control" sample concurrently with test samples, it is not necessary to do so. A reference value can be determined at one time point, and the value recorded to comparison at later time points. It will be understood that the aforementioned examples are for illustration and not limitation. A variety of reference values are described throughout the specification.

In one aspect, the reference value is characteristic of the rate of change in the optical property of the dye in a similar mixture containing no polynucleotide/nucleic acid analog hybrid. In one embodiment, the reference value may be characterized by the optical property of the dye prior to the combination of all the components in the mixture. In another embodiment, the reference value may be a standard value. For embodiments in which the mixture is exposed to light stimulus, the reference value may be characteristic of the optical property of the dye prior to applying light stimulus. It will be clearly understood that the reference value need not be determined simultaneously with the optical property of the dye in the sample, nucleic acid analog, and dye mixture. In addition it is understood that the reference value may a constant.

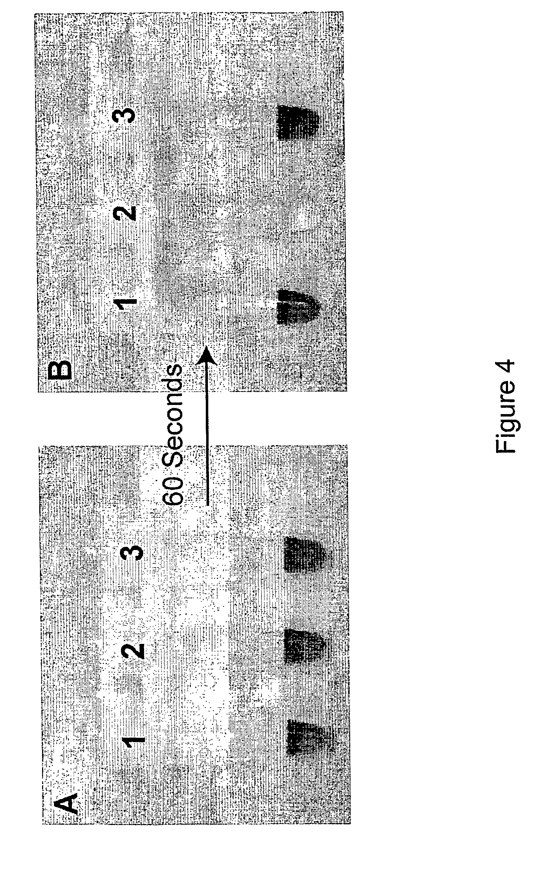

Reference to a specific example will assist in the understanding of the invention. A liquid-based version of this method using PNA is illustrated in Example 1 and Figure 4. 10 uM of a PNA molecule complementary to the cauliflower mosaic virus 35S promoter (35S PNA), and 1 uM 35S promoter DNA (35S DNA) were mixed in a microfuge tube and 150 uM dye was added. The tube was exposed to light stimulus and over the course of 1 minute the color change was observed (Figure 4); a change in the optical property of the dye. In the control tubes, the target polynucleotide was absent and the color remained pink even after 24 hours (without light stimulus), indicating a very slow rate of change in the optical property. Little change in an optical property (in this case color change) is observed in the tubes where the 35S PNA is either absent (Tube 3) or does not have a specific target (Tube 1).

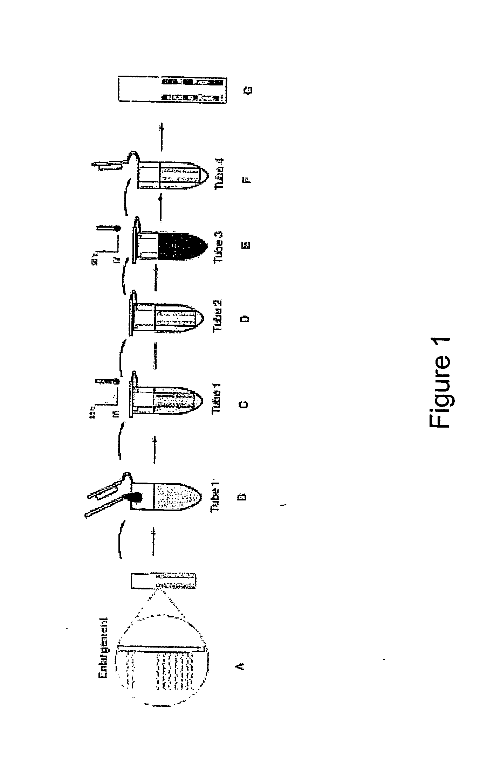

A further example using a PNA probe is illustrated in Figure 1. A) A membrane strip with discrete addresses of PNA sequences complementary to one or more target polynucleotide sequence is placed in the lysis/hybridization tube 1. B) A sample is added to the lysis/hybridization buffer and the mixture is heated to 95°> C for 3 minutes and C) cooled to room temperature. D) The strip is transferred to a fresh tube (tube 2), which contains washing buffer for incubation. Unbound DNA, RNA, and other cellular debris are removed. E) The strip is transferred to a fresh tube (tube 3) containing the detection dye and allowed to incubate for approximately 1 minute. F) The tube is briefly w ashed in washing buffer (tube 4) to remove residual unbound dye. G) In this membrane system the dye only binds to PNA/target polynucleotide complexes. Based on the pattern of hybridization the presence of specific target polynucleotides can be determined. By a comparison with a card of known patterns the presence and identity of the substance can be determined.

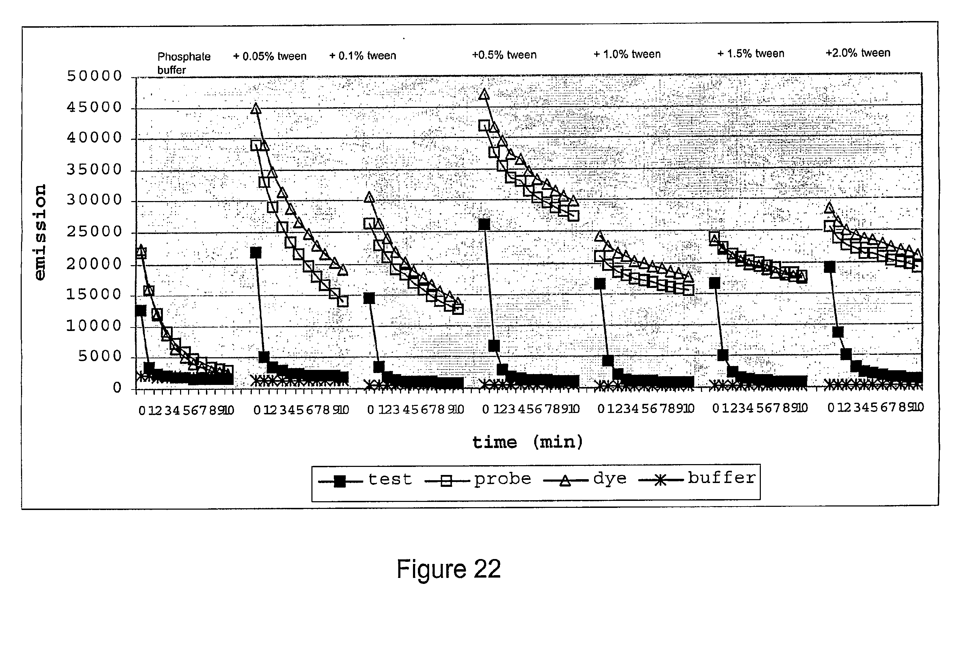

A further example is illustrated in Figure 22. 10 uM of a PNA molecule complementary to a target nucleic acid sequence and 1 uM target polynucleotide were mixed in a microfuge tube. 2 uM dye and various amounts of tween 20 were added. The tubes were exposed to light stimulus and over the course of 10 minutes the change in optical properties was observed (Figure 22). When greater than 1.0% tween20 is present, little change in the optical property is observed for samples containing dye and not the target polynucleotide/nucleic acid analog hybrid. When the sample containing the target polynucleotide/nucleic acid hybrid is exposed to light stimulus, the fluorescence (or other optical property) of the dye changes. The presence or amount of a target polynuleotide is detected by observing the change in the fluorescence (or other optical property). It will be appreciated that the presence or amount of target polynucleotide can be accomplished by a single measurement.

In a further embodiment, a plurality of nucleic acid analog sequence can be combined in the mixture to detect a plurality of target polynucleotides by multiplexing. Multiplex reactions involving known nucleic acid assay systems may be found at, for example, US patent 5,582,989.

Without intending to be bound to a particular mechanism or theory, in one aspect the nucleic acid analog/polynucleotide hybrid may catalyze a chemical reaction involving the dye. The dye binds to the minor groove of the nucleic acid analog/polynucleotide hybrids, which acts as a catalytic site. Application of a light stimulus adds energy to the mixture and causes a change in the optical property of the dye in the nucleic acid analog/polynucleotide hybrid at a faster rate than in the absence of a nucleic acid analog/polynucleotide hybrid. 3,3' diethylthiacarbocyanine iodide, for example, turns clear on application of light stimulus. The higher rate of change in an optical property of the dye corresponds to an increased presence of target polynucleotide in the sample.

The following sections describe aspects of the invention in further detail.

### A. Designing Nucleic Acid Analog Sequences

For use in the present invention, nucleic acid analogs may be designed to be complementary or exactly complementary to a nucleic acid sequence in a target polynucleotide. In one embodiment, the nucleic acid analog is greater than about 4 nucleotides in length and less than about 24 nucleic acid bases in length excluding linkers, amino acids and labels. In other embodiments, the nucleic acid analog may be from 5-100, 8-60, or 10-25 nucleic acid bases in length. In another embodiment, the nucleic acid analog may be about 8, 10, 12, 14, or 18 nucleic acid bases in length, excluding linkers, amino acids and labels. In other embodiments, the target nucleic acid can be at least 4, at least 5, at least 6, at least 7, at least 8, at least 9, at least 10, at least 12, at least 14, at least 15, at least 18, at least 20, at least 25 , at least 30, at least 35, at least 40, at least 45, or at least 50 bases in length. Nucleic acid analogs may be designed to have a portion that is non-complementary to the target polynucleotide, such as a sequence that hangs off the end of the polynucleotide.

The sequence of the nucleic acid analog molecules may be designed in a variety of ways. By way of example, and not limitation, the nucleic acid analog molecules may be designed to have sequences based on known primers used for PCR-based amplification and detection of specific target sequences. The nucleic acid analog molecule may also be designed to be complementary or exactly complementary to any target nucleic acid sequence of the polynucleotide. By way of example, the sequence of the nucleic acid analog molecule may be based on the sequence of PCR primers used to detect polynucleotides associated with pathogens, the presence of a pathogen in a host, a disease gene, or genetic condition. The nucleic acid analog molecule may also be complementary or exactly complementary to all or part of the sequence encoding the active or functional domains of a protein or the intact protein and or non-coding sequences (e.g., regulatory sequences, introns etc).

In one embodiment, nucleic acid analogs can be used to distinguish between polynucleotides having an exactly complementary sequence and one with a single base mismatch. For example and without limitation, nucleic acid analogs for use in the invention may be designed to detect single nucleotide polymorphisms (SNPs). Nucleic acid analog/polynucleotide hybridization is affected by base mismatches. According to the methods of the invention, upon the addition of a dye, a single base mismatch between a target sequence (e.g. SNP) and a nucleic acid analog results in a different rate of change in optical property of the dye compared to a nucleic acid analog that does not have the mismatch. The identification of SNPs for diagnosis and other methods is well known in the art.

In another embodiment, the nucleic acid analog may be designed to detect the presence or amount of a class of organisms. By class of organisms, it is meant that all organisms have one or more sequences that are complementary to, or exactly complementary to, a nucleic acid analog sequence. Such classes of organisms can be distinguished from other organisms based on the complementarity to nucleic acid sequences.

In an embodiment, the nucleic acid analog molecule has a purine content of less than about 60%, and have a maximum of 4 purine bases or three guanine bases in a row. Purine-rich nucleic acid analog molecules tend to aggregate and have low solubility in aqueous solutions. The nucleic acid analog molecules are selected to minimize or avoid self-complementary sequences with inverse repeats, hairpins and palindromes since these types of probes are prone to aggregate.

Nucleic acid analog molecules may hybridize to polynucleotides in either orientation, but an anti-parallel orientation is preferred. Anti-parallel is the preferred configuration for antisense and DNA probe-type applications. When the orientation of the nucleic acid analog is anti-parallel, the N-terminal of the nucleic acid analog probe is equivalent to the 5'-end of the DNA. Both N' and 5' are used herein.

One of skill in the art, guided by this disclosure, will recognize that in addition to nucleic acid analogs specifically listed herein, other nucleic acid analogs (including nucleic acid analogs discovered or developed in the future) may be used in the methods of the invention. Nucleic acid analogs that form a polynucleotide/nucleic acid analog hybrid under the assay conditions described herein are suitable for the present methods, and affect the rate of change in an optical property.

Nucleic acid analogs suitable for use in the methods can be identified using any of a variety of screening methods. In one method, for example and not limitation, a sample containing a candidate nucleic acid analog, optionally at varying concentrations, is combined with a polynucleotide having a complementary sequence under conditions under which an nucleic acid analog/polynucleotide hybrid is formed. In an embodiment, a dye is then added. The rate of change in optical property of the dye is then determined. This rate is compared to a reference value characteristic of the rate of change in optical property of the dye in the absence of a nucleic acid analog/polynucleotide hybrid. In a further embodiment, the reference value is characteristic of the absence of polynucleotide. In a still further embodiment, the reference value is characteristic of the presence of target polynucleotide (single stranded or double stranded). It will be recognized that the order of addition is not critical and components can be added in other orders.

In another embodiment, the reference value is characteristic of a non-zero concentration of polynucleotide. In this embodiment, nucleic acid analogs/polynucleotide hybrids result a different rate of change in optical property over time compared to a reference value (e.g., the presence of double stranded polynucleotide but absence of nucleic acid analog/polynucleotide hybrid) are selected for use in the claimed methods. The relative rate of change in the optical property is correlated with the presence or amount of specific polynucleotide.

### B. Peptide Nucleic Acids (PNAs)

In one aspect, the nucleic acid analogs are PNAs. The PNA hybridizes to all or part of a target nucleic acid sequence in a target polynucleotide by sequence specific hybridization. Alternatively, the conditions may be varied such that a single base pair change can be distinguished.

PNA molecules can hybridize rapidly to target polynucleotides. PNA hybridization to polynucleotides is independent of salt concentration (Demidov et al., 1994). PNAs are resistant to nuclease and protease attack, and bind to polynucleotides more specifically than conventional DNA probes. Short probes can be used with great sequence specificity (Ray and Norden, 2000). Furthermore, PNA/polynucleotide hybrids have higher thermal stability than the corresponding DNA/polynucleotide hybrids, and the melting point of PNA/polynucleotide hybrids is relatively insensitive to ionic strength, showing equal thermal stability under low (<10 mM NaCl) and moderate (500 mM NaCl) salt concentrations. This ability of PNA/polynucleotide hybrids to form under low salt conditions is significant because the internal structure of dsRNA and rRNA is significantly destabilized at salt concentrations below 200 mM. Therefore, assay conditions can be chosen that favor the disruption of the target nucleic acid while still promoting strong hybridization of PNA molecules (Stefano and Hyldig-Nielsen, 1997). PNA/polynucleotide hybridization is severely affected by base mismatches and PNA molecules can maintain sequence discrimination up to the level of a single mismatch.

PNA molecules may be purchased, for example, from Eurogentec (UK), Blue Heron Biotechnology (Bothell, WA), , and Applied Biosystems Inc. (ABI), or synthesized by methods known in the art.

### C. Hybridization Conditions

Generally, the design and/or choice of hybridization conditions is governed by several parameters, such as, but not limited to, the degree of complementarity of the nucleic acid analog molecule to the target polynucleotide, the length of the of the nucleic acid analog molecule to be utilized and the target polynucleotide itself. Preferred hybridization conditions allow for one or more of the following: efficient binding of nucleic acid analogs to target polynucleotides, minimization of RNA or DNA secondary structure, minimization of RNA degradation and either discrimination of one or more base pair changes or inclusion of one or more base pair changes.

Hybridization reactions can be performed under conditions of different "stringency". Conditions that effect stringency of a hybridization reaction are widely known and published in the art. See, for example, Molecular Cloning, A Laboratory Manual, third edition (Sambrook et al. 2000), Cold Spring Harbor Press. Examples of relevant conditions include but are not limited to, salt concentrations, pH (buffers) and temperature. Hybridization conditions utilizing lower salt concentrations generally enhance DNA instability and PNA/polynucleotide stability. Examples of buffers that may be used include, but are not limited to, Na3PO4, NaHSO4, K2HPO4, K2SO4, or CaSO4. By way of example, the molarity of the buffers may range between about 10mM and about 0.5 M and have a pH between about 4 to about 10, or between about 7 to about 10, such as about 7.0 or about 7.5. By way of example, Na3PO4 may be used at between about 0.5mM and about 0.5 M, such as for example, 2.5mM, and at a pH between about 4 to about 10 or between about 7 to about 10, such as about 7 or about 7.5.

Examples of sample conditions include but are not limited to (in order of increasing stringency): incubation temperatures of 25°C, 37°C, 50°C and 68°C; buffer concentrations of 10 X SSC, 6 X SSC, 4 X SSC, 1 X SSC, 0.1 X SSC (where SSC is 0.15 M NaCl and 15 mM of any buffer as described herein) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 X SSC, 1 X SSC, 0.1 X SSC, or deionized water. In a preferred embodiment hybridization and wash conditions are done at high stringency. By way of example hybridization may be performed at 50% formamide and 4 X SSC followed by washes of 2x SSC /formamide at 50°C and with 1 x SSC.

Buffers may contain ions or other compounds, or different buffering capacity. Alternatively a component in the buffer may have a stabilization capacity; such as neomycin or other aminoglycosides, that stabilizes triplex DNA, (Arya et al, 2003) or naphthalene diimides that enhance triplex stability (Gianolio and McLaughlin, 2001), or naphthylquinoline dimers (Keppler et al, 1999).

### D. Dyes

The presence or amount of a polynucleotide may be determined by using one or more dyes for which the rate of change in an optical property is different in the presence of a nucleic acid analog/polynucleotide hybrid compared to the a known concentration of nucleic acid analog/polynucleotide hybrid. In one aspect, the optical property may be a change in color, absorbance, fluorescence, reflectance, or chemiluminescence. The optical property may also be measured at a single or multiple times during an assay.

The rate of change in an optical property of the dye may be compared to a reference value characteristic of the rate of change in the optical property of the dye in a mixture containing a known amount of a nucleic acid analog/polynucleotide hybrid or polynucleotide/PNA hybrid to determine a relative rate of change in the optical property. The reference value may be a qualitative or approximate value (e.g. the presence or absence of color). Alternatively, the reference value may be a numerical value. As another example, the reference value may be measured or determined before, during, or after the determination of the rate of change in the optical property of the dye for the sample. In a further example, the reference value may be a constant, such as a rate constant. The reference value may also be the rate of change in the optical property of dye in the absence of a target polynucleotide or polynucleotide/nucleic acid hybrids (i.e. the known amount of a nucleic acid analog/polynucleotide hybrid is zero).

In some cases, the dye has a higher rate of change in the optical property in the presence of nucleic acid analog/polynucleotide hybrid than a reference value characteristic of the absence of a nucleic acid analog/polynucleotide hybrid. The presence or amount of the polynucleotide can thus be detected by an increase in the relative rate of change in the optical property compared to a reference value. Examples of dyes in which an optical property changes more rapidly in the presence of a nucleic acid analog/DNA hybrid include carbocyanine dyes, which are multi-ring aromatic compounds. These dyes absorb intensely in the visible range and bind preferentially to nucleic acid analog/polynucleotide hybrids in solution, changing color upon binding. Examples of cyanine dyes include but are not limited to 3,3'-diethylthiacyanine iodide, 3,3' diethylthiacarbocyanine iodide, 3,3' diethylthiadicarbocyanine iodide, and 3,3' diethylthiatricarbocyanine iodide.

In one exemplary embodiment, under assay conditions described herein, 3,3' diethylthiacarbocyanine turns from hot pink to clear with light stimulus in the presence of target nucleic acids and complementary PNA. In the absence of target nucleic acids the rate of change of the dye is slower. Light stimulus also decreases the fluorescent emission of the dye in the presence of target nucleic acids and complementary PNA. In the absence of light stimulus the dye immediately turns from hot pink to dull pink in the presence of target nucleic acids, and the fluorescent emission of the dye immediately decreases in the presence of target nucleic acids.

Under assay conditions described herein, 3,3'-diethylthiadicarbocyanine turns from blue to purple in the presence of target nucleic acids.

Under assay conditions described herein, 3,3-diethylthiatricarbocyanine remains aqua blue in the presence of target nucleic acids and turns clear in the absence of target nucleic acids.

In other cases, the dye has a lower rate of change in the optical property in the presence of nucleic acid analog/polynucleotide hybrid than in the absence of a nucleic acid analog/polynucleotide hybrid. The presence or amount of the polynucleotide can thus be detected by a decrease in the relative rate of change in the optical property compared to a reference value characteristic of the rate in the absence of a nucleic acid analog/polynucleotide hybrid.

Dyes may be selected from, for example, molecules that associate with nucleic acids in any of a variety of ways. Useful dyes include minor groove binders, major groove binders, intercalators and other polynucleotide-binding molecules, derivatives thereof, and conjugates thereto. Some dyes useful in the methods of the invention bind the minor groove of nucleic acid analog/polynucleotide hybrids. These dyes include carbocyanine dyes, such as 3,3' diethylthiacarbocyanine iodide, 3,3' diethylthiadicarbocyanine iodide, and 3,3' diethylthiatricarbocyanine iodide. Examples include, but are not limited to, ethidium bromide (Fiebig et al, 1999), flourescein, phenothiazine dyes, such as methylene blue (Wagner, 2002), DAPI (Kapuscinski, 1995), thiazole orange (Boger and Tse, 2001, Carreon et al, 2004), Hoechst 33258 (Adhikary et al, 2003, Maiti et al, 2003, Morozkin et al, 2003, Tanious et al., 2004, Tawar et al, 2003), SYBR Green II (Morozkin et al., 2003), BEBO, BETO, BOXTO, BO, BO-PRO, TO-PRO, YO-PRO (Karlsson et al., 2003, Eriksson et al, 2003), PicoGreen (Tolu and Myers, 2003), TO-PRO-3 (Sovenyhazy et al, 2003), biscyanine dye (Schaberle et al, 2003), methyl green-pyronin Y (Prento and Lyon, 2003), ethidium bromide and acridine orange (Johnson et al, 2003, Lauretti et al, 2003, Luedtke et al, 2003), neutral red (Wang et al, 2003), BO (Karlsson et al, 2003), mono- and bis-lexitropsins and pentamidine (Puckowska et al, 2004), 2-(methylthio)phenylsalicylaldimine Schiff base copper (II) (Reddy et al, 2004), a bifunctional platinum (II) complex (Ma and Che, 2003), bis(9-aminoacridine-4-carboxamides) (Wakelin et al, 2003), bisimidazoacridones (Tarasov et al, 2003), parallel or anitparallel carboxamide minor groove binders (Boutorine et al, 2003), conjugates of oligo(2'-O-methylribonucleotides) with minor groove binders (Novopashina et al, 2003), pyrroleimidazole polyamines (Briehn et al, 2003, Dervan and Edelson, 2003, Reddy et al, 2003, Renneberg and Dervan, 2003), pyrrole (Huang et al, 2000), bispyrrole (Carrasco et al., 2003), ruthenium complex (Kuwabara et al, 2003), thallium (Ouameur et al, 2003), aromatic diamidine (Nguyen et al, 2002), chartreusin (Barcelo et al, 2002), platinum complex (Silverman et al, 2002), S-3-nitro-2-pyridinesulfenyl-N-acetyl-cysteine (Shim et al, 2004), methylsulfonate esters (Varadarajan et al, 2003), peptide bis-intercalator (Guelev et al, 2001), metallointercalators (Proudfoot et al, 2001), 2,2'-binaphthalene (Kondo et al, 2004), intercalating nucleic acid (Christensen and Pedersen, 2002, Nielsen et al, 2004), ruthenium (II) complex (Liu et al, 2004), cyclic polyamine neotrien/copper (II) complex (Biver et al, 2004), 2,6-disulfonic acid anthraquinone (Wong and Gooding, 2003), ferrocenyl anthracene, ferrocenyl, and other naphthalene diimdie derivatives (Gianolio and McLaughlin, 2001, Takenaka et al, 2002, Tok and Fenker, 2001), doxorubicin (Patolsky et al, 2002, Xiao et al, 2003), acridin-9-ylthiourea (Baruah and Bierbach, 2003), naphthalene diimide (Nojima et al, 2001, Nunez et al, 2000), mitoxantrone (Wang et al, 2003), cryptolepine and neocryptolepine (Guittat et al, 2003), iminodiacetic acid-linked polyamides (Woods et al, 2002), dendritic polyamine conjugates (Brana et al, 2002), bis-intercalator delta-delta [mu-C49cpdppz)(2)-(phen)(4)Ru(2)] (Onfelt et al , 2001, Onfelt et al, 2002), ditercalinium (Berge et al, 2002), 8-methoxypsoralen (Arabzadeh et al, 2002), daunomycin and ellipticine (Reha et al, 2002), 1,4,5,8-naphthalene tetracarboxylic diimide (Guelev et al, 2002), cryptolepine (Lisgarten et al, 2002), AMAC (Ferry et al, 2001), (-)-6-[[(aminoalkyl)oxy]methyl]-4-demethoxy-6,7-dideoxydaunomcinones(1) (Dienes and Vogel, 1996), NLCQ-1 (Papadopoulou et al, 2000), YOYO-1 (Wong et al, 2001), DACA (Hicks et al, 2001), cyclometalated Rh(III)(Kisko and Barton, 2000), CI-958 (Dees et al, 2000), pyrazoloacridine (Pelley et al, 2000), cis-dichloroplatinum (II) complexes (Perrin et al, 2000), imidazoacridinones (Mazerski and Muchewicz, 2000), carbazole (Sajewicz and Dlugosz, 2000), 5,11-dimethyl-5H-indole[2,3-b]quinoline (Osiadacz et al, 2000), YOYO-3, netropsin, SN6999, A3 and SN6113 (Kirschstein et al, 2000), oxazole yellow (Inoue et al, 1999), 5,6-chrysenequinone diimine complexes of rhodium(III) (Jackson and Barton, 2000), Nile blue (Chen et al, 1999), usambarensine (Dassonneville et al, 1999), 3-methosybenzanthrone (Yang et al, 1999), 1,8-dihydroxyanthraquinones (Mueller and Stopper, 1999), cyclopropapyrroloindole (Dempcy et al, 1999), anthracene (Ostaszewski et al, 1998), pyrrolizines and imidazoles (Atwell et al, 1998), anthracycline complexes (Milano et al, 1998), heterodimers such athizole orange - thiazole blue, thiazole orange - ethidium and flourescein ethidium (Benson et al, 1993a, Benson et al 1993b). In addition companies (e.g. Molecular Probes) sell many types of nucleic acid stains that may be compatible with the system. Other classes of cyanine dyes and state reactive dyes may be found in Journal of the American Chemical Society 125:4132-4145 (2003) and Bioconjugate Chemistry 13:387-391 (2002). Examples of additional dyes include, but are not limited to, ternary copper(II) complexes (Dhar et al, 2003), distamycin A (Hiraku et al, 2002, Woods et al, 2002), indolo[2,3-b]-quinolizinium bromide (Viola, 2002), ecteinascidins (Anthoney and Twelves, 2001), metal ammines (Barry et al, 2002), or 2-phenylquinoline-carbohydrate hybrids (Toshima et al, 1999). In some embodiments, the dye is not a compound that promotes cleavage.

Dyes may also include malachite green, red biarsenical dye and flourescein. In some embodiments, the dyes is not malachite green, red biarsenical dye or flourescein.

One of skill in the art will recognize that dyes may be screened to identify those dyes that may be used in the present methods. Dyes that bind nucleic acid analog/polynucleotide hybrids and exhibit a change in an optical property over time, optionally after being provided with a stimulus, may be readily identified and selected.

One of skill in the art, guided by this disclosure, will recognize that in addition to the dyes listed herein, other dyes may be used in the methods of the invention. Dyes for which the rate of change in an optical property is different in the presence and absence of a target polynucleotide/nucleic acid analog hybrid under the assay conditions described herein are suitable for the present methods.

Suitable dyes can be identified using any of a variety of screening methods. For example and not limitation, a sample containing a nucleic acid analog is combined with a polynucleotide having a complementary sequence under conditions under which an nucleic acid analog/polynucleotide hybrid is formed. In an embodiment, the candidate dye is then added, optionally at varying concentrations. The order of addition is not critical and components can be added in other orders. The rate of change in optical property over time is then determined. This rate is compared to a reference value characteristic of the rate of change in optical property of the dye in the absence of a nucleic acid analog/polynucleotide hybrid. In one embodiment, the reference value is characteristic of the absence of polynucleotide. In one embodiment, the reference value is characteristic of the presence of polynucleotide (single stranded or double stranded). In another embodiment, the reference value is characteristic of a non-zero concentration of polynucleotide. Dyes that exhibit a different rate of change in optical property over time compared to a reference value are selected for use in the claimed methods. The relative rate of change in the optical property is correlated with the presence or amount of specific polynucleotide.

### E. Light Stimulus

Light stimulus can be provided to a sample, nucleic acid analog, and dye mixture either concurrently with the production of the mixture, or at a specified time after the production of the mixture. The light stimulus causes a change in the rate of change in an optical property of the dye.

The light stimulus may be in the visible spectrum or outside the visible spectrum. The light stimulus may be white light of a number of wavelengths. Alternatively, the light stimulus may be a specific wavelength, or range of wavelengths. The light stimulus may also have a specific intensity.

Light sources are known in the art. Different light sources result in different reaction rates because of differences in intensity or wavelength of the light sources. Examples of light sources, in ascending order of reaction rate, include Sylvania Cool White T8-CW, General Electric T8 - C50, and Fritz Aurora 50/50. Other light sources include a Sylvania dulux S9W CF9DS/blue and a Osram F9TT/50K, which both result in faster light stimulated reaction rates than the General Electric T8 - C50.

Those of skill in the art will recognize the optimal light stimulus may be determined without undue experimentation for a specific dye, or a specific nucleic acid analog, polynucleotide, and dye mixture. A single set of temperature and concentration conditions can be tested for a specific mixture.

### IV. Forming a Target Polynucleotide/Nucleic Acid Analog Hybrid

Assays for detection of target polynucleotides can be carried out using a variety of hybridization schemes. In one format, the polynucleotide sequence may be identified by hybridization of a target polynucleotide directly to a nucleic acid analog to form a target polynucleotide/nucleic acid analog hybrid.

In one format, the nucleic acid analog may be PNA. PNA/PNA has a distance that is different from the DNA/DNA distance and from the PNA/DNA distance. The detailed structure of PNA/PNA and PNA/DNA duplex hybrids have been solved by NMR and X-ray crystallography. A PNA/PNA duplex hybrid has a very wide and deep major groove and a very narrow and shallow minor groove, and the duplex has a very large pitch of 18 base pairs per turn and a large pitch height (57.6 z). A canonical B-form helix seen for a DNA/DNA duplex hybrid has a pitch of 10 base pairs per turn and 34 Å of pitch height, whereas a PNA/DNA duplex hybrid has a pitch of 13 base pairs per turn and 42 Å of pitch height. Because base pairs in a PNA/DNA duplex hybrid possess a different geometry compared with DNA double helices, the strength of the stacking interaction of PNA/DNA duplex hybrids is expected to be different from that of DNA/DNA duplex hybrids. CD spectra of 10, 12, and 16 mer PNA/DNA duplex hybrids suggest different base configuration for these duplex hybrids.

Depending on the dye and its binding site on a nucleic analog/polynucleotide hybrid can affect whether the reaction proceeds. For example, dyes that bind the major or minor groove of a hybrid may require the hybrid to contain a certain number of base pairs in order to bind effectively. The minimum number of base pairs can be easily determined easily by those of ordinary skill in the art.

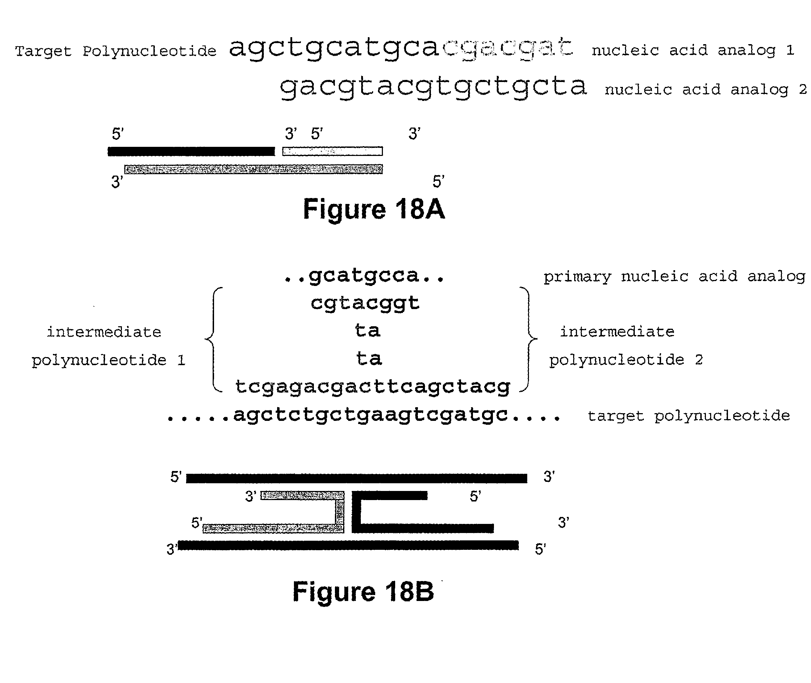

In one aspect, a target nucleic acid analog molecule is complementary to a partially complementary nucleic acid analog. The nucleic acid analog hybridizes to both a nucleic acid analog molecule and a target polynucleotide, as depicted in Figure 18A. This may be accomplished in a one step or multistep process. In a one step process, the target polynucleotide and nucleic acid analogs are combined in a single step. In a multistep process, the target polynucleotide and nucleic acid analogs are combined sequentially.

In a further aspect, the presence of a target polynucleotide may be detected by forming branched reaction crucifix form structure, an example of which is depicted in Figure 18B. In this format, a target polynucleotide is hybridized to two intermediate polynucleotides that are partially complementary to the target polynucleotide. The intermediate polynucleotides form a branched structure that hybridizes to a target polynucleotide and a primary nucleic acid analog molecule. The target hybridizing regions of the intermediate polynucleotides may be designed to be too short for a dye to bind the nucleic acid analog molecule separately, but large enough to bind the nucleic acid analog molecules when hybridized. The rate of optical change of a dye may be then determined. In one embodiment, the single nucleic acid analog molecule may be a universal nucleic acid analog that is used for all assays, and optimized for effective changes in the optical property of a dye. The universal nucleic acid analog could be used for any target nucleic acid, and the intermediate sequences could be varied. This scheme can be adapted to a format using an immobilized nucleic acid analog.

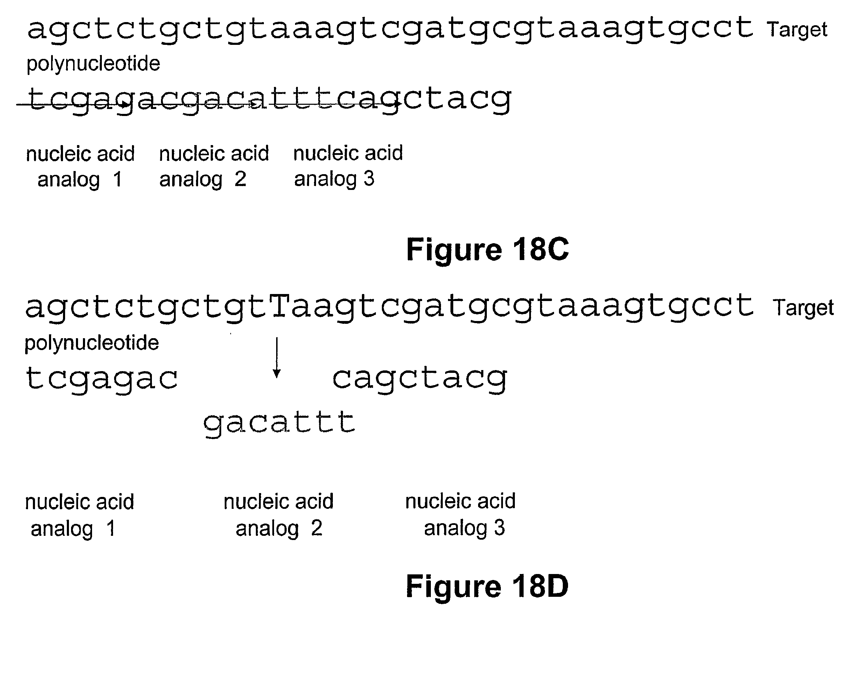

In another format, multiple nucleic acid analog molecules form a nucleic acid analog/polynucleotide hybrid with adjacent regions of a target polynucleotide. In this format, each nucleic acid analog molecule is too short for a dye to bind and result in a change in the rate of optical property of the dye, but multiple nucleic acid analog molecules may provide a large enough region for a rate of change in optical property to result. As depicted in Figure 18C, a target polynucleotide as a single molecule may be bound to form a nucleic acid analog/polynucleotide duplex by three separate nucleic acid analog molecules that hybridize to adjacent sequences. If the center nucleic acid analog molecule cannot hybridize as in Figure 18D, however, then a change in optical property may not be observed. In this format, one of the nucleic acid analog molecules may be immobilized.

### V. Quantifying the Amount of a Target Polynucleotide

The methods, compositions, and assay systems may be used to quantify the amount of target polynucleotide in a sample. In one embodiment, the amount of a target polynucleotide may be detected by establishing serial dilutions of the nucleic acid analog molecule, adding various amounts of the target polynucleotide samples, and comparing the samples to controls of known concentrations. In another embodiment, the amount of a target polynucleotide may be detected by establishing serial dilutions of the target polynucleotide, adding various amounts of the nucleic acid analog molecules, and comparing the samples to controls of known concentrations.

Alternatively, the amount of a target polynucleotide can be detected by measuring the kinetics of the assay based on time. Measurements of the dye in the combined mixture are taken at regular intervals after preparation of the mixture, or after application of light stimulus. The dye may be detected at distinct times after combination of the mixture, or after application of the light stimulus. The time may be any time period, for example the total time for the change in optical property, or the time required for the optical property to have changed by a certain percentage, such as, but not limited to, about 20%. The reactions can be frozen (further change stopped), for example with the addition of solvents such as 20% methanol, 15% isopropanol, 15% DMSO, or 10% butanol.

The reaction can also include a range of buffers and solvents. These buffers and solvents include phosphate buffers, water, 0.1% SDS, 0.1% Triton X, 0.1% Tween-20, 3% butanol, 10% methanol, 10% isopropanol, 10% DMSO, 1X blood lysis buffer (0.15M NH4Cl, 10mM NaHC03, 0.1mM EDTA pH 7.4), and sucrose lysis buffer (0.32M sucrose, 10mM Tris, 1% Triton X-100, 5mM MgCl2).

The quantity of polynucleotide in a sample may be determined after exposure to the light stimulus. The change in the optical property of the dye may be measured following pre-exposure to the light stimulus for the starting optical property. Measurements may be taken at distinct times (for example, taken at 30 second intervals) after exposure to the light stimulus. The reactions can be frozen (further change stopped) as described above.

Changes in the sample due to exposure to the light stimulus can be observed in several ways. The change in the optical property may be observed as a change in color, absorbance, fluorescence, reflectance, chemiluminescence, or a combination thereof. Alternatively, the change in optical property can be read using a reader. This change is measured using a spectrophotometer, such as Tecan Genios or a Tecan Safire. Specific wavelengths may be observed, for example by a filter. A positive control expresses a change in absorbance faster than a negative test. It can be measured as a difference in the rate of change, or the difference in the change at a set time. If a light stimulus is used and fluorescent properties are observed, the light stimulus provided to the sample is at a higher energy (lower wavelength) than the observed emission. The excitation may be at, for example, 535nm and the emission may be read at 590nm. The fluorescence may be measured as a difference in the rate of change the difference in the change at a set time.

In addition the mixture may have a change that occurs before exposure to the light stimulus. This difference may be observed in either a spectrophotometric system, a fluorescence emission system, or a chemiluminescent system.

### VI. ASSAY FORMATS

### A. Liquid-Based Assay System

As demonstrated in Examples 1-4, the methods and assay system for detecting a target polynucleotide may be liquid-based. The sample, a nucleic acid analog that binds a target nucleic acid sequence of the polynucleotide in a sequence specific manner, and a dye for which the rate of change in the optical property is different in the presence and absence of a nucleic acid analog/polynucleotide hybrid, are combined to produce a mixture in liquid solution. The rate of change in the optical property of the dye in the mixture is compared to a reference value characteristic of the rate of change in the optical property of the dye in a similar mixture containing a known amount of a nucleic acid analog/polynucleotide hybrid to determine a relative rate of change in the optical property. The relative rate of change in the optical property of dye in the mixture correlates to the presence or amount of the specified polynucleotide in a sample to determine the presence or amount of polynucleotide in the sample.

The method and assay system may also be prepared in any vessel, such as microfuge tubes, test tubes, and chips that hold a liquid by surface tension. The methods and assay system may also be prepared in multiwell plates. The plates may contain any number of wells. In one format, 96 well plates are used. In another format, 384 plates are used. When the assay format is in a microwell format, the liquid is retained in each well of a microtiter plate.

### B. Solid Support-Based Assay System

The methods and assay system may be solid based (e.g. one or more components are immobilized on a solid support). Most often, the nucleic acid analog or target polynucleotide is immobilized. There are many types of solid supports that the nucleic acid analog or target polynucleotide molecules may be attached to, including but not limited to: cast membranes (nitrocellulose, nylon), ceramic, track-etched membranes (TEM), polyvinylidenedifloride, latex, paramagnetic beads, plastic supports of all types, glass; powdered silica or alumina on a support matrix. If a grid pattern is used, the nucleic acid analog molecule/solid support forms a microarray. In another variation, the nucleic acid analog or target polynucleotide molecules may be covalently modified to include a linking moiety, such as a biotin or amide linkage, which binds to membranes. In a further variation, the nucleic acid analog or target polynucleotide molecules may be immobilized via sequence specific hybridization to one or more sequences.

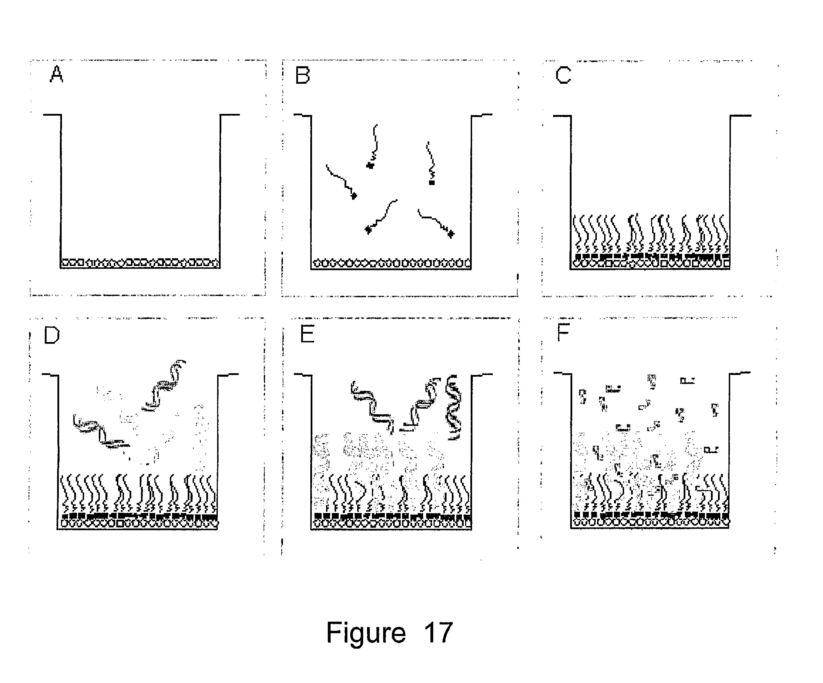

Figure 17 depicts a schematic representation of light stimulus activated, surface immobilized detection of nucleic acid analogs. A) Streptavidin well. B) Biotinylated nucleic acid analogs are added to the well and allowed to attach to the support. C) The well is washed and excess nucleic acid analogs are removed. D) Sample polynucleotide is added and specific sequence target attaches to the complementary nucleic acid analogs. E) Nonspecific and excess polynucleotide is washed off. F) Dye is added and exposed to the light stimulus.

Any means of attaching a nucleic acid analog molecule to a support is contemplated by the instant invention. In one aspect, the nucleic acid analog molecule may be attached directly to a membrane. The nucleic acid analog may be a PNA (e.g., A Giger, Lester A, Kleiber J, and Orum H, 1998, PNA array technology in molecular diagnostics. Nucleotides and Nucleoside, 17(1717-1724)). A solution of nucleic acid analog molecules (in water) is simply applied to a charged or chemically modified filter and allied to air dry. The filter is then used for hybridization.

In another aspect, a biotin labeled nucleic acid analog molecule may be attached to a strepavidin coated surface, such as a bead or well (see, e.g. Chandler, D.P., Stults, J.R., Anderson, K.K., Cebula, S., Schuck, B.L., and Brockman, F.J. 2000. Affinity Capture and Recovery of DNA at Femtomolar Concentrations with Peptide Nucleic Acid Probes. Analytical Biochem. 283:241-249). Biotin labeled nucleic acid analog molecules mixed with strepavidin labeled latex or polycarbonate beads. The Biotin binds strongly with strepavidin, allowing the nucleic acid analog molecule to bind to the bead in a unidirectional fashion. The beads are then applied to a non-charged membrane with a mesh size 25-30% greater that the diameter of the bead. Beads become trapped in the mesh, hence making a localized area of "attached nucleic acid analog molecules." Direct synthesis of nucleic acid analog molecules on a solid support such as a polypropylene membrane may be accomplished using standard 9-fluorenylmethoxycarboyl (Fmoc) protein synthesis chemistry (see, e.g., Matysiak S., Reuthner F., and Hoheisel JD. 2001 Automating parallel peptide synthesis for the production of nucleic acid analog library arrays, BioTechniques 31:896-904).

In another aspect, the nucleic acid analog molecules may be fixed to a glass or other solid support by applying a solution containing nucleic acid analog molecules in water directly to the glass or other support and letting it air dry.

In one variation, the nucleic acid analog molecule is designed to produce a net positive charge, and may bind a negatively charged membrane. For example, a positively charged lysine or glycine at a 5' or 3' end of the nucleic acid analog molecule may be used to attach the nucleic acid analog molecule to a negatively changed nylon membrane. The negatively charged membrane repels any nucleic acid that is not complementary and/or exactly complementary to the nucleic acid analog, thus minimizing non-specific binding.

Any target polynucleotide, or group of target polynucleotides, may be detected by the solid support-based system. In this case, a solid support contains multiple nucleic acid analog molecules immobilized on a solid support. A control nucleic acid analog that does not form nucleic acid analog/polynucleotide hybrids molecule does not hybridize, may be included on the solid support.

The solid support-based assay system may be used to detect at least one target polynucleotide. In other variations, the solid support-based system detects or measures the expression of at least about 8, at least about 10, at least about 20, at least about 30, at least about 40, at least about 50, or at least about 60 different target polynucleotides. In another variation, the solid support-based system detects and distinguishes expression of 60 or more target polynucleotides.

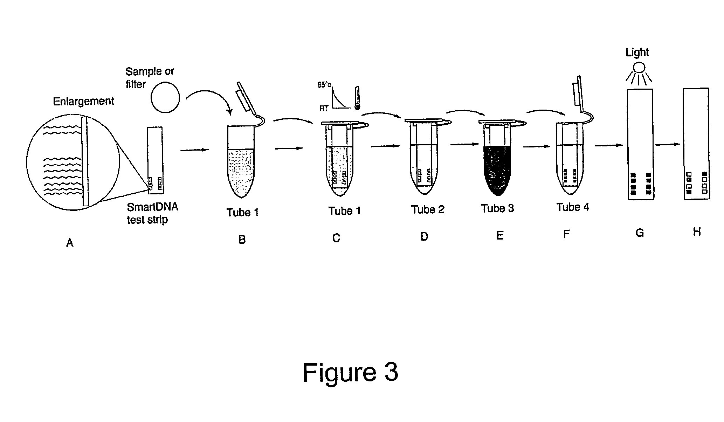

As an example, and not for limitation, a solid support-based version of this assay using a membrane as a solid support is depicted in Figure 3. The assay detects the presence of a target polynucleotide. The system allows small amounts of specific target polynucleotide to be identified and/or quantified within minutes, and gives a visual signal (e.g. rate of pink color changes to clear) without the need for extensive equipment. After very rapid sample lysis, hybridization, and introduction of the dye, a light stimulus is provided, and the rate of change of the colored pattern on the membrane is observed. The rate of change in the pattern of the colored bands on the membrane allows the user to easily determine the presence and identity of target polynucleotides. Optionally, the method or a variation thereof may be performed on a small battery operated hand-held device. In an automated robotic version all steps can be performed by a machine without human intervention.

The solid support based assay system may be in a microtiter plate arrangement. Any microtiter plate known in the art may be used. The assay components may remain liquid in such an assay system. In one format, 96 well plates are used. In another format, 384 plates are used. When the assay is in a microtiter format, all components except for those bound to the solid surface remain in solution in each well of a microtiter plate.

### VII. Target Polynucleotides and Sources of Target Polynucleotides

The target polynucleotide may be any polynucleotide, including naturally occurring, synthetic, and amplifying. Other types of polynucleotides may be single or double stranded. Non-limiting examples of target polynucleotides include DNA, RNA, regulatory RNA, mRNA, regulatory microRNA, siRNA, artificial RNA, and chimeric RNA. Other non-limiting examples of target polynucleotides include epigenomic DNA, epigenetic DNA, in vitro amplified DNA, and chimeric DNA. The target polynucleotide may contain SNPs that are identified or quantitated by the methods disclosed herein.

The methods, systems, and assays described herein have a variety of uses. Non-limiting examples of these uses include detecting and quantifying organisms, pathogens, such as foodborne pathogens, environmental pathogens, waterborne pathogens, or pathogens implicated in agroterrorism. Other non-limiting uses include disease diagnosis such as sexually transmitted disease diagnosis, detection of genes conferring anti-biotic resistance, detection of genes conferring a predisposition for drug responses, detection of genes implicated in an effective drug response, detection of genetically modified organisms, and detection of non-indigenous fluora or fauna. Additional non-limiting applications include agricultural applications and veterinary applications.

Examples, for illustration and not for limitation, are described below.

### A. Pathogens

In one aspect, the invention relates to methods, compositions and assay systems for detecting the presence of a pathogen and/or the infection of a host by a pathogen. Generally, the presence of a pathogen and/or the presence of a pathogen in a host is detected by analysis of target polynucleotides in a sample. More specifically, the invention relates to methods, compositions of matter and assay systems for analyzing target polynucleotides by sequence specific hybridization to a nucleic acid analog molecule and addition of the dye to form a mixture. The rate of dye change in optical property is then observed to detect the presence or quantity of the target polynucleotide.

Examples of pathogens or presence of the pathogen in a host that may be detected by the methods and assay systems includes, but is not limited to, Staphylococcus epidermidis, Escherichia coli, methicillin-resistant Staphylococcus aureus (MSRA), Staphylococcus aureus, Staphylococcus hominis, Enterococcus faecalis, Pseudomonas aeruginosa, Staphylococcus capitis, Staphylococcus wameri, Klebsiella pneumoniae, Haemophilus influnzae, Staphylococcus simulans, Streptococcus pneumoniae and Candida albicans.

Additional examples include, but are not limited to, Bacillus anthracis (Anthrax), Clostridium botulinum (Botulism), Brucellae (Brucellosis), Vibrio cholera (Cholera), Clostridium perfringens (gas gangrene, Clostridial myonecrosis, enteritis necroticans), Ebola virus (Ebola Hemorrhagic Fever), Yersinia pesits (Plague), Coxiella burnetii (Q Fever), and Smallpox virus (Smallpox). In a preferred variation, infection by Bacillus anthracis is detected.

Examples of host response polynucleotides for Bacillus anthracis include, but are not limited to, those disclosed by Mock M, Mignot T. Anthrax toxins and the host: a story of intimacy. Cell Microbiol. 2003 Jan;5(1): 15-23. and Reed DS, Smoll J, Gibbs P, Little SF. Related Articles, Links Mapping of antibody responses to the protective antigen of Bacillus anthracis by flow cytometric analysis. Cytometry. 2002 Sep 1;49(1):1-7, or are easily obtained by methods known in the art.

Pathogens may be distinguished from other pathogens based on their target polynucleotides. Specific pathogens have specific target polynucleotides that are not found in other pathogens. Nucleic acid analog molecules are designed to be complementary or exactly complementary to one or more target polynucleotides that identify a specific pathogen. When contacted with the target polynucleotides of the pathogen, the nucleic acid analog molecule hybridizes to the polynucleotides containing the target polynucleotide, but not to polynucleotides that do not contain the target polynucleotide. Specific pathogens that contain a target polynucleotide may thus be distinguished from pathogens that do not contain the target polynucleotide.

In addition, different strains of the same pathogen may be distinguished. Different strains of the same pathogen have different polynucleotide sequences. Frequently, the differences are as few as a single nucleotide. Nucleic acid analog molecules are designed to be complementary or exactly complementary to one or more target polynucleotides that identify a specific strain of pathogen. When contacted with the target polynucleotides of the pathogen, the nucleic acid analog molecule hybridizes to the polynucleotides containing the target polynucleotide, but not to polynucleotides that do not contain the target polynucleotide. When the target polynucleotide is specific to a specific pathogen strain, nucleic acid analog molecules hybridize to the target polynucleotides contained in the pathogen strain, and do not hybridize to target polynucleotides of the different strain.

Examples of pathogens of clinical importance and the references for the PCR-based detection are listed below. Nucleic acid analog molecules may be designed to have the sequence of specific pathogen PCR primer sequences, and can be used in combination in many of the embodiments of the invention described herein.

In one embodiment, the pathogen may be Staphylococcus epidermidis. Nucleic acid analogs may be designed to have sequences or fragments of sequences similar or identical to PCR primers used to identify Staphylococcus epidermidis. Examples of these PCR primers are disclosed in the art (see, e.g., Francois, P., D. Pittet, M. Bento, B. Pepey, P. Vaudaux, D. Lew, and J. Schrenzel. 2003. Rapid Detection of Methicillin-Resistant Staphylococcus aureus Directly from Sterile or Nonsterile Clinical Samples by a New Molecular Assay. J Clin Microbiol 41(1):254-60., Fitzpatrick, F., H. Humphreys, E. Smyth, C. A. Kennedy, and J. P. O'Gara. 2002. Environmental regulation of biofilm formation in intensive care unit isolates of Staphylococcus epidermidis. J Hosp Infect 52(3):212-8., and Yugueros, J., A. Temprano, B. Berzal, M. Sanchez, C. Hemanz, J. M. Luengo, and G. Naharro. 2000. Glyceraldehyde-3-phosphate dehydrogenase-encoding gene as a useful taxonomic tool for Staphylococcus spp. J Clin Microbiol 38(12):4351-5.

The pathogen may be Escherichia coli. Nucleic acid analogs may be designed to have sequences or fragments of sequences similar or identical to PCR primers used to identify Escherichia coli. Examples of these PCR primers are disclosed in the art (see, e.g., Heller, L. C., C. R. Davis, K. K. Peak, D. Wingfield, A. C. Cannons, P. T. Amuso, and J. Cattani. 2003. Comparison of Methods for DNA Isolation from Food Samples for Detection of Shiga Toxin-Producing Escherichia coli by Real-Time PCR. Appl Environ Microbiol 69(3):1844-6., Rahman, H. 2002. Multiplex PCR for detection of stx genes of Escherichia coli. Indian J Med Res 115:251-4., and Frahm, E., and U. Obst. 2003. Application of the fluorogenic probe technique (TaqMan PCR) to the detection of Enterococcus spp. and Escherichia coli in water samples. J Microbiol Methods 52(1):123-31).

The pathogen may be methicillin-resistant Staphylococcus aureus (MSRA). Nucleic acid analogs may be designed to have sequences or fragments of sequences similar or identical to PCR primers used to identify Staphylococcus aureus (MSRA). Examples of these PCR primers are disclosed in the art (see, e.g., (van Leeuwen, W. B., C. van Pelt, A. Luijendijk, H. A. Verbrugh, and W. H. Goessens. 1999. Rapid detection of methicillin resistance in Staphylococcus aureus isolates by the MRSA-screen latex agglutination test. J Clin Microbiol 37(9):3029-30., Francois, P., D. Pittet, M. Bento, B. Pepey, P. Vaudaux, D. Lew, and J. Schrenzel. 2003. Rapid Detection of Methicillin-Resistant Staphylococcus aureus Directly from Sterile or Nonsterile Clinical Samples by a New Molecular Assay. J Clin Microbiol 41(1):254-60, and Tsuruoka, M., and I. Karube. 2003. Rapid hybridization at high salt concentration and detection of bacterial DNA using fluorescence polarization. Comb Chem High Throughput Screen 6(3):225-34),

The pathogen may be Candida. Nucleic acid analogs may be designed to have sequences or fragments of sequences similar or identical to PCR primers used to identify Candida. Examples of these PCR primers are disclosed in the art (see, e.g., (Ahmad, S., Z. Khan, A. S. Mustafa, and Z. U. Khan. 2002. Seminested PCR for diagnosis of candidemia: comparison with culture, antigen detection, and biochemical methods for species identification. J Clin Microbiol 40(7):2483-9, and Tirodker, U. H., J. P. Nataro, S. Smith, L. LasCasas, and K. D. Fairchild. 2003. Detection of fungemia by polymerase chain reaction in critically ill neonates and children. J Perinatol 23(2):117-22),

The pathogen may be Candida albicans. Nucleic acid analogs may be designed to have sequences or fragments of sequences similar or identical to PCR primers used to identify Candida albicans. Examples of these PCR primers are disclosed in the art (see, e.g., White, P. L., A. Shetty, and R. A. Barnes. 2003. Detection of seven Candida species using the Light-Cycler system. J Med Microbiol 52(Pt 3):229-38., Grijalva, M., R. Horvath, M. Dendis, J. Emy, and J. Benedik. 2003. Molecular diagnosis of culture negative infective endocarditis: clinical validation in a group of surgically treated patients. Heart 89(3):263-8., and Willinger, B., A. Obradovic, B. Selitsch, J. Beck-Mannagetta, W. Buzina, H. Braun, P. Apfalter, A. M. Hirschl, A. Makristathis, and M. Rotter. 2003. Detection and identification of fungi from fungus balls of the maxillary sinus by molecular techniques. J Clin Microbiol 41(2):581-5.),

The pathogen may be Staphylococcus aureus. Nucleic acid analogs may be designed to have sequences or fragments of sequences similar or identical to PCR primers used to identify Staphylococcus aureus. Examples of these PCR primers are disclosed in the art (see, e.g., Francois, P., D. Pittet, M. Bento, B. Pepey, P. Vaudaux, D. Lew, and J. Schrenzel. 2003. Rapid Detection of Methicillin-Resistant Staphylococcus aureus Directly from Sterile or Nonsterile Clinical Samples by a New Molecular Assay. J Clin Microbiol 41(1):254-60., Palomares, C., M. J. Torres, A. Torres, J. Aznar, and J. C. Palomares. 2003. Rapid detection and identification of Staphylococcus aureus from blood culture specimens using real-time fluorescence PCR. Diagn Microbiol Infect Dis 45(3):183-9., and Xu, J., B. C. Millar, J. E. Moore, K. Murphy, H. Webb, A. J. Fox, M. Cafferkey, and M. J. Crowe. 2003. Employment of broad-range 16S rRNA PCR to detect aetiological agents of infection from clinical specimens in patients with acute meningitis-- rapid separation of 16S rRNA PCR amplicons without the need for cloning. J Appl Microbiol 94(2):197-206.),

The pathogen may be Staphylococcus hominis. Nucleic acid analogs may be designed to have sequences or fragments of sequences similar or identical to PCR primers used to identify Staphylococcus hominis. Examples of these PCR primers are disclosed in the art (see, e.g., Marsou, R., M. Bes, Y. Brun, M. Boudouma, L. Idrissi, H. Meugnier, J. Freney, and J. Etienne. 2001. Molecular techniques open up new vistas for typing of coagulase- negative staphylococci. Pathol Biol (Paris) 49(3):205-15., Yugueros, J., A. Temprano, B. Berzal, M. Sanchez, C. Hemanz, J. M. Luengo, and G. Naharro. 2000. Glyceraldehyde-3-phosphate dehydrogenase-encoding gene as a useful taxonomic tool for Staphylococcus spp. J Clin Microbiol 38(12):4351-5., and Wieser, M., and H. J. Busse. 2000. Rapid identification of Staphylococcus epidermidis. Int J Syst Evol Microbiol 50 Pt 3:1087-93.),

The pathogen may be Enterococcus faecalis. Nucleic acid analogs may be designed to have sequences or fragments of sequences similar or identical to PCR primers used to identify Enterococcus faecalis. Examples of these PCR primers are disclosed in the art (see, e.g., Hudson, C. R., P. J. Fedorka-Cray, M. C. Jackson-Hall, and L. M. Hiott. 2003. Anomalies in species identification of enterococci from veterinary sources using a commercial biochemical identification system. Lett Appl Microbiol 36(4):245-50., Donabedian, S. M., L. A. Thal, E. Hershberger, M. B. Perri, J. W. Chow, P. Bartlett, R. Jones, K. Joyce, S. Rossiter, K. Gay, J. Johnson, C. Mackinson, E. Debess, J. Madden, F. Angulo, and M. J. Zervos. 2003. Molecular characterization of gentamicin-resistant Enterococci in the United States: evidence of spread from animals to humans through food. J Clin Microbiol 41(3):1109-13., and Gauduchon, V., L. Chalabreysse, J. Etienne, M. Celard, Y. Benito, H. Lepidi, F. Thivolet-Bejui, and F. Vandenesch. 2003. Molecular diagnosis of infective endocarditis by PCR amplification and direct sequencing of DNA from valve tissue. J Clin Microbiol 41(2):763-6.).

The pathogen may be Pseudomonas aeriginosa. Nucleic acid analogs may be designed to have sequences or fragments of sequences similar or identical to PCR primers used to identify Pseudomonas aeriginosa. Examples of these PCR primers are disclosed in the art (see, e.g., Corbella, M. E., and A. Puyet. 2003. Real-Time Reverse Transcription-PCR Analysis of Expression of Halobenzoate and Salicylate Catabolism-Associated Operons in Two Strains of Pseudomonas aeruginosa. Appl Environ Microbiol 69(4):2269-75., Agarwal, G., A. Kapil, S. K. Kabra, R. Chandra, B. Das, and S. N. Diwedi. 2002. Phenotypic & genotypic variants of Pseudomonas aeruginosa isolated from children with cystic fibrosis in India. Indian J Med Res 116:73-81., and Dabrowski, W., U. Czekajlo-Kolodziej, D. Medrala, and S. Giedrys-Kalemba. 2003. Optimisation of AP-PCR fingerprinting discriminatory power for clinical isolates of Pseudomonas aeruginosa. FEMS Microbiol Lett 218(1):51-7.).

The pathogen may be Staphylococcus capitis. Nucleic acid analogs may be designed to have sequences or fragments of sequences similar or identical to PCR primers used to identify Staphylococcus capitis. Examples of these PCR primers are disclosed in the art (see, e.g., Wieser, M., and H. J. Busse. 2000. Rapid identification of Staphylococcus epidermidis. Int J Syst Evol Microbiol 50 Pt 3:1087-93.,and Martineau, F., F. J. Picard, P. H. Roy, M. Ouellette, and M. G. Bergeron. 1996. Species-specific and ubiquitous DNA-based assays for rapid identification of Staphylococcus epidermidis. J Clin Microbiol 34(12):2888-93.).

The pathogen may be Staphylococcus wameri. Nucleic acid analogs may be designed to have sequences or fragments of sequences similar or identical to PCR primers used to identify Staphylococcus warneri. Examples of these PCR primers are disclosed in the art (see, e.g., Wieser, M., and H. J. Busse. 2000. Rapid identification of Staphylococcus epidermidis. Int J Syst Evol Microbiol 50 Pt 3:1087-93., and Sashihara, T., H. Kimura, T. Higuchi, A. Adachi, H. Matsusaki, K. Sonomoto, and A. Ishizaki. 2000. A novel antibiotic, nukacin ISK-1, of Staphylococcus wameri ISK-1: cloning of the structural gene and identification of the structure. Biosci Biotechnol Biochem 64(11):2420-8.).

The pathogen may be Klebsiella pneumoniae. Nucleic acid analogs may be designed to have sequences or fragments of sequences similar or identical to PCR primers used to identify Klebsiella pneumoniae. Examples of these PCR primers are disclosed in the art (see, e.g., Perez-Perez, F. J., and N. D. Hanson. 2002. Detection of plasmid-mediated AmpC beta-lactamase genes in clinical isolates by using multiplex PCR. J Clin Microbiol 40(6):2153-62, Steward, C. D., J. K. Rasheed, S. K. Hubert, J. W. Biddle, P. M. Raney, G. J. Anderson, P. P. Williams, K. L. Brittain, A. Oliver, J. E. McGowan, Jr., and F. C. Tenover. 2001. Characterization of clinical isolates of Klebsiella pneumoniae from 19 laboratories using the National Committee for Clinical Laboratory Standards extended-spectrum beta-lactamase detection methods. J Clin Microbiol 39(8):2864-72., and Carter, J. S., and D. J. Kemp. 2000. A colorimetric detection system for Calymmatobacterium granulomatis. Sex Transm Infect 76(2):134-6.).

The pathogen may be Haemophilus influnzae. Nucleic acid analogs may be designed to have sequences or fragments of sequences similar or identical to PCR primers used to identify Haemophilus influnzae. Examples of these PCR primers are disclosed in the art (see, e.g., Shoma, S., M. Rahman, and M. Yasmin. 2001. Rapid detection of Haemophilus influenzae type b in Bangladeshi children with pneumonia and meningitis by PCR and analysis of antimicrobial resistance. J Health Popul Nutr 19(4):268-74., Corless, C. E., M. Guiver, R. Borrow, V. Edwards-Jones, A. J. Fox, and E. B. Kaczmarski. 2001. Simultaneous detection of Neisseria meningitidis, Haemophilus influenzae, and Streptococcus pneumoniae in suspected cases of meningitis and septicemia using real-time PCR. J Clin Microbiol 39(4):1553-8., and Carter, J. S., and D. J. Kemp. 2000. A colorimetric detection system for Calymmatobacterium granulomatis. Sex Transm Infect 76(2):134-6.).

The pathogen may be Staphylococcus simulans. Nucleic acid analogs may be designed to have sequences or fragments of sequences similar or identical to PCR primers used to identify Staphylococcus simulans. Examples of these PCR primers are disclosed in the art (see, e.g., Yugueros, J., A. Temprano, B. Berzal, M. Sanchez, C. Hemanz, J. M. Luengo, and G. Naharro. 2000. Glyceraldehyde-3-phosphate dehydrogenase-encoding gene as a useful taxonomic tool for Staphylococcus spp. J Clin Microbiol 38(12):4351-5, and Szweda, P., R. Pladzyk, R. Kotlowsky, and J. Kur. 2001. Cloning, expression, and purification of the Staphylococcus simulans lysostaphin using the intein-chitin-binding domain (CBD) system. Protein Expr Purif 22(3):467-71.).

The pathogen may be Streptococcus pneumoniae. Nucleic acid analogs may be designed to have sequences or fragments of sequences similar or identical to PCR primers used to identify Streptococcus pneumoniae. Examples of these PCR primers are disclosed in the art (see, e.g., Xu, J., B. C. Millar, J. E. Moore, K. Murphy, H. Webb, A. J. Fox, M. Cafferkey, and M. J. Crowe. 2003. Employment of broad-range 16S rRNA PCR to detect aetiological agents of infection from clinical specimens in patients with acute meningitis-- rapid separation of 16S rRNA PCR amplicons without the need for cloning. J Appl Microbiol 94(2):197-206., Greiner, O., P. J. Day, P. P. Bosshard, F. Imeri, M. Altwegg, and D. Nadal. 2001. Quantitative detection of Streptococcus pneumoniae in nasopharyngeal secretions by real-time PCR. J Clin Microbiol 39(9):3129-34., and Corless, C. E., M. Guiver, R. Borrow, V. Edwards-Jones, A. J. Fox, and E. B. Kaczmarski. 2001. Simultaneous detection of Neisseria meningitidis, Haemophilus influenzae, and Streptococcus pneumoniae in suspected cases of meningitis and septicemia using real-time PCR. J Clin Microbiol 39(4):1553-8.).

The pathogen may be Coronavirus, an RNA virus, for the detecting SARS. The sequence of coronavirus may be found, for example at the Center for Disease Control website. Examples of these target polynucleotides are disclosed in the art (see, e.g., Ksiazek, T. G., D. Erdman, C. S. Goldsmith, S. R. Zaki, T. Peret, S. Emery, S. Tong, C. Urbani, J. A. Corner, W. Lim, P. E. Rollin, S. F. Dowell, A. E. Ling, C. D. Humphrey, W. J. Shieh, J. Guamer, C. D. Paddock, P. Rota, B. Fields, J. DeRisi, J. Y. Yang, N. Cox, J. M. Hughes, J. W. LeDuc, W. J. Bellini, and L. J. Anderson. 2003. A Novel Coronavirus Associated with Severe Acute Respiratory Syndrome. N Engl J Med 16:16; Drosten, C., S. Gunther, W. Preiser, S. Van Der Werf, H. R. Brodt, S. Becker, H. Rabenau, M. Panning, L. Kolesnikova, R. A. Fouchier, A. Berger, A. M. Burguiere, J. Cinatl, M. Eickmann, N. Escriou, K. Grywna, S. Kramme, J. C. Manuguerra, S. Muller, V. Rickerts, M. Sturmer, S. Vieth, H. D. Klenk, A. D. Osterhaus, H. Schmitz, and H. W. Doerr. 2003. Identification of a Novel Coronavirus in Patients with Severe Acute Respiratory Syndrome. N Engl J Med 10:10).

The pathogen may be Bordetella pertussis, or whopping cough. Examples of these target polynucleotides are disclosed in the art (Doucet-Populaire, F., N. Bourgeois, O. Charara, M. Bellaiche, F. Richardin, J. L. Salomon, L. Berardi-Grassias, J. C. Ghnassia, and P. Foucaud. 2002. [Routine use of gene amplification for pertussis diagnosis in children]. Arch Pediatr 9(11):1145-52; Lingappa, J. R., W. Lawrence, S. West-Keefe, R. Gautom, and B. T. Cookson. 2002. Diagnosis of community-acquired pertussis infection: comparison of both culture and fluorescent-antibody assays with PCR detection using electrophoresis or dot blot hybridization. J Clin Microbiol 40(8):2908-12).

The pathogen may be Phytophthora ramorum (the cause of sudden oak death). Examples of these target polynucleotides are disclosed in the art (see, e.g., Levy, L., and V. Mavrodieva. 2003. Evaluation of the PCR Detection and DNA isolation methods for use in the Phytophthora ramorum. National Pilot Survey, vol. 2003, available at the Purdue University website; McPherson, B. A., D. M. Rizzo, M. Garbelotto, P. Svihra, D. L. Wood, A. J. Storer, N. M. Kelly, N. Palkovsky, S. A. Tjosvold, R. B. Standiford, and S. T. Koike. 2002. Sudden Oak Death in California, vol. 2003. Home & Landscape, available at the University of California at Davis website).

The pathogen may be norwalk virus. Examples of these target polynucleotides are disclosed in the art (see, e.g., Khan, A. S., C. L. Moe, R. I. Glass, S. S. Monroe, M. K. Estes, L. E. Chapman, X. Jiang, C. Humphrey, E. Pon, J. K. Iskander, and et al. 1994. Norwalk virus-associated gastroenteritis traced to ice consumption aboard a cruise ship in Hawaii: comparison and application of molecular method-based assays. J Clin Microbiol 32(2):318-22; Herwaldt, B. L., J. F. Lew, C. L. Moe, D. C. Lewis, C. D. Humphrey, S. S. Monroe, E. W. Pon, and R. 1. Glass. 1994. Characterization of a variant strain of Norwalk virus from a food-borne outbreak of gastroenteritis on a cruise ship in Hawaii. J Clin Microbiol 32(4):861-6).

The pathogen may be HIV. Nucleic acid analogs may be designed to have sequences or fragments of sequences similar or identical to PCR primers used to identify HIV. Examples of these PCR primers are disclosed in the art (see, e.g., Smith, K. M., K. A. Crandall, M. L. Kneissl, and B. A. Navia. 2000. PCR detection of host and HIV-1 sequences from archival brain tissue. J Neurovirol 6(2):164-71;Cuchacovich, R., S. Quinet, and A. M. Santos. 2003. Applications of polymerase chain reaction in rheumatology. Rheum Dis Clin North Am 29(1):1-20, v;Cunningham, P., D. Marriott, C. Harris, S. Hancock, A. Carr, and D. Cooper. 2003. False negative HIV-1 proviral DNA polymerase chain reaction in a patient with primary infection acquired in Thailand. J Clin Virol 26(2):163-9).

The pathogen may be Mycobacterium tuberculosis. Nucleic acid analogs may be designed to have sequences or fragments of sequences similar or identical to PCR primers used to identify tuberculosis. Examples of these PCR primers are disclosed in the art (see, e.g., Torres, M. J., A. Criado, M. Ruiz, A. C. Llanos, J. C. Palomares, and J. Aznar. 2003. Improved real-time PCR for rapid detection of rifampin and isoniazid resistance in Mycobacterium tuberculosis clinical isolates. Diagn Microbiol Infect Dis 45(3):207-12;Bhattacharya, B., K. Karak, A. G. Ghosal, A. Roy, S. Das, P. Dandapat, D. Khetawat, D. K. Mondal, S. Bhattacharya, and S. Chakrabarti. 2003. Development of a new sensitive and efficient multiplex polymerase chain reaction (PCR) for identification and differentiation of different mycobacterial species. Trop Med Int Health 8(2):150-7;Kafwabulula, M., K. Ahmed, T. Nagatake, J. Gotoh, S. Mitarai, K. Oizumi, and A. Zumla. 2002. Evaluation of PCR-based methods for the diagnosis of tuberculosis by identification of mycobacterial DNA in urine samples. Int J Tuberc Lung Dis 6(8):732-7).

In another embodiment, nucleic acid analogs are designed that bind to target polynucleotides common to an entire group of pathogens. For example, nucleic acid analogs may be designed to detect bacteria (BP6), gram positive bacteria (BP19), gram negative bacteria (BP3), and Fungi (FP8). Examples of the sequences of the nucleic acid analogs for BP6, BP19, BP3, and FP8 are shown below.

| | | |
|---|---|---|
| BP6 | oI2018 | 5'gaaSSMYcYaacacYtagcact (SEQ ID No: 3) |
| | oI2019 | 5'tacaaMgagYYgcWagacSgYgaS (SEQ ID NO: 4) |
| | | |
| BP19 | oI2021 | 5'gcagYWaacgcattaagcact (SEQ ID NO: 5) |
| | oI2022 | 5'acgacacgagctgacgacaa (SEQ ID NO: 6) |
| | | |
| BP3 | oI2003 | 5'tctagctggtctgagaggatgac (SEQ ID NO: 7) |
| | oI2004 | 5'gagttagccggtgcttcttct (SEQ ID NO: 8) |
| | | |
| FP8 | OI2055 | 5'cctgcggcttaatttgactca (SEQ ID NO: 9) |
| | OI2057 | 5'tagcgacgggcggtgtgta (SEQ ID NO: 10) |

The nucleic acid analogs can be used in clinical applications for the diagnosis of the microbial cause of sepsis or in other applications where the microbial content of products in important in evaluating their shelf-life and stability or other products where the sterility is being assessed.

Target polynucleotides may be specific to ribosomal RNA sequences, such as 16S RNA in E. coli. Ribosomal RNA contains specific sequences that are characteristic to their organism. By using nucleic acid analog sequences that are complementary or exactly complementary to a target polynucleotide characteristic of the ribosomal RNA sequence, pathogens may be identified based on their ribosomal RNA sequences. Ribosomal RNA sequences characteristic of different pathogens or strains of pathogens, may be found, for example, at (Dinshaw J. Patel, Asif K. Suri, Feng Jiang, Licong Jiang, Pei FanR. Ajay Kumar and Sylvie Nonin, Structure, Recognition and Adaptive Binding in RNA Aptamer Complexes J. Mol. Biol. (1997) 272, 645- 664).

### B. Host Response Polynucleotides

The target nucleotide may also be a host response polynucleotide. After a host is infected with a pathogen, host genes may be differentially expressed in response to the pathogen to produce a pattern of mRNA expression (gene expression profile). The host genes whose pattern of expression correlates with infection by a specific pathogen are referred to as "diagnostic marker genes" for that agent. The mRNA is typically derived from white blood cells in the sample. Methods of identifying a host response and altered gene expression profiles may be found, for example, in 1) Das, R., Mendis, C., Yan, Z., Neill, R., Boyle, T., and Jett, M. (1998) Alterations in gene expression show unique patterns in response to agents. In Proceeings of the 21st Army Science Conference, F-P9, and Mendis, C., Das, R., Yang, D., and Jett, M. (1998) Identification of alterations in gene expression in response to Staphylococcal enterotoxin B (SEB) using differential display (DD). In the ASCB 38th Annual Meeting, San Francisco, CA.

Methods for gene expression profiling have been developed to diagnose various medical conditions, as described, for example, in U.S. 5,723,290 to Eberwine et al., U.S. 6,218,122 to Friend et al., WO 99/10536 to Yarramilli et at., and WO 99/57130 to Prashar et al. Briefly, the method involves obtaining the level of mRNA expression in selected cells associated with a selected condition and comparing the relative levels obtained with established controls. A diagnosis is then made based upon the comparison of the mRNA expression levels. The mRNA is isolated from cells by any one of a variety of techniques well known in the art, but in general, lyse the cells and then enrich for or purify RNA. Gene expression profiles are then produced by any means known in the art, including, but not limited to the methods disclosed by: Prashar et al. in WO 97/05286; Liang et al. in Science 257:967-971 1 (1992); Ivanova et al. in Nucleic Acids Res. 23:2954-2959 (1995); and Prashar et al. in Proc. Natl. Acad. Sci. USA 93:659-663 (1996). These techniques typically produce gene expression profiles using northern analysis, FRET detection, hybridization to an oligonucleotide array, hybridization to a cDNA array, cDNA sequencing, cDNA fingerprinting, and the like. Oftentimes, the gene expression profile refers to a representation of the expression of mRNA species such as may be found in an autoradiograph of labeled cDNA fragments produced from total cellular mRNA separated on the basis of size by slab gel electrophoresis, capillary gel electrophoresis, HPLC, and other separation methods.

The assay systems and methods of this invention provide an alternative way to assess mRNA expression. As further described below, levels of mRNA expression (i.e., gene expression profiles) are compared, or alternatively alterations in mRNA expression from diagnostic marker genes are detected by assaying for hybridization of host response mRNAs to their complementary or exactly complementary nucleic acid analogs.

### C. Foodborne and Environmental Pathogens

Any foodborne or environmental pathogens may be detected by the present invention. Foodborne pathogens include, but are not limited to, Listeria, Campylobacter, E.coli and Salmonella. The nucleic acid analog methods disclosed herein readily allow the detection of specific foodborne pathogens. For example, Listeria may be readily distinguished from Salmonella. Further, specific strains of foodborne pathogens, such as specific strains of L. monocytogenes, may be detected.

Any target polynucleotide associated with foodborne pathogens may be identified by the methods of the present invention. Target polynucleotides that characterize specific pathogens and strains of pathogens may be identified, as discussed above. The method may be used to detect target polynucleotides associated with Listeria, Campylobacter, E.coli and Salmonella. For example, specific Listeria genes that may be identified by the methods disclosed herein include hly (listeriolysin O) and iap (invasion associated protein) genes and mRNA (HLYPNA and IAPPNA, respectively).

Target polynucleotides having polynucleotide sequences corresponding to Salmonella spp may be detected by the instant invention. Sequences specific to Salmonella spp are known in the art (see, e.g., Perez-Perez, F. J., and N. D. Hanson. 2002. Detection of plasmid-mediated AmpC beta-lactamase genes in clinical isolates by using multiplex PCR. J Clin Microbiol 40(6):2153-62., Oliveira, S. D., C. R. Rodenbusch, M. C. Ce, S. L. Rocha, and C. W. Canal. 2003. Evaluation of selective and non-selective enrichment PCR procedures for Salmonella detection. Lett Appl Microbiol 36(4):217-21., and Strapp, C. M., A. E. Shearer, and R. D. Joerger. 2003. Survey of retail alfalfa sprouts and mushrooms for the presence of Escherichia coil O157:H7, Salmonella, and Listeria with BAX, and evaluation of this polymerase chain reaction-based system with experimentally contaminated samples. J Food Prot 66(2):182-7.)

In another embodiment, target polynucleotides having polynucleotide sequences corresponding to Escherichia coli may be detected. Sequences specific to Escherichia coli are known in the art (see, e.g., Heller, L. C., C. R. Davis, K. K. Peak, D. Wingfield, A. C. Cannons, P. T. Amuso, and J. Cattani. 2003. Comparison of Methods for DNA Isolation from Food Samples for Detection of Shiga Toxin-Producing Escherichia coli by Real-Time PCR. Appl Environ Microbiol 69(3):1844-6., Rahman, H. 2002. Multiplex PCR for detection of stx genes of Escherichia coli. Indian J Med Res 115:251-4., and Frahm, E., and U. Obst. 2003. Application of the fluorogenic probe technique (TaqMan PCR) to the detection of Enterococcus spp. and Escherichia coli in water samples. J Microbiol Methods 52(1):123-31.).

In another embodiment, target polynucleotides having polynucleotide sequences corresponding to Campylobacter spp. may be detected. Sequences specific to Campylobacter spp. are known in the art (see, e.g., Carter, J. S., and D. J. Kemp. 2000. A colorimetric detection system for Calymmatobacterium granulomatis. Sex Transm Infect 76(2):134-6., Moreno, Y., S. Botella, J. L. Alonso, M. A. Ferrus, M. Hernandez, and J. Hernandez. 2003. Specific detection of Arcobacter and Campylobacter strains in water and sewage by PCR and fluorescent in situ hybridization. Appl Environ Microbiol 69(2):1181-6., and Bang, D. D., A. Wedderkopp, K. Pedersen, and M. Madsen. 2002. Rapid PCR using nested primers of the 16S rRNA and the hippuricase (hip O) genes to detect Campylobacter jejuni and Campylobacter coli in environmental samples. Mol Cell Probes 16(5):359-69.).

In a further embodiment, target polynucleotides having polynucleotide sequences corresponding to Bacillus spp. may be detected. Sequences specific to Bacillus spp. are known in the art (see, e.g., Mantynen, V., and K. Lindstrom. 1998. A rapid PCR-based DNA test for enterotoxic Bacillus cereus. Appl Environ Microbiol 64(5):1634-9., Schraft, H., and M. W. Griffiths. 1995. Specific oligonucleotide primers for detection of lecithinase-positive Bacillus spp. by PCR. Appl Environ Microbiol 61(1):98-102., and Ley, B. E., C. J. Linton, D. M. Bennett, H. Jalal, A. B. Foot, and M. R. Millar. 1998. Detection of bacteraemia in patients with fever and neutropenia using 16S rRNA gene amplification by polymerase chain reaction. Eur J Clin Microbiol Infect Dis 17(4):247-53).

The target polynucleotides having sequences corresponding to Pseudomonas spp. may also be detected. Sequences specific to Pseudomonas spp. are known in the art (see, e.g., Xu, J., B. C. Millar, J. E. Moore, K. Murphy, H. Webb, A. J. Fox, M. Cafferkey, and M. J. Crowe. 2003. Employment of broad-range 16S rRNA PCR to detect aetiological agents of infection from clinical specimens in patients with acute meningitis-- rapid separation of 16S rRNA PCR amplicons without the need for cloning. J Appl Microbiol 94(2):197-206, Ley, B. E., C. J. Linton, D. M. Bennett, H. Jalal, A. B. Foot, and M. R. Millar. 1998. Detection of bacteraemia in patients with fever and neutropenia using 16S rRNA gene amplification by polymerase chain reaction. Eur J Clin Microbiol Infect Dis 17(4):247-53, and Johnsen, K., O. Enger, C. S. Jacobsen, L. Thirup, and V. Torsvik. 1999. Quantitative selective PCR of 16S ribosomal DNA correlates well with selective agar plating in describing population dynamics of indigenous Pseudomonas spp. in soil hot spots. Appl Environ Microbiol 65(4):1786-8.

The target polynucleotides may also have polynucleotide sequences corresponding to Enterococcus spp. may be detected. Sequences specific to Enterococcus spp. are known in the art (see, e.g., Hudson, C. R., P. J. Fedorka-Cray, M. C. Jackson-Hall, and L. M. Hiott. 2003. Anomalies in species identification of enterococci from veterinary sources using a commercial biochemical identification system. Lett Appl Microbiol 36(4):245-50, Donabedian, S. M., L. A. Thal, E. Hershberger, M. B. Perri, J. W. Chow, P. Bartlett, R. Jones, K. Joyce, S. Rossiter, K. Gay, J. Johnson, C. Mackinson, E. Debess, J. Madden, F. Angulo, and M. J. Zervos. 2003. Molecular characterization of gentamicin-resistant Enterococci in the United States: evidence of spread from animals to humans through food. J Clin Microbiol 41(3): 1109-13., and Vakulenko, S. B., S. M. Donabedian, A. M. Voskresenskiy, M. J. Zervos, S. A. Lerner, and J. W. Chow. 2003. Multiplex PCR for detection of aminoglycoside resistance genes in enterococci. Antimicrob Agents Chemother 47(4):1423-6).

Target polynucleotides may also have polynucleotide sequences corresponding to E. coli 0157. Sequences specific to E. coli 0157 are known in the art (see, e.g., Pan TM, Chen LM, Su YC. Identification of Escherichia coli O157:H7 by multiplex PCR with primers specific to the hlyA, eaeA, stx1, stx2, fliC and rfb genes, J Formos Med Assoc. 2002 Sep;101(9):661-4; Bopp DJ, Sauders BD, Waring AL, Ackelsberg J, Dumas N, Braun-Howland E, Dziewulski D, Wallace BJ, Kelly M, Halse T, Musser KA, Smith PF, Morse DL, Limberger RJ. Detection, Isolation, and Molecular Subtyping of Escherichia coli O157:H and Campylobacter jejuni Associated with a Large Waterborne Outbreak,J Clin Microbiol. 2003 Jan;41(1):174-80; Ibekwe AM, Grieve CM. Detection and quantification of Escherichia coli 0157:H7 in environmental samples by real-time PCR, J Appl Microbiol. 2003;94(3):421-31).

Target polynucleotides may have polynucleotide sequences corresponding to Listeria spp. and. L. monocytogenes. Sequences specific to Listeria spp. and. L. monocytogenes are known in the art (see, e.g., Volokhov D, Rasooly A, Chumakov K, Chizhikov V. Identification of Listeria species by microarray-based assay. J Clin Microbiol. 2002 Dec;40(12):4720-8; Cocolin L, Rantsiou K, Iacumin L, Cantoni C, Comi G. Direct identification in food samples of Listeria spp. and Listeria monocytogenes by molecular methods. Appl Environ Microbiol. 2002 Dec;68(12):6273-82; Shearer AE, Strapp CM, Joerger RD. Evaluation of a polymerase chain reaction-based system for detection of Salmonella enteritidis, Escherichia coli 0157:H7, Listeria spp., and Listeria monocytogenes on fresh fruits and vegetables. J Food Prot. 2001 Jun;64(6):788-95; Bubert A, Hein I, Rauch M, Lehner A, Yoon B, Goebel W, Wagner M. Detection and differentiation of Listeria spp. by a single reaction based on multiplex PCR. Appl Environ Microbiol. 1999 Oct;65(10):4688-92; Jung YS, Frank JF, Brackett RE, Chen J. Polymerase chain reaction detection of Listeria monocytogenes on frankfurters using oligonucleotide primers targeting the genes encoding internalin AB. J Food Prot. 2003 Feb;66(2):237-41; and Pangallo D, Kaclikova E, Kuchta T, Drahovska H. Detection of Listeria monocytogenes by polymerase chain reaction oriented to inlB gene. New Microbiol. 2001 Oct;24(4):333-9).

The target polynucleotides having polynucleotide sequences corresponding to coliforms may be detected. Sequences specific to coliforms are known in the art (see, e.g., Casas N, Sunen E. Detection of enteroviruses, hepatitis A virus and rotaviruses in sewage by means of an immunomagnetic capture reverse transcription-PCR assay. Microbiol Res. 2002;157(3):169-7; Schvoerer E, Ventura M, Dubos O, Cazaux G, Serceau R, Gournier N, Dubois V, Caminade P, Fleury HJ, Lafon ME Qualitative and quantitative molecular detection of enteroviruses in water from bathing areas and from a sewage treatment plant; Res Microbiol. 2001 Mar;152(2):179-86.Bernhard AE, Field KG. Identification of nonpoint sources of fecal pollution in coastal waters by using host-specific 16S ribosomal DNA genetic markers from fecal anaerobes. Appl Environ Microbiol. 2000 Apr;66(4):1587-94).

In another embodiment, target polynucleotides having polynucleotide sequences corresponding to Vibrio cholerae and V. parahaemolyticus may be detected. Sequences specific to Vibrio cholerae and V. parahaemolyticus are known in the art (see, e.g., Myers ML, Panicker G, Bej AK. PCR Detection of a Newly Emerged Pandemic Vibrio parahaemolyticus O3:K6 Pathogen in Pure Cultures and Seeded Waters from the Gulf of Mexico. Appl Environ Microbiol. 2003 Apr;69(4):2194-200; Blackstone GM, Nordstrom JL, Vickery MC, Bowen MD, Meyer RF, DePaola A. Detection of pathogenic Vibrio parahaemolyticus in oyster enrichments by real time PCR. J Microbiol Methods. 2003 May;53(2):149-155; and Lyon WJ. TaqMan PCR for detection of Vibrio cholerae O1, 0139, non-O1, and non-O139 in pure cultures, raw oysters, and synthetic seawater.Appl Environ Microbiol. 2001 Oct;67(10):4685-93).

In a further embodiment, target polynucleotides having polynucleotide sequences corresponding to molds may be detected. Sequences specific to molds are known in the art (see, e.g., Zur G, Shimoni E, Hallerman E, Kashi Y. Detection of Alternaria fungal contamination in cereal grains by a polymerase chain reaction-based assay. J Food Prot. 2002 Sep;65(9):1433-40; Jimenez L, Smalls S, Ignar R. Use of PCR analysis for detecting low levels of bacteria and mold contamination in pharmaceutical samples. J Microbiol Methods. 2000 Aug;41(3):259-65; and Vanittanakom N, Vanittanakom P, Hay RJ. Rapid identification of Penicillium marneffei by PCR-based detection of specific sequences on the rRNA gene, J Clin Microbiol. 2002 May;40(5):1739-42).

The target polynucleotides may have polynucleotide sequences corresponding to Legionella. Sequences specific to Legionella are known in the art (see, e.g., Aoki S, Hirakata Y, Miyazaki Y, Izumikawa K, Yanagihara K, Tomono K, Yamada Y, Tashiro T, Kohno S, Kamihira S. Detection of Legionella DNA by PCR of whole-blood samples in a mouse model. J Med Microbiol. 2003 Apr;52(Pt 4):325-9; Raggam RB, Leitner E, Muhlbauer G, Berg J, Stocher M, Grisold AJ, Marth E, Kessler HH. Qualitative detection of Legionella species in bronchoalveolar lavages and induced sputa by automated DNA extraction and real-time polymerase chain reaction. Med Microbiol Immunol (Berl). 2002 Oct;191(2):119-25; and Alexiou-Daniel S, Stylianakis A, Papoutsi A, Zorbas I, Papa A, Lambropoulos AF, Antoniadis A. Application of polymerase chain reaction for detection of Legionella pneumophila in serum samples. Clin Microbiol Infect. 1998 Mar;4(3):144-148).

Host response mRNA may also be detected in response to infection by foodborne pathogens.

### D. Waterborne Pathogens

The methods disclosed herein also provide a rapid and sensitive diagnostic test for the , presence and enumeration of waterborne pathogens. Waterborne pathogens include bacteria, viruses, and protozoans. Examples of water-borne pathogens include Enterococci, E. coli, Bacillus spp., Psuedomonas spp, Cryptosporidium, and Giardia. Waterborne pathogens may be obtained from any water sample, including, but not limited to, swimming pools, aquatic parks, wells, home drinking water, reservoirs, beaches, lakes, oceans, fish and shellfish farms, agricultural water, dialysis water, medication reconstitution water, water treatment facilities, cruise ships, space shuttle effluent, and bottled water.

The methods disclosed herein readily allow the detection of water-borne pathogens. By way of example, specific strains may be detected. For example, specific strains of Enterococci that be can be detected over other strains include Enterococci avium, E. casseliflavus, E. cecorum, E. columbae, E. dispar, E. durans, E. faecium, E. faecalis, E. gallinarum, E. hirae, E. malodoratus, E. mundtii, E. pseudovium, E. raffinosus, E. saccharolyticus, and E. sulfurous.

Any polynucleotide associated with waterborne pathogens may be identified by the methods of the present invention. The method may be used to detect genes associated with Enterococcus spp., E. coli, Bacillus spp., and Psuedomonas spp., which have been described (see, e.g., Escherichia coli: Heller, L. C., C. R. Davis, K. K. Peak, D. Wingfield, A. C. Cannons, P. T. Amuso, and J. Cattani. 2003, Comparison of Methods for DNA Isolation from Food Samples for Detection of Shiga Toxin-Producing Escherichia coli by Real-Time PCR. Appl Environ Microbiol 69(3):1844-6.; Rahman, H. 2002. Multiplex PCR for detection of stx genes of Escherichia coli. Indian J Med Res 115:251-4., and Frahm, E., and U. Obst. 2003. Application of the fluorogenic probe technique (TaqMan PCR) to the detection of Enterococcus spp. and Escherichia coli in water samples. J Microbiol Methods 52(1):123-31. Bacillus spp: Mantynen, V., and K. Lindstrom. 1998. A rapid PCR-based DNA test for enterotoxic Bacillus cereus. Appl Environ Microbiol 64(5): 1634-9., Schraft, H., and M. W. Griffiths. 1995. Specific oligonucleotide primers for detection of lecithinase-positive Bacillus spp. by PCR. Appl Environ Microbiol 61(1):98-102., and Ley, B. E., C. J. Linton, D. M. Bennett, H. Jalal, A. B. Foot, and M. R. Millar. 1998. Detection of bacteraemia in patients with fever and neutropenia using 16S rRNA gene amplification by polymerase chain reaction. Eur J Clin Microbiol Infect Dis 17(4):247-53. Pseudomonas spp: Xu, J., B. C. Millar, J. E. Moore, K. Murphy, H. Webb, A. J. Fox, M. Cafferkey, and M. J. Crowe. 2003. Employment of broad-range 16S rRNA PCR to detect aetiological agents of infection from clinical specimens in patients with acute meningitis - rapid separation of 16S rRNA PCR amplicons without the need for cloning. J Appl Microbiol 94(2):197-206., Ley, B. E., C. J. Linton, D. M. Bennett, H. Jalal, A. B. Foot, and M. R. Millar. 1998. Detection of bacteraemia in patients with fever and neutropenia using 16S rRNA gene amplification by polymerase chain reaction. Eur J Clin Microbiol Infect Dis 17(4):247-53., and Johnsen, K., O. Enger, C. S. Jacobsen, L. Thirup, and V. Torsvik. 1999. Quantitative selective PCR of 16S ribosomal DNA correlates well with selective agar plating in describing population dynamics of indigenous Pseudomonas spp. in soil hot spots. Appl Environ Microbiol 65(4):1786-8. Enterococcus spp: Hudson, C. R., P. J. Fedorka-Cray, M. C. Jackson-Hall, and L. M. Hiott. 2003. Anomalies in species identification of enterococci from veterinary sources using a commercial biochemical identification system. Lett Appl Microbiol 36(4):245-50., Donabedian, S. M., L. A. Thal, E. Hershberger, M. B. Perri, J. W. Chow, P. Bartlett, R. Jones, K. Joyce, S. Rossiter, K. Gay, J. Johnson, C. Mackinson, E. Debess, J. Madden, F. Angulo, and M. J. Zervos. 2003. Molecular characterization of gentamicin-resistant Enterococci in the United States: evidence of spread from animals to humans through food. J Clin Microbiol 41(3):1109-13., and Vakulenko, S. B., S. M. Donabedian, A. M. Voskresenskiy, M. J. Zervos, S. A. Lerner, and J. W. Chow. 2003. Multiplex PCR for detection of aminoglycoside resistance genes in enterococci. Antimicrob Agents Chemother 47(4):1423-6. For example, specific Enterococci genes that may be identified by the methods disclosed herein include 16S ribosomal RNA sequences that are conserved in Enterococci.

Host response mRNA may also be detected in response to infection by waterborne pathogens.

### E. Agroterrorism

Nucleic acid analogs may be designed to have the sequences of primers used for PCR-based detection of specific pathogens implicated in agroterrorism. Examples of pathogens of clinical importance and the references for the PCR-based detection are listed below. Nucleic acid analogs may be designed to have the sequence of specific PCR primer sequences, and can be used in combination in many of the embodiments of the invention described herein.

Nucleic acid analogs may be designed, for example, to have the sequences of primers, or fragments thereof, used for PCR-based detection of Toxoplasma gondii. Toxoplasma gondii has very low host specificity, and probably infects almost any mammal. It has also been reported from birds, and has been found in virtually every country of the world. Like most of the Apicomplexa, Toxoplasma is an obligate intracellular parasite. In most humans infected with Toxoplasma, the disease is asymptomatic. However, under some conditions, toxoplasmosis can cause serious pathology, including hepatitis, pneumonia, blindness, and severe neurological disorders. Primers used in PCR-based detection of Toxoplasma gondii are known in the art (see, e.g., Aspinall, T. V., D. Marlee, J. E. Hyde, and P. F. Sims. 2002. Prevalence of Toxoplasma gondii in commercial meat products as monitored by polymerase chain reaction--food for thought? Int J Parasitol 32(9):1193-9., Aspinall, T. V., E. C. Guy, K. E. Roberts, D. H. Joynson, J. E. Hyde, and P. F. Sims. 2003. Molecular evidence for multiple Toxoplasma gondii infections in individual patients in England and Wales: public health implications. Int J Parasitol 33(1):97-103., Fuentes, I., J. M. Rubio, C. Ramirez, and J. Alvar. 2001. Genotypic characterization of Toxoplasma gondii strains associated with human toxoplasmosis in Spain: direct analysis from clinical samples. J Clin Microbiol 39(4):1566-70., and Wamekulasuriya, M. R., J. D. Johnson, and R. E. Holliman. 1998. Detection of Toxoplasma gondii in cured meats. Int J Food Microbiol 45(3):211-5.).

In another embodiment, nucleic acid analogs may be designed to have the sequences of primers, or fragments thereof, used for PCR-based detection of Shingella spp, a gram negative bacterium. Primers used in PCR-based detection of Shingella spp are known in the art (Hartman, A. B., Essiet, II, D. W. Isenbarger, and L. E. Lindler. 2003. Epidemiology of Tetracycline Resistance Determinants in Shigella spp. and Enteroinvasive Escherichia coli: Characterization and Dissemination of tet(A)-1. J Clin Microbiol 41 (3):1023-32., Lampel, K. A., and P. A. Orlandi. 2002. Polymerase chain reaction detection of invasive Shigella and Salmonella enterica in food. Methods Mol Biol 179:235-44., Wang, R. F., W. W. Cao, and C. E. Cerniglia. 1997. A universal protocol for PCR detection of 13 3 species of foodborne pathogens in foods. J Appl Microbiol 83(6):727-36., and Lampel, K. A., J. A. Jagow, M. Trucksess, and W. E. Hill. 1990. Polymerase chain reaction for detection of invasive Shigella flexneri in food. Appl Environ Microbiol 56(6):1536-40.).

In a further embodiment, nucleic acid analogs may be designed to have the sequences of primers, or fragments thereof, used for PCR-based detection of Cyclospora cayetanensis. Cyclospora cayetanensis is a parasite composed of one cell, too small to be seen without a microscope. The first known human cases of illness caused by Cyclospora infection (i.e., cyclosporiasis) were reported in 1979. Cases began being reported more often in the mid-1980s. In the last several years, outbreaks of cyclosporiasis have been reported in the United States and Canada. Cyclospora is spread ingestion of water or food that was contaminated with infected stool. Outbreaks of cyclosporiasis have been linked to various types of fresh produce. Cyclospora needs time (days or weeks) after being passed in a bowel movement to become infectious. Therefore, it is unlikely that Cyclospora is passed directly from one person to another.

Primers used in PCR-based detection of Cyclospora cayetanensis are known in the art (see, e.g., Eberhard, M. L., N. J. Pieniazek, and M. J. Arrowood. 1997. Laboratory diagnosis of Cyclospora infections. Arch Pathol Lab Med 121(8):792-7., Pieniazek, N. J., S. B. Slemenda, A. J. da Silva, E. M. Alfano, and M. J. Arrowood. 1996. PCR confirmation of infection with Cyclospora cayetanensis. Emerg Infect Dis 2(4):357-9., Quintero-Betancourt, W., E. R. Peele, and J. B. Rose. 2002. Cryptosporidium parvum and Cyclospora cayetanensis: a review of laboratory methods for detection of these waterborne parasites. J Microbiol Methods 49(3):209-24., and Varma, M., J. D. Hester, F. W. Schaefer, 3rd, M. W. Ware, and H. D. Lindquist. 2003. Detection of Cyclospora cayetanensis using a quantitative real-time PCR assay. J Microbiol Methods 53(1):27-36.)

### F. Disease Diagnostics

The methods disclosed herein also provide a rapid and sensitive diagnostic test for the presence of genes (e.g., disease alleles) or altered expression of genes. The detected gene sequences may be implicated in genetic diseases, disorders, and predispositions. One example of target polynucleotides includes specific genomic sequences, including mutations, polymorphisms, additions and deletions. Another example of target polynucleotides are polynucleotide sequences that have altered gene expression (e.g. up-regulated or down-regulated expression) in a specific disease or disorder. Examples of disease and disorder-related genes include, but are not limited to, genes implicated in cancer, cystic fibrosis, and Down's syndrome, and disease associated polymorphisms and mutations. Hereditary hemachromatosis, factor 11 and factor V, and HLA genotypes for tissue transplants may also be identified.

Nucleic acid analogs may be designed to have the sequences of primers, or fragments thereof, used for PCR-based detection of target polynucleotides implicated in cancer, or altered cell proliferation. Target polynucleotides corresponding to genes implicated in cancer, or altered cell proliferation, may be detected. Numerous genes implicated in cancer are known in the art.

In one embodiment, nucleic acid analogs may be designed to have the sequences of primers, or fragments thereof, used for PCR-based detection of pancreatic cancer. Primers used in PCR-based detection of pancreatic cancer, such as loss of DPC4, are known in the art (see, e.g., Barbera, V. M., M. Martin, L. Marinoso, A. Munne, A. Carrato, F. X. Real, and M. Fabre. 2000. The 18q21 region in colorectal and pancreatic cancer: independent loss of DCC and DPC4 expression. Biochim Biophys Acta 1502(2):283-96, Bartsch, D., P. Barth, D. Bastian, A. Ramaswamy, B. Gerdes, B. Chaloupka, Y. Deiss, B. Simon, and A. Schudy. 1999. Higher frequency of DPC4/Smad4 alterations in pancreatic cancer cell lines than in primary pancreatic adenocarcinomas. Cancer Lett 139(1):43-9, and Bartsch, D., S. A. Hahn, K. D. Danichevski, A. Ramaswamy, D. Bastian, H. Galehdari, P. Barth, W. Schmiegel, B. Simon, and M. Rothmund. 1999. Mutations of the DPC4/Smad4 gene in neuroendocrine pancreatic tumors. Oncogene 18(14):2367-71).

In another embodiment, nucleic acid analogs may be designed to have the sequences of primers, or fragments thereof, used for PCR-based detection of cervical cancer. Primers used in PCR-based detection of cervical cancer. Target polynucleotides corresponding to all or part of the genes associated with cervical cancer may also be identified (see, e.g., (Si, H. X., S. W. Tsao, C. S. Poon, L. D. Wang, Y. C. Wong, and A. L. Cheung. 2003. Viral load of HPV in esophageal squamous cell carcinoma. Int J Cancer 103(4):496-500, Si, H. X., S. W. Tsao, C. S. Poon, L. D. Wang, Y. C. Wong, and A. L. Cheung. 2003. Viral load of HPV in esophageal squamous cell carcinoma. Int J Cancer 103(4):496-500, and Castle, P. E., M. Schiffman, P. E. Gravitt, H. Kendall, S. Fishman, H. Dong, A. Hildesheim, R. Herrero, M. C. Bratti, M. E. Sherman, A. Lorincz, J. E. Schussler, and R. D. Burk. 2002. Comparisons of HPV DNA detection by MY09/11 PCR methods. J Med Virol 68(3):417-23).

Nucleic acid analogs may be designed to have the sequences of primers, or fragments thereof, used for PCR-based detection of breast cancer. Primers used in PCR-based detection of breast cancer. Target polynucleotides may be selected to have sequences corresponding to all or part of the genes associated with breast cancer (see, e.g., Min, C. J., L. Tafra, and K. M. Verbanac. 1998. Identification of superior markers for polymerase chain reaction detection of breast cancer metastases in sentinel lymph nodes. Cancer Res 58(20):4581-4, Abbaszadegan, M. R., J. P. Struewing, K. M. Brown, J. V. Snider, F. Goodsaid, R. Gore-Langton, and M. R. Hughes. 1997. Automated detection of prevalent mutations in BRCA1 and BRCA2 genes, using a fluorogenic PCR allelic discrimination assay. Genet Test 1(3):171-80, and Tamura, G., C. Maesawa, Y. Suzuki, M. Kashiwaba, M. Ishida, K. Saito, and R. Satodate. 1994. Improved detection of loss of heterozygosity at retinoblastoma gene locus in human breast carcinoma. Pathol Int 44(1):34-8).

Nucleic acid analogs may be designed to have the sequences of primers, or fragments thereof, used for PCR-based detection of melanoma. Primers used in PCR-based detection of melanoma. Target polynucleotides may be selected to have sequences corresponding to all or part of the genes associated with melanoma. For example, target polynucleotides may include mutations in CDKN2 (see, e.g., Gonzalgo, M. L., C. M. Bender, E. H. You, J. M. Glendening, J. F. Flores, G. J. Walker, N. K. Hayward, P. A. Jones, and J. W. Fountain. 1997. Low frequency of p16/CDKN2A methylation in sporadic melanoma: comparative approaches for methylation analysis of primary tumors. Cancer Res 57(23):5336-47, Chan, J., E. S. Robinson, J. Atencio, Z. Wang, S. Kazianis, L. D. Coletta, R. S. Nairn, and J. R. McCarrey. 2001. Characterization of the CDKN2A and ARF genes in UV-induced melanocytic hyperplasias and melanomas of an opossum (Monodelphis domestica). Mol Carcinog 31(1):16-26, and Pollock, P. M., J. Welch, and N. K. Hayward. 2001. Evidence for three tumor suppressor loci on chromosome 9p involved in melanoma development. Cancer Res 61(3):1154-61).

In addition, nucleic acid analogs may be designed to have the sequences of primers, or fragments thereof, used for PCR-based detection of colorectal cancer. Primers used in PCR-based detection of colorectal cancer. Target polynucleotides may be selected to have sequences corresponding to all or part of the genes associated with colorectal cancer (see, e.g., Aoki, S., Y. Takagi, M. Hayakawa, K. Yamaguchi, M. Futamura, K. Kunieda, and S. Saji. 2002. Detection of peritoneal micrometastases by reverse transcriptase- polymerase chain reaction targeting carcinoembryonic antigen and cytokeratin 20 in colon cancer patients. J Exp Clin Cancer Res 21 (4):555-62., Shtoyerman-Chen, R., E. Friedman, A. Figer, M. Carmel, Y. Patael, P. Rath, H. H. Fidder, S. Bar-Meir, and L. Theodor. 2001. The I1307K APC polymorphism: prevalence in non-Ashkenazi Jews and evidence for a founder effect. Genet Test 5(2):141-6.,and Smith, W. M., N. J. Van Orsouw, E. A. Fox, R. D. Kolodner, J. Vijg, and C. Eng. 1998. Accurate, high-throughput "snapshot" detection of hMLH1 mutations by two-dimensional DNA electrophoresis. Genet Test 2(1):43-53.).

Nucleic acid analogs may be designed to have the sequences of primers, or fragments thereof, used for PCR-based detection of retinoblastoma. Primers used in PCR-based detection of retinoblastoma. Target polynucleotides may be selected to have sequences corresponding to all or part of the genes associated with retinoblastoma (see, e.g., Acikbas, I., I. Keser, S. Kilic, H. Bagci, G. Karpuzoglu, and G. Luleci. 2002. Detection of LOH of the RB1 gene in bladder cancers by PCR-RFLP. Urol Int 68(3):189-92, Tamura, G., C. Maesawa, Y. Suzuki, M. Kashiwaba, M. Ishida, K. Saito, and R. Satodate. 1994. Improved detection of loss of heterozygosity at retinoblastoma gene locus in human breast carcinoma. Pathol Int 44(1):34-8, and Lohmann, D., B. Horsthemke, G. Gillessen-Kaesbach, F. H. Stefani, and H. Hofler. 1992. Detection of small RB1 gene deletions in retinoblastoma by multiplex PCR and high-resolution gel electrophoresis. Hum Genet 89(1):49-53.

In a further embodiment, nucleic acid analogs may be designed to have the sequences of primers, or fragments thereof, used for PCR-based detection of hemopheilia. Primers used in PCR-based detection of hemophelia. Target polynucleotides may be selected to have sequences corresponding to genes associated with hemophelia (see, e.g., Mannucci, P. M. 2002. Ham-wasserman lecture: hemophilia and related bleeding disorders: a story of dismay and success. Hematology (Am Soc Hematol Educ Program):1-9,and Citron, M., L. Godmilow, T. Ganguly, and A. Ganguly. 2002. High throughput mutation screening of the factor VIII gene (F8C) in hemophilia A: 37 novel mutations and genotype-phenotype correlation. Hum Mutat 20(4):267-74).

Target polynucleotides that have sequences corresponding to genes associated with phenylketylurea (PKU) may be selected (see, e.g., Pronina, N., S. Giannattasio, P. Lattanzio, R. Lugovska, P. Vevere, and A. Kornejeva. 2003. The molecular basis of phenylketonuria in Latvia. Hum Mutat 21(4):398-9, Sueoka, H., M. Nagao, and S. Chiba. 2000. Rapid mutation screening of phenylketonuria by polymerase chain reaction-linked restriction enzyme assay and direct sequence of the phenylalanine hydroxylase gene: clinical application in northern Japan and northern China. Genet Test 4(3):249-56, and Eisensmith, R. C., A. A. Goltsov, and S. L. Woo. 1994. A simple, rapid, and highly informative PCR-based procedure for prenatal diagnosis and carrier screening of phenylketonuria. Prenat Diagn 14(12):1113-8.). Alford, R. L., T. Ashizawa, J. Jankovic, C. T. Caskey, and C. S. Richards. 1996. Molecular detection of new mutations, resolution of ambiguous results and complex genetic counseling issues in Huntington disease. Am J Med Genet 66(3):281-6, Vnencak-Jones, C. L. 2003. Fluorescence PCR and GeneScan analysis for the detection of CAG repeat expansions associated with Huntington's disease. Methods Mol Biol 217:101-8, and Panagopoulos, I., C. Lassen, U. Kristoffersson, and P. Aman. 1999. A novel PCR-based approach for the detection of the Huntington disease associated trinucleotide repeat expansion. Hum Mutat 13(3):232-6).

Target polynucleotides that have sequences corresponding to genes associated with Huntington's Disease may be selected, for example due to increased CAG repeats (see, e.g., Alford, R. L., T. Ashizawa, J. Jankovic, C. T. Caskey, and C. S. Richards. 1996. Molecular detection of new mutations, resolution of ambiguous results and complex genetic counseling issues in Huntington disease. Am J Med Genet 66(3):281-6, Vnencak-Jones, C. L. 2003. Fluorescence PCR and GeneScan analysis for the detection of CAG repeat expansions associated with Huntington's disease. Methods Mol Biol 217:101-8, and Panagopoulos, I., C. Lassen, U. Kristoffersson, and P. Aman. 1999. A novel PCR-based approach for the detection of the Huntington disease associated trinucleotide repeat expansion. Hum Mutat 13(3):232-6.)

Target polynucleotides that have sequences corresponding to genes associated with hemophelia may be selected (see, e.g., Mannucci, P. M. 2002. Ham-wasserman lecture: hemophilia and related bleeding disorders: a story of dismay and success. Hematology (Am Soc Hematol Educ Program): 1-9,and Citron, M., L. Godmilow, T. Ganguly, and A. Ganguly. 2002. High throughput mutation screening of the factor VIII gene (F8C) in hemophilia A: 37 novel mutations and genotype-phenotype correlation. Hum Mutat 20(4):267-74.)

Target polynucleotides that have sequences corresponding to genes associated with Tay-Sach's Disease may be selected (see, e.g., Rice, J. E., J. A. Sanchez, K. E. Pierce, and L. J. Wangh. 2002. Real-time PCR with molecular beacons provides a highly accurate assay for detection of Tay-Sachs alleles in single cells. Prenat Diagn 22(12):1130-4, Tamasu, S., H. Nishio, H. Ayaki, M. J. Lee, M. Mizutori, Y. Takeshima, H. Nakamura, M. Matsuo, T. Maruo, and K. Sumino. 1999. Prenatal diagnosis of a Japanese family at risk for Tay-Sachs disease. Application of a fluorescent competitive allele-specific polymerase chain reaction (PCR) method. Kobe J Med Sci 45(6):259-70, and Sermon, K., W. Lissens, Z. P. Nagy, A. Van Steirteghem, and I. Liebaers. 1995. Simultaneous amplification of the two most frequent mutations of infantile Tay-Sachs disease in single blastomeres. Hum Reprod 10(8):2214-7.).

Target polynucleotides that have sequences corresponding to genes associated with Multiple Schlorosis (MS) may be selected (see, e.g., Lauwers, S., Y. Vander Heyden, and B. Rombaut. 2002. Screening and optimisation of an ELISA method for the quantitative detection of enterovirus specific RT-PCR products by means of a two- level experimental design. J Pharm Biomed Anal 29(4):659-68, Perron, H., J. A. Garson, F. Bedin, F. Beseme, G. Paranhos-Baccala, F. Komurian-Pradel, F. Mallet, P. W. Tuke, C. Voisset, J. L. Blond, B. Lalande, J. M. Seigneurin, and B. Mandrand. 1997. Molecular identification of a novel retrovirus repeatedly isolated from patients with multiple sclerosis. The Collaborative Research Group on Multiple Sclerosis. Proc Natl Acad Sci U S A 94(14):7583-8, and Kuhne, B. S., and P. Oschmann. 2002. Quantitative real-time RT-PCR using hybridization probes and imported standard curves for cytokine gene expression analysis. Biotechniques 33(5):1078, 1080-2, 1084 passim.)

Target polynucleotides may be selected to have sequences corresponding to genes associated with Burkitt's lymphoma (see, e.g., Wu, Y., Y. Chen, J. Xu, and L. Lu. 2002. Anticancer activities of curcumin on human Burkitt's lymphoma. Zhonghua Zhong Liu Za Zhi 24(4):348-52, Basso, K., E. Frascella, L. Zanesco, and A. Rosolen. 1999. Improved long-distance polymerase chain reaction for the detection of t(8;14)(q24;q32) in Burkitt's lymphomas. Am J Pathol 155(5):1479-85, and Asada, M., T. Miyashita, F. Bessho, N. Kobayashi, and S. Mizutani. 1991. Malignant cell detection in Burkitt's lymphoma using third- complementarity-determining region (CDRIII), clone-specific probe developed by sequencing DNA from stored slides. Jpn J Cancer Res 82(7):848-53).

Target polynucleotides may be selected to have sequences corresponding to genes associated with cystic fibrosis (see, e.g., Tomaiuolo, R., M. Spina, and G. Castaldo. 2003. Molecular diagnosis of cystic fibrosis: comparison of four analytical procedures. Clin Chem Lab Med 41(1):26-32, Gonzalez-Gonzalez, M. C., M. Garcia-Hoyos, M. J. Trujillo, M. Rodriguez de Alba, I. Lorda-Sanchez, J. Diaz-Recasens, E. Gallardo, C. Ayuso, and C. Ramos. 2002. Prenatal detection of a cystic fibrosis mutation in fetal DNA from maternal plasma. Prenat Diagn 22(10):946-8, and Corbetta, C., M. Seia, A. Bassotti, A. Ambrosioni, A. Giunta, and R. Padoan. 2002. Screening for cystic fibrosis in newborn infants: results of a pilot programme based on a two tier protocol (IRT/DNA/IRT) in the Italian population. J Med Screen 9(2):60-3.).

Target polynucleotides may be selected to have sequences corresponding to genes associated with juvenile onset diabetes (see, e.g., Knerr, I., R. Repp, J. Dotsch, N. Gratzki, J. Hanze, T. Kapellen, and W. Rascher. 1999. Quantitation of gene expression by real-time PCR disproves a "retroviral hypothesis" for childhood-onset diabetes mellitus. Pediatr Res 46(1):57-60, Lauwers, S., Y. Vander Heyden, and B. Rombaut. 2002. Screening and optimisation of an ELISA method for the quantitative detection of enterovirus specific RT-PCR products by means of a two- level experimental design. J Pharm Biomed Anal 29(4):659-68, and Salminen, K., K. Sadeharju, M. Lonnrot, P. Vahasalo, A. Kupila, S. Korhonen, J. Ilonen, O. Simell, M. Knip, and H. Hyoty. 2003. Enterovirus infections are associated with the induction of beta-cell autoimmunity in a prospective birth cohort study. J Med Virol 69(1):91-8.).

Target polynucleotides may be selected to have sequences corresponding to genes associated with Parkinson's disease (see, e.g., Hoenicka, J., L. Vidal, B. Morales, I. Ampuero, F. J. Jimenez-Jimenez, J. Berciano, T. del Ser, A. Jimenez, P. G. Ruiz, and J. G. de Yebenes. 2002. Molecular findings in familial Parkinson disease in Spain. Arch Neurol 59(6):966-70.; Lucking, C. B., and A. Brice. 2003. Semiquantitative PCR for the detection of exon rearrangements in the Parkin gene. Methods Mol Biol 217:13-26; Le, W. D., P. Xu, J. Jankovic, H. Jiang, S. H. Appel, R. G. Smith, and D. K. Vassilatis. 2003. Mutations in NR4A2 associated with familial Parkinson disease. Nat Genet 33(1):85-9.; and Foroud, T., S. K. Uniacke, L. Liu, N. Pankratz, A. Rudolph, C. Halter, C. Shults, K. Marder, P. M. Conneally, and W. C. Nichols. 2003. Heterozygosity for a mutation in the parkin gene leads to later onset Parkinson disease. Neurology 60(5):796-801).

Target polynucleotides may be selected to have sequences corresponding to genes associated with Alzheimer's Disease (see, e.g., Maresh, G. A., D. Erezyilmaz, C. E. Murry, D. Nochlin, and A. D. Snow. 1996. Detection and quantitation of perlecan mRNA levels in Alzheimer's disease and normal aged hippocampus by competitive reverse transcription-polymerase chain reaction. J Neurochem 67(3):1132-44; and Smith, M. J., R. J. Gardner, M. A. Knight, S. M. Forrest, K. Beyreuther, E. Storey, C. A. McLean, R. G. Cotton, R. Cappal, and C. L. Masters. 1999. Early-onset Alzheimer's disease caused by a novel mutation at codon 219 of the presenilin-1 gene. Neuroreport 10(3):503-7).

Target polynucleotides may be selected to have sequences corresponding to genes associated with thrombosis (see, e.g. Sanders Sevall, J. 2000. Factor V Leiden genotyping using real-time fluorescent polymerase chain reaction. Mol Cell Probes 14(4):249-53; Ferreira-Gonzalez, A., L. M. Fisher, C. M. Lehman, M. H. Langley, D. H. Lofland, Q. Xia, N. X. Nguyen, D. Modesto, J. B. Willoughby, D. S. Wilkinson, and C. T. Garrett. 1997. Detection of a common mutation in factor V gene responsible for resistance to activate protein C causing predisposition to thrombosis. J Clin Lab Anal 11(6):328-35; Warshawsky, I., C. Hren, L. Sercia, B. Shadrach, S. R. Deitcher, E. Newton, and K. Kottke-Marchant. 2002. Detection of a novel point mutation of the prothrombin gene at position 20209. Diagn Mol Pathol 11(3):152-6.).

Target polynucleotides may be selected to have sequences corresponding to genes associated with susceptibility to tuberculosis (see, e.g., Bellamy R. Susceptibility to mycobacterial infections: the importance of host genetics. Genes Immun. 2003 Jan;4(1):4-11; Awomoyi AA, Marchant A, Howson JM, McAdam KP, Blackwell JM, Newport MJ. Interleukin-10, polymorphism in SLC11A1 (formerly NRAMP1), and susceptibility to tuberculosis. J Infect Dis. 2002 Dec 15;186(12):1808-14. Bellamy R. NRAMP1 and susceptibility to tuberculosis. Int J Tuberc Lung Dis. 2002 Sep;6(9):747).

Target polynucleotides may be selected to have sequences corresponding to genes associated with human immunodeficiency virus (HIV) (see, e.g.Ifergan I, Bernard NF, Bruneau J, Alary M, Tsoukas CM, Roger M. Allele frequency of three functionally active polymorphisms of the MDR-1 Gene in high-risk HIV-negative and HIV-positive Caucasians. AIDS. 2002 Nov 22;16(17):2340-2; and Farzan M, Mirzabekov T, Kolchinsky P, Wyatt R, Cayabyab M, Gerard NP, Gerard C, Sodroski J, Choe H. Tyrosine sulfation of the amino terminus of CCR5 facilitates HIV-1 entry. Cell. 1999 Mar 5;96(5):667-76).The methods herein also provide a rapid and sensitive diagnostic test for the presence of genes or altered expression of genes in other kinds of genetic analysis. For example, the may be used to determine the identity of human samples, for example in paternity tests, forensics, or in matching related or unrelated organ donors. Diagnostics may also be conducted in non-human samples, such as to identify cattle lineages.

### G. Sexually Transmitted Diseases

The nucleic acid analogs may be designed to have the sequences, or fragments of the sequences, of primers used for PCR-based detection of pathogens that cause sexually transmitted diseases (STDs). Examples of STDs of clinical importance and the references for the PCR-based detection are listed below. Nucleic acid analogs may be designed to have the sequence of specific PCR primer sequences, and can be used in combination in many of the embodiments of the invention described herein.

In one embodiment, the nucleic acid analogs are designed to have the sequences of primers used for PCR-based detection of chlamydia. Many such primers are known in the art (see, e.g., Koumans, E. H., C. M. Black, L. E. Markowitz, E. Unger, A. Pierce, M. K. Sawyer, and J. R. Papp. 2003. Comparison of methods for detection of Chlamydia trachomatis and Neisseria gonorrhoeae using commercially available nucleic acid amplification tests and a liquid pap smear medium. J Clin Microbiol 41(4):1507-11; Puolakkainen, M., E. Hiltunen-Back, T. Reunala, S. Suhonen, P. Lahteenmaki, M. Lehtinen, and J. Paavonen. 1998. Comparison of performances of two commercially available tests, a PCR assay and a ligase chain reaction test, in detection of urogenital Chlamydia trachomatis infection. J Clin Microbiol 36(6):1489-93; Betsou F, Beaumont K, Sueur JM, Orfila J. Construction and evaluation of internal control DNA for PCR amplification of Chlamydia trachomatis DNA from urine samples. J Clin Microbiol. 2003 Mar; 41(3):1274-6; Knox J, Tabrizi SN, Miller P, Petoumenos K, Law M, Chen S, Garland SM. Evaluation of self-collected samples in contrast to practitioner-collected samples for detection of Chlamydia trachomatis, Neisseria gonorrhoeae, and Trichomonas vaginalis by polymerase chain reaction among women living in remote areas. Sex Transm Dis. 2002 Nov;29(11):647-54;Mygind T, Birkelund S, Birkebaek N, Oestergaard L, Jensen J, Christiansen G. Determination of PCR efficiency in chelex-100 purified clinical samples and comparison of real-time quantitative PCR and conventional PCR for detection of Chlamydia pneumoniae. BMC Microbiol. 2002 Jul 9;2(1):17).

In another embodiment, the nucleic acid analogs are designed to have the sequences of primers used for PCR-based detection of Treponema pallidum, have the sequences of primers used for PCR-based detection of Treponema pallidum. Examples of primers used to identify Treponema pallidum by PCR based assays are known in the art (see, e.g., Centurion-Lara, A., C. Castro, J. M. Shaffer, W. C. Van Voorhis, C. M. Marra, and S. A. Lukehart. 1997. Detection of Treponema pallidum by a sensitive reverse transcriptase PCR. J Clin Microbiol 35(6):1348-52; Marfin, A. A., H. Liu, M. Y. Sutton, B. Steiner, A. Pillay, and L. E. Markowitz. 2001. Amplification of the DNA polymerase I gene of Treponema pallidum from whole blood of persons with syphilis. Diagn Microbiol Infect Dis 40(4):163-6; Orton, S. L., H. Liu, R. Y. Dodd, and A. E. Williams. 2002. Prevalence of circulating Treponema pallidum DNA and RNA in blood donors with confirmed-positive syphilis tests. Transfusion 42(1):94-9.

In another embodiment, the nucleic acid analogs are designed to have the sequences of primers used for PCR-based detection of HIV. Examples of primers used to identify HIV by PCR based assays are known in the art (see, e.g., Gibney, L., M. Macaluso, K. Kirk, M. S. Hassan, J. Schwebke, S. H. Vermund, and P. Choudhury. 2001. Prevalence of infectious diseases in Bangladeshi women living adjacent to a truck stand: HIV/STD/hepatitis/genital tract infections. Sex Transm Infect 77(5):344-50; Spinillo, A., M. Debiaggi, F. Zara, R. Maserati, F. Polatti, and A. De Santolo. 2001. Factors associated with nucleic acids related to human immunodeficiency virus type 1 in cervico-vaginal secretions. Bjog 108(6):634-41).

In a further embodiment, the nucleic acid analogs are designed to have the sequences of primers used for PCR-based detection of human papillomavirus (HPV). Examples of primers used to identify HPV by PCR based assays are known in the art (see, e.g., Winer, R. L., S. K. Lee, J. P. Hughes, D. E. Adam, N. B. Kiviat, and L. A. Koutsky. 2003. Genital human papillomavirus infection: incidence and risk factors in a cohort of female university students. Am J Epidemiol 157(3):218-26; Levi, J. E., B. Kleter, W. G. Quint, M. C. Fink, C. L. Canto, R. Matsubara, I. Linhares, A. Segurado, B. Vanderborght, J. E. Neto, and L. J. Van Doom. 2002. High prevalence of human papillomavirus (HPV) infections and high frequency of multiple HPV genotypes in human immunodeficiency virus- infected women in Brazil. J Clin Microbiol 40(9):3341-5; Skerlev, M., M. Grce, M. Sirotkoviae-Skerlev, K. Husnjak, and J. Lipozencic. 2002. Human papillomavirus male genital infections: clinical variations and the significance of DNA typing. Clin Dermatol 20(2):173-8).

In another embodiment, the nucleic acid analogs are designed to have the sequences of primers used for PCR-based detection of Neisseria gonorrhoeae. Examples of primers used to identify Neisseria gonorrhoeae by PCR based assays are known in the art (see, e.g., Mgone, C. S., T. Lupiwa, and W. Yeka. 2002. High prevalence of Neisseria gonorrhoeae and multiple sexually transmitted diseases among rural women in the Eastern Highlands Province of Papua New Guinea, detected by polymerase chain reaction. Sex Transm Dis 29(12):775-9; Gomes, J. P., L. Tavira, F. Exposto, E. Prieto, and M. A. Catry. 2001. Neisseria gonorrhoeae and Chlamydia trachomatis infections in patients attending STD and family planning clinics in Bissau, Guinea-Bissau. Acta Trop 80(3):261-4; Diemert, D. J., M. D. Libman, and P. Lebel. 2002. Confirmation by 16S rRNA PCR of the COBAS AMPLICOR CT/NG test for diagnosis of Neisseria gonorrhoeae infection in a low-prevalence population. J Clin Microbiol 40(11):4056-9).

In another embodiment, the nucleic acid analogs are designed to have the sequences of primers used for PCR-based detection of genital herpes. Examples of primers used to identify genital gerpes by PCR based assays are known in the art (see, e.g., Wolff, M. H., J. Schmitt, M. Rahaus, H. Dudda, and W. Hatzmann. 2002. Clinical and subclinical reactivation of genital herpes virus. Intervirology 45(1):20-3; Wald, A. 2002. Testing for genital herpes: how, who, and why. Curr Clin Top Infect Dis 22:166-80; Scoular, A. 2002. Using the evidence base on genital herpes: optimising the use of diagnostic tests and information provision. Sex Transm Infect 78(3):160-5).

The nucleic acid analogs may also be designed to have the sequences of primers used for PCR-based detection of Trichomonas vanginalis. Examples of primers used to identify Trichomonas vanginalis by PCR based assays are known in the art (see, e.g., Wendel, K. A., E. J. Erbelding, C. A. Gaydos, and A. M. Rompalo. 2002. Trichomonas vaginalis polymerase chain reaction compared with standard diagnostic and therapeutic protocols for detection and treatment of vaginal trichomoniasis. Clin Infect Dis 35(5):576-80; Wendel, K. A., E. J. Erbelding, C. A. Gaydos, and A. M. Rompalo. 2003. Use of urine polymerase chain reaction to define the prevalence and clinical presentation of Trichomonas vaginalis in men attending an STD clinic. Sex Transm Infect 79(2):151-153).

Finally, the nucleic acid analogs may also be designed to detect general STDs. Examples of primers used to identify STDs by PCR based assays are known in the art (see, e.g.,Mitrani-Rosenbaum, S., R. Tsvieli, O. Lavie, R. Boldes, E. Anteby, S. Shimonovitch, T. Lazarovitch, and A. Friedmann. 1994. Simultaneous detection of three common sexually transmitted agents by polymerase chain reaction. Am J Obstet Gynecol 171(3):784-90).

### H. Antibiotic Resistance

The methods, compositions, and assay systems may be used to detect the presence or change in expression of genes conferring antibiotic resistance. The target polynucleotide may correspond to genes that confer antibiotic resistance to a pathogen. Alternatively, the target polynucleotide may correspond to a gene in a host having an altered metabolism that may affect the efficacy of the antibiotic.

Examples of genes expressing antibiotic resistance can be found in the following references. Examples are provide but not limited to the examples provided. Genes encoding such as Beta Lactamases convey resistance to antibiotics including, but not limited to, penicillian, errythromycin, cefotaxime, ampicillin, piperacillin, cefoperazone, ceftazidime, cefepime, moxalactam, imipenem (see, e.g., Livermore, D.M. 1995. β-Lactamases in Laboratory and Clinical Resistance. Clin. Microbiol. Reviews. 8:557-584., Hsueh, P., Teng, L., Lee, L., Yang, P., Ho, S., and Luh, K, 1999. Dissemination of High-Level Penicillin-, Extended-Spectrum Cephalosporin-, and Erythromycin-Resistant Streptococcus pneumoniae Clones in Taiwan. J. Clin. Microbiol. 37:221-224., Setchanova, L., and Tomasz, A. 1999. Molecular Characterization of Penicillin-Resistant Streptococcus pneumoniae Isolates from Bulgaria. J. Clin. Microbiol. 37:638-648., Wichelhaus, T.A., Kern, S., Schäfer, V., Brade, V. 1999. Rapid Detection of Epidemic Strains of Methicillin-Resistant Staphylococcus aureus. J. Clin. Microbiol. 37:690-693., and Jacoby G.A. 1994. Genetics of Extended-Spectrum Beta-Lactamases. Eur.J. Clin. Microbiol. Infect. Dis.13:suppl.1:2-11).

Other non-limiting examples of antibiotic confering genes include quinolones resistance genes, which convey resistance to antibiotics such as ceprofloxacin, ofloxacin, norfloxacin, enoxacin, sparfloxacin (see, e.g.,Margerrison, E. E. C., Hopewell, R., and Fisher, L.M. 1992. Nucleotide Sequence of the Staphylococcus aureus gyrB-gyrA Locus Encoding the DNA Gyrase A and B Proteins. J. Bacteriol. 174:1596-1603., McWhinney, P.H.M., Patel, S., Whiley, R.A., Hardie, J.M., Gillespie, S.H., and Kibbler, C.C. 1993. Activities of Potential Therapeutic and Prophylactic Antibiotics against Blood Culture Isolates of Viridans Group Streptococci from Neutropenic Patients Receiving Ciprofloxacin. Antimicrob. Agents. Chemother. 37:2493-2495., and Yoshida, H., Bogaki, M., Nakamura, S., Ubukata, K., and Konno, M. 1990. Nucleotide Sequence and Characterization of the Staphylococcus aureus norA Gene, Which Confers Resistance to Quinolones. J. Bacteriol. 172:6942-6949).

Additional examples of antibiotic resistance genes include glycopeptide resistance genes, which convey resistance to antibiotics such as vancomycin and teicoplanin (see, e.g., Tremlett, C.H., Brown, D.F.J, and Woodford, N. 1999. Variation in Structure and Location of VanA Glycopeptide Resistance Elements among Enterococci from a Single Patient. J. Clin. Microbiol. 37:818-820., and Arthur, M., Depardieu, F., Molinas, C., Reynolds, P., and Courvalin, P. 1995. The vanZ gene of Tn1546 from Enterococcus faecium BM4147 confers resistance to teicoplanin. Gene, 154 (1995) 87-92.) aminoglycoside resistance genes which convey resistance to antibiotics such as tobramycin, gentamicin, strepomycin, kanamycin, amikacin (Galimand M., Lambert, T., Gerbaud, G., and Courvalin, P. 1993. Characterization of the aac(6')-Ib gene Encoding an Aminoglycoside 6'-N-Acetyltransferase in Pseudomonas aeruginosa BM2656. Antimicrob. Agents. Chemother. 37:1456-1462., Lambert, T., Gerbaud, G., and Courvalin, P. 1994. Characterization of Transposon Tn1528, Which Confers Amikacin Resistance by Synthesis of Aminoglycoside 3'-O-Phosphotransferase Type VI. Antimicrob. Agents. Chemother. 38:702-706,and Shaw, K.J., Munayyer, H., Rather, P.N., Hare, R.S., and Miller, G.H. 1993. Nucleotide Sequence Analysis and DNA Hybridization Studies of the ant(4')-IIa Gene from Pseudomonas aeruginosa. Antimicrob. Agents. Chemother. 37:708-714.

In some cases there are particular types of resistance that are of high concern. These include methicillin resistant Staphylococcus aureus (see, e.g., Ito, T., K. Okuma, X. X. Ma, H. Yuzawa, and K. Hiramatsu. 2003. Insights on antibiotic resistance of Staphylococcus aureus from its whole genome: genomic island SCC. Drug Resist Update 6(1):41-52, Kuroda, M., T. Ohta, I. Uchiyama, T. Baba, H. Yuzawa, I. Kobayashi, L. Cui, A. Oguchi, K. Aoki, Y. Nagai, J. Lian, T Ito, M. Kanamori, H. Matsumaru, A. Maruyama, H. Murakami, A. Hosoyama, Y. Mizutani-Ui, N. K. Takahashi, T. Sawano, R. Inoue, C. Kaito, K. Sekimizu, H. Hirakawa, S. Kuhara, S. Goto, J. Yabuzaki, M. Kanehisa, A. Yamashita, K. Oshima, K. Furuya, C. Yoshino, T. Shiba, M. Hattori, N. Ogasawara, H. Hayashi, and K. Hiramatsu. 2001. Whole genome sequencing of meticillin-resistant Staphylococcus aureus. Lancet 357(9264):1225-40, and Skurray, R. A., and N. Firth. 1997. Molecular evolution of multiply-antibioticresistant staphylococci. Ciba Found Symp 207:167-83').

Other antibiotic resistance conferring genes that may be detected include tuberculosis antibiotic resistance genes (see, e.g., Mokrousov, I., T. Otten, M. Filipenko, A. Vyazovaya, E. Chrapov, E. Limeschenko, L. Steklova, B. Vyshnevskiy, and O. Narvskaya. 2002. Detection of isoniazid-resistant Mycobacterium tuberculosis strains by a multiplex allele-specific PCR assay targeting katG codon 315 variation. J Clin Microbiol 40(7):2509-12; Gong, G., H. Lee, G. H. Kang, Y. H. Shim, J. Huh, and S. K. Khang. 2002. Nested PCR for diagnosis of tuberculous lymphadenitis and PCR-SSCP for identification of rifampicin resistance in fine-needle aspirates. Diagn Cytopathol 26(4):228-31; Garcia de Viedma, D., M. del Sol Diaz Infantes, F. Lasala, F. Chaves, L. Alcala, and E. Bouza. 2002. New real-time PCR able to detect in a single tube multiple rifampin resistance mutations and high-level isoniazid resistance mutations in Mycobacterium tuberculosis. J Clin Microbiol 40(3):988-95).

### I. Genetic Screening for a Predisposition for Drug Responses

The methods, compositions, and assay systems may be used to detect genes conferring a predisposition to drug responses. The target polynucleotide may correspond to any polynucleotide sequence that confers a predisposition to the drug response. The target polynucleotide may correspond to sequences involved in predisposition to drugs that affect the cardiovascular system (see, e.g., Dudley, C., B. Keavney, B. Casadei, J. Conway, R. Bird, and P. Ratcliffe. 1996. Prediction of patient responses to antihypertensive drugs using genetic polymorphisms: investigation of renin-angiotensin system genes. J Hypertens 14(2):259-62; Lima, P. R., J. A. Gontijo, J. B. Lopes de Faria, F. F. Costa, and S. T. Saad. 1997. Band 3 Campinas: a novel splicing mutation in the band 3 gene (AE1) associated with hereditary spherocytosis, hyperactivity of Na+/Li+ countertransport and an abnormal renal bicarbonate handling. Blood 90(7):2810-8; Sakaeda, T., T. Nakamura, M. Horinouchi, M. Kakumoto, N. Ohmoto, T. Sakai, Y. Morita, T. Tamura, N. Aoyama, M. Hirai, M. Kasuga, and K. Okumura. 2001. MDR1 genotype-related pharmacokinetics of digoxin after single oral administration in healthy Japanese subjects. Pharm Res 18(10):1400-4.). The target polynucleotide may also correspond to genes and other polynucleotide sequences implicated a predisposition to psychiatric drugs (see, e.g., Nikoloff, D., J. C. Shim, M. Fairchild, N. Patten, B. A. Fijal, W. H. Koch, A. MacPherson, D. Flockhart, Y. R. Yoon, J. S. Yoon, Y. H. Kim, and J. G. Shin. 2002. Association between CYP2D6 genotype and tardive dyskinesia in Korean schizophrenics. Pharmacogenomics J 2(6):400-7; Bertilsson, L., M. L. Dahl, and G. Tybring. 1997. Pharmacogenetics of antidepressants: clinical aspects. Acta Psychiatr Scand Suppl 391:14-21.; Chen, S., W. H. Chou, R. A. Blouin, Z. Mao, L. L. Humphries, Q. C. Meek, J. R. Neill, W. L. Martin, L. R. Hays, and P. J. Wedlund. 1996. The cytochrome P450 2D6 (CYP2D6) enzyme polymorphism: screening costs and influence on clinical outcomes in psychiatry. Clin Pharmacol Ther 60(5):522-34). The target polynucleotide may also correspond to gene sequences, or other polynucleotide sequences, corresponding to a predisposition to respond to analgesic drugs (see, e.g., Torres-Galvan, M. J., N. Ortega, F. Sanchez-Garcia, C. Blanco, T. Carrillo, and J. Quiralte. 2001. LTC4-synthase A-444C polymorphism: lack of association with NSAID- induced isolated periorbital angioedema in a Spanish population. Ann Allergy Asthma Immunol 87(6):506-10.).

### J. Genes Implicated in Effective Drug Response

The methods, compositions, and assays of the instant invention may be used in genetic testing is used a potential indication of a reaction to medication. The methods, compositions, and assays may be used to determine if a current treatment is working, or whether a resistance (or tolerance) is developing. Gene expression testing could be used to determine whether a tumor is becoming, or will become, resistant to a treatment. Gene based diagnostic testing may also includes pre-screening the likelihood of developing a disease or disorder in the future, pre-screening for genetic pre-disposition certain types of cancer, and identifying genetic polymorphisms.

Nucleic acid analogs may be designed to detect the presence and expression of specific genes. In one aspect, the methods may be used to determine the expression level of enzymes that can metabolize the associated drug. Polymorphic enzymes alter the effects of the drug and thus alter their ability to be used as a treatment. Genetic polymorphisms in the genes that influence the response of the associated drug, and thus affect the drugs ability to be used as a treatment, may be detected.

In one embodiment, nucleic acid analogs are designed to detect the sequences, or fragments of the sequences, encoding Cytochrome CYP2D6. Cytochrome CYP2D6 can metabolize Debrisoquin, which is used to treat antihypertension. Elevated expression of CYP2D6 indicates resistance to Debrisoquin (see, e.g., Mahgoub, A., J. R. Idle, L. G. Dring, R. Lancaster, and R. L. Smith. 1977. Polymorphic hydroxylation of Debrisoquine in man. Lancet 2(8038):584-6; and Wennerholm, A., I. Johansson, A. Y. Massele, M. Lande, C. Alm, Y. Aden-Abdi, M. L. Dahl, M. Ingelman-Sundberg, L. Bertilsson, and L. L. Gustafsson. 1999. Decreased capacity for debrisoquine metabolism among black Tanzanians: analyses of the CYP2D6 genotype and phenotype. Pharmacogenetics 9(6):707-14).

The presence or expression of Cytochrome CYP2D6 may be used to determine resistance to Sparteine, which is used to treat post-operative pain (see, e.g., Eichelbaum, M., N. Spannbrucker, B. Steincke, and H. J. Dengler. 1979. Defective N-oxidation of sparteine in man: a new pharmacogenetic defect. Eur J Clin Pharmacol 16(3):183-7; Broly, F., D. Marez, N. Sabbagh, M. Legrand, S. Millecamps, J. M. Lo Guidice, P. Boone, and U. A. Meyer. 1995. An efficient strategy for detection of known and new mutations of the CYP2D6 gene using single strand conformation polymorphism analysis. Pharmacogenetics 5(6):373-84).

The presence or expression of Cytochrome CYP2D6 may be used to determine resistance to Nortriptyline which is used to treat depression or cardiovascular disease (see, e.g., Dalen, P., M. L. Dahl, M. L. Ruiz, J. Nordin, and L. Bertilsson. 1998. 10-Hydroxylation of nortriptyline in white persons with 0, 1, 2, 3, and 13 functional CYP2D6 genes. Clin Pharmacol Ther 63(4):444-52; Yue, Q. Y., Z. H. Zhong, G. Tybring, P. Dalen, M. L. Dahl, L. Bertilsson, and F. Sjoqvist. 1998. Pharmacokinetics of nortriptyline and its 10-hydroxy metabolite in Chinese subjects of different CYP2D6 genotypes. Clin Pharmacol Ther 64(4):384-90).

The presence or expression of Cytochrome P459 may be used to determine altered codeine metabolism (see, e.g., Sindrup, S. H., and K. Brosen. 1995. The pharmacogenetics of codeine hypoalgesia. Pharmacogenetics 5(6):335-46; Mortimer, O., K. Persson, M. G. Ladona, D. Spalding, U. M. Zanger, U. A. Meyer, and A. Rane. 1990. Polymorphic formation of morphine from codeine in poor and extensive metabolizers of dextromethorphan: relationship to the presence of immunoidentified cytochrome P-450IID1. Clin Pharmacol Ther 47(1):27-35).

In another embodiment, nucleic acid analogs are designed to detect the sequences, or fragments of the sequences, encoding Cytochrome CYP2C9. Cytochrome CYP2C9 can metabolize Warfarin which is used as an anticoagulant (see, e.g., Aithal, G. P., C. P. Day, P. J. Kesteven, and A. K. Daly. 1999. Association of polymorphisms in the cytochrome P450 CYP2C9 with warfarin dose requirement and risk of bleeding complications. Lancet 353(9154):717-9; Loebstein, R., H. Yonath, D. Peleg, S. Almog, M. Rotenberg, A. Lubetsky, J. Roitelman, D. Harats, H. Halkin, and D. Ezra. 2001. Interindividual variability in sensitivity to warfarin--Nature or nurture? Clin Pharmacol Ther 70(2):159-64).

Cytochrome CYP2C9 can also metabolize Phenytoin which is used to treat brain injury (see, e.g., van der Weide, J., L. S. Steijns, M. J. van Weelden, and K. de Haan. 2001. The effect of genetic polymorphism of cytochrome P450 CYP2C9 on phenytoin dose requirement. Pharmacogenetics 11(4):287-91; Brandolese, R., M. G. Scordo, E. Spina, M. Gusella, and R. Padrini. 2001. Severe phenytoin intoxication in a subject homozygous for CYP2C9*3. Clin Pharmacol Ther 70(4):391-4).

In addition, Cytochrome CYP2C19 can metabolize Omeprazole which I used to treat acid-reflux disease (see, e.g., Balian, J. D., N. Sukhova, J. W. Harris, J. Hewett, L. Pickle, J. A. Goldstein, R. L. Woosley, and D. A. Flockhart. 1995. The hydroxylation of omeprazole correlates with S-mephenytoin metabolism: a population study. Clin Pharmacol Ther 57(6):662-9; Tybring, G., Y. Bottiger, J. Widen, and L. Bertilsson. 1997. Enantioselective hydroxylation of omeprazole catalyzed by CYP2C19 in Swedish white subjects. Clin Pharmacol Ther 62(2):129-37).

In a further embodiment, nucleic acid analogs are designed to detect the sequences, or fragments of the sequences, encoding dihydropyrimidine dehydrogenase. Dihydropyrimidine dehydrogenase can metabolize Fluorouracil, which is used to treat antineoplastic diseases (various cancer treatments) (see, e.g, Tuchman, M., J. S. Stoeckeler, D. T. Kiang, R. F. O'Dea, M. L. Ramnaraine, and B. L. Mirkin. 1985. Familial pyrimidinemia and pyrimidinuria associated with severe fluorouracil toxicity. N Engl J Med 313(4):245-9; Diasio, R. B., T. L. Beavers, and J. T. Carpenter. 1988. Familial deficiency of dihydropyrimidine dehydrogenase. Biochemical basis for familial pyrimidinemia and severe 5-fluorouracil-induced toxicity. J Clin Invest 81(1):47-51).

Nucleic acid analogs may also be designed to detect the sequences, or fragments of the sequences, encoding butyrylcholinesterase. Butyrylcholinesterase can metabolize Succinylcholine which is used as a muscle relaxant (see, e.g., Lockridge, O. 1990. Genetic variants of human serum cholinesterase influence metabolism of the muscle relaxant succinylcholine. Pharmacol Ther 47(1):35-60; and Ceppa, F., S. Gidenne, A. Benois, E. Fontan, and P. Burnat. 2002. Rapid identification of atypical variant of plasma butyrylcholinesterase by PCR. Clin Chem Lab Med 40(8):799-801.

Nucleic acid analogs may also be designed to detect the sequences, or fragments of the sequences, encoding N-Acetyltransferase 2. N-Acetyltransferase 2 can metabolize Isoniazid which is used to treat tuberculosis (see, e.g., Furet, Y., Y. Bechtel, C. Le Guellec, P. R. Bechtel, E. Autret-Leca, and G. Paintaud. 2002. Clinical relevance of N-acetyltransferase type 2 (NAT2) genetic polymorphism, Therapie 57(5):427-31; Kita, T., Y. Tanigawara, S. Chikazawa, H. Hatanaka, T. Sakaeda, F. Komada, S. Iwakawa, and K. Okumura. 2001. N-Acetyltransferase2 genotype correlated with isoniazid acetylation in Japanese tuberculous patients. Biol Pharm Bull 24(5):544-9; and Hiratsuka, M. 2002. Development of simplified and rapid detection assay for genetic polymorphisms influencing drug response and its clinical applications. Yakugaku Zasshi 122(7):451-63).

N-Acetyltransferase 2 can also metabolize Hydralazine which is used to treat antihypertensive (see, e.g., Timbrell, J. A., S. J. Harland, and V. Facchini. 1980. Polymorphic acetylation of hydralazine. Clin Pharmacol Ther 28(3):350-5; and

Zschieschang, P., F. Hiepe, E. Gromnica-Ihle, I. Roots, and I. Cascorbi. 2002. Lack of association between arylamine N-acetyltransferase 2 (NAT2) polymorphism and systemic lupus erythematosus. Pharmacogenetics 12(7):559-63).

N-Acetyltransferase 2 can metabolize Procainamide, which is used to treat antiarrhythmic (see, e.g., Reidenberg, M. M., D. E. Drayer, M. Levy, and H. Warner. 1975. Polymorphic acetylation procainamide in man. Clin Pharmacol Ther 17(6):722-30; and Hickman, D., J. R. Palamanda, J. D. Unadkat, and E. Sim. 1995. Enzyme kinetic properties of human recombinant arylamine N-acetyltransferase 2 allotypic variants expressed in Escherichia coli. Biochem Pharmacol 50(5):697-703).

In another aspect, nucleic acid analogs may also be designed to detect the sequences, or fragments of the sequences, encoding uridine disphosphate-glucuronosyltransferase 1A1. Uridine disphosphate-glucuronosyltransferase 1A1 can metabolize Irinotecan which is used to treat colorectal cancer (see, e.g., Iyer, L., D. Hall, S. Das, M. A. Mortell, J. Ramirez, S. Kim, A. Di Rienzo, and M. J. Ratain. 1999. Phenotype-genotype correlation of in vitro SN-38 (active metabolite of irinotecan) and bilirubin glucuronidation in human liver tissue with UGT1A1 promoter polymorphism. Clin Pharmacol Ther 65(5):576-82; Ando, Y., H. Saka, G. Asai, S. Sugiura, K. Shimokata, and T. Kamataki. 1998. UGT1A1 genotypes and glucuronidation of SN-38, the active metabolite of irinotecan. Ann Oncol 9(8):845-7).

Uridine disphosphate-glucuronosyltransferase 1A1 can also metabolize Bilirubin which is used to treat Gilbert's syndrome, a mild liver disorder (see, e.g., Bosma, P. J., J. R. Chowdhury, C. Bakker, S. Gantla, A. de Boer, B. A. Oostra, D. Lindhout, G. N. Tytgat, P. L. Jansen, R. P. Oude Elferink, and et al. 1995. The genetic basis of the reduced expression of bilirubin UDP-glucuronosyltransferase 1 in Gilbert's syndrome. N Engl J Med 333(18):1171-5; Kim, Y. H., J. E. Yeon, G. M. Jung, H. J. Kim, J. S. Kim, K. S. Byun, Y. T. Bak, and C. H. Lee. 2002. A study of polymorphism in UDP-glucuronosyltransferase 1 (UGT-1A1) promoter gene in Korean patients with Gilbert's syndrome. Taehan Kan Hakhoe Chi 8(2):132-8).

Nucleic acid analogs may also be designed to detect the sequences, or fragments of the sequences, encoding thiopurine S-methyltransferase. Thiopurine S-methyltransferase can metabolize Mercaptopurine which is used to treat Crohn's disease

(see, e.g., Weinshilboum, R. M. 1992. Methylation pharmacogenetics: thiopurine methyltransferase as a model system. Xenobiotica 22(9-10):1055-71; and Wennerholm, A., I. Johansson, A. Y. Massele, M. Lande, C. Alm, Y. Aden-Abdi, M. L. Dahl, M. Ingelman-Sundberg, L. Bertilsson, and L. L. Gustafsson. 1999. Decreased capacity for debrisoquine metabolism among black Tanzanians: analyses of the CYP2D6 genotype and phenotype. Pharmacogenetics 9(6):707-14).

Thiopurine S-methyltransferase can also metabolize Azathioprine which is also used to treat Crohn's disease (see, e.g., Kaskas, B. A., E. Louis, U. Hindorf, E. Schaeffeler, J. Deflandre, F. Graepler, K. Schmiegelow, M. Gregor, U. M. Zanger, M. Eichelbaum, and M. Schwab. 2003. Safe treatment of thiopurine S-methyltransferase deficient Crohn's disease patients with azathioprine. Gut 52(1):140-2; and Marra, C. A., J. M. Esdaile, and A. H. Anis. 2002. Practical Pharmacogenetics: The Cost Effectiveness of Screening for Thiopurine s-Methyltransferase Polymorphisms in Patients with Rheumatological Conditions Treated with Azathioprine. J Rheumatol 29(12):2507-12).

Nucleic acid analogs may also be designed to detect the sequences, or fragments of the sequences,encoding catechol O-methyltransferase. Catechol O-methyltransferase can metabolize Levodopa, which is used to treat Parkinson's disease (see, e.g., Reilly, D. K., L. Rivera-Calimlim, and D. Van Dyke. 1980. Catechol-O-methyltransferase activity: a determinant of levodopa response. Clin Pharmacol Ther 28(2):278-86; and Wennerholm, A., I. Johansson, A. Y. Massele, M. Lande, C. Alm, Y. Aden-Abdi, M. L. Dahl, M. Ingelman-Sundberg, L. Bertilsson, and L. L. Gustafsson. 1999. Decreased capacity for debrisoquine metabolism among black Tanzanians: analyses of the CYP2D6 genotype and phenotype. Pharmacogenetics 9(6):707-14).

Nucleic acid analogs may also be designed to detect the genetic polymorphisms associated with drug resistance. In one embodiment, the Nucleic acid analogs may be designed to detect ACE polymorphisms which can influence the response of the ACE inhibitor antihypertensice, used to treat heart failure (see, e.g., Jacobsen, P., K. Rossing, P. Rossing, L. Tarnow, C. Mallet, O. Poirier, F. Cambien, and H. H. Parving. 1998. Angiotensin converting enzyme gene polymorphism and ACE inhibition in diabetic nephropathy. Kidney Int 53(4):1002-6; Kohno, M., K. Yokokawa, M. Minami, H. Kano, K. Yasunari, T. Hanehira, and J. Yoshikawa. 1999. Association between angiotensin-converting enzyme gene polymorphisms and regression of left ventricular hypertrophy in patients treated with angiotensin-converting enzyme inhibitors. Am J Med 106(5):544-9).

ACE polymorphisms can also influence the response of Fluvastatin used to treat Coronary atherosclerosis (see, e.g., Marian, A. J., F. Safavi, L. Ferlic, J. K. Dunn, A. M. Gotto, and C. M. Ballantyne. 2000. Interactions between angiotensin-I converting enzyme insertion/deletion polymorphism and response of plasma lipids and coronary atherosclerosis to treatment with fluvastatin: the lipoprotein and coronary atherosclerosis study. J Am Coll Cardiol 35(1):89-95; Bosse, Y., M. C. Vohl, M. Dumont, M. Brochu, J. Bergeron, J. P. Despres, and D. Prud'homme. 2002. Influence of the angiotensin-converting enzyme gene insertion/deletion polymorphism on lipoprotein/lipid response to gemfibrozil. Clin Genet 62(1):45-52).

In another embodiment, the nucleic acid analogs may be designed to detect Arachidonate 5-lipoxygenase polymorphisms, which can influence the response of

Leukotriene inhibitors used as anti-inflammatories (see e.g. Fowler, S. J., I. P. Hall, A. M. Wilson, A. P. Wheatley, and B. J. Lipworth. 2002. 5-Lipoxygenase polymorphism and in-vivo response to leukotriene receptor antagonists. Eur J Clin Pharmacol 58(3):187-90; and Koshino, T., S. Takano, S. Kitani, N. Ohshima, Y. Sano, T. Takaishi, K. Hirai, K. Yamamoto, and Y. Morita. 1999. Novel polymorphism of the 5-lipoxygenase activating protein (FLAP) promoter gene associated with asthma. Mol Cell Biol Res Commun 2(1):32-5).

The nucleic acid analogs may be designed to detect β2- Adrenergic receptor polymorphisms which can influence the response of β2-Agonists used to treat asthma and pulmonary disease (see e.g. Liggett, S. B. 2000. beta(2)-adrenergic receptor pharmacogenetics. Am J Respir Crit Care Med 161(3 Pt 2):S197-201; Dishy, V., G. G. Sofowora, H. G. Xie, R. B. Kim, D. W. Byrne, C. M. Stein, and A. J. Wood. 2001. The effect of common polymorphisms of the beta2-adrenergic receptor on agonist-mediated vascular desensitization. N Engl J Med 345(14):1030-5.

The nucleic acid analogs may also be designed to detect Bradykinin B2 receptor polymorphisms which can influence the response of ACE-inhibitor used to treat heart failure (see e.g.Mukae, S., S. Aoki, S. Itoh, T. Iwata, H. Ueda, and T. Katagiri. 2000. Bradykinin B(2) receptor gene polymorphism is associated with angiotensin-converting enzyme inhibitor-related cough. Hypertension 36(1): 127-31; Mukae, S., S. Itoh, S. Aoki, T. Iwata, K.Nishio, R. Sato, and T. Katagiri. 2002. Association of polymorphisms of the renin-angiotensin system and bradykinin B2 receptor with ACE-inhibitor-related cough. J Hum Hypertens 16(12):857-63).

The nucleic acid analogs may be designed to detect Dopamine receptors (D2, D3, D4) polymorphisms which can influence the response of Antipsychotics (see e.g. Arranz, M. J., J. Munro, J. Birkett, A. Bolonna, D. Mancama, M. Sodhi, K. P. Lesch, J. F. Meyer, P. Sham, D. A. Collier, R. M. Murray, and R. W. Kerwin. 2000. Pharmacogenetic prediction of clozapine response. Lancet 355(9215):1615-6;1 Basile, V. S., M. Masellis, F. Badri, A. D. Paterson, H. Y. Meltzer, J. A. Lieberman, S. G. Potkin, F. Macciardi, and J. L. Kennedy. 1999. Association of the MscI polymorphism of the dopamine D3 receptor gene with tardive dyskinesia in schizophrenia. Neuropsychopharmacology 21(1):17-27).

Nucleic acid analogs may also be designed to detect Estrogen receptor-α polymorphisms which can influence the response of Conjugated estrogens and are used in Hormone-replacement therapy for menopause, osteoporosis (see e.g. Herrington, D. M., T. D. Howard, G. A. Hawkins, D. M. Reboussin, J. Xu, S. L. Zheng, K. B. Brosnihan, D. A. Meyers, and E. R. Bleecker. 2002. Estrogen-receptor polymorphisms and effects of estrogen replacement on high-density lipoprotein cholesterol in women with coronary disease. N Engl J Med 346(13):967-74; Ongphiphadhanakul, B., S. Chanprasertyothin, P. Payatikul, S. S. Tung, N. Piaseu, L. Chailurkit, S. Chansirikarn, G. Puavilai, and R. Rajatanavin. 2000. Oestrogen-receptor-alpha gene polymorphism affects response in bone mineral density to oestrogen in post-menopausal women. Clin Endocrinol (Oxf) 52(5):581-5).

The nucleic acid analogs may also be designed to detect Glycoprotein IIIa subunit of glycoprotein IIb/IIIa polymorphisms, which can influence the response of Aspirin or glycoprotein IIb/IIIa inhibitors used to treat myocardial infarction (see e.g., Michelson, A. D., M. I. Furman, P. Goldschmidt-Clermont, M. A. Mascelli, C. Hendrix, L. Coleman, J. Hamlington, M. R. Barnard, T. Kickler, D. J. Christie, S. Kundu, and P. F. Bray. 2000. Platelet GP IIIa P1(A) polymorphisms display different sensitivities to agonists. Circulation 101(9):1013-8; Andrioli, G., P. Minuz, P. Solero, S. Pincelli, R. Ortolani, S. Lussignoli, and P. Bellavite. 2000. Defective platelet response to arachidonic acid and thromboxane A(2) in subjects with P1(A2) polymorphism of beta(3) subunit (glycoprotein IIIa). Br J Haematol 110(4):911-8).

In a further embodiment, the nucleic acid analogs may be designed to detect Serotonin (5-hydroxytryptamine) transporter polymorphisms which can influence the response of Antidepressants used to treat Alzheimer's disease and depression (see e.g. Kim, D. K., S. W. Lim, S. Lee, S. E. Sohn, S. Kim, C. G. Hahn, and B. J. Carroll. 2000. Serotonin transporter gene polymorphism and antidepressant response. Neuroreport 11(1):215-9; and Smeraldi, E., R. Zanardi, F. Benedetti, D. Di Bella, J. Perez, and M. Catalano. 1998. Polymorphism within the promoter of the serotonin transporter gene and antidepressant efficacy of fluvoxamine. Mol Psychiatry 3(6):508-11).

The nucleic acid analogs may be designed to detect adducin polymorphisms which can influence the response of Diuretics used to treat myocardial infarction, hypertension (see, e.g., Sciarrone, M. T., P. Stella, C. Barlassina, P. Manunta, C. Lanzani, G. Bianchi, and D. Cusi. 2003. ACE and alpha-adducin polymorphism as markers of individual response to diuretic therapy. Hypertension 41(3):398-403; Psaty, B. M., N. L. Smith, S. R. Heckbert, H. L. Vos, R. N. Lemaitre, A. P. Reiner, D. S. Siscovick, J. Bis, T. Lumley, W. T. Longstreth, Jr., and F. R. Rosendaal. 2002. Diuretic therapy, the alpha-adducin gene variant, and the risk of myocardial infarction or stroke in persons with treated hypertension. Jama 287(13):1680-9).

The nucleic acid analogs may further be designed to detect apolipoprotein E (APOE) polymorphisms which can influence the response of Statins used to treat coronary arterial disease, cholesterol, atherosclerosis (see e.g. Ordovas, J. M., J. Lopez-Miranda, F. Perez-Jimenez, C. Rodriguez, J. S. Park, T. Cole, and E. J. Schaefer. 1995. Effect of apolipoprotein E and A-IV phenotypes on the low density lipoprotein response to HMG CoA reductase inhibitor therapy. Atherosclerosis 113(2):157-66; Gerdes, L. U., C. Gerdes, K. Kervinen, M. Savolainen, I. C. Klausen, P. S. Hansen, Y. A. Kesaniemi, and O. Faergeman. 2000. The apolipoprotein epsilon4 allele determines prognosis and the effect on prognosis of simvastatin in survivors of myocardial infarction : a substudy of the Scandinavian simvastatin survival study. Circulation 101(12):1366-71).

APOE polymorphisms which can influence the response of Tacrine used to treat Alzheimer's disease (see, e.g., Poirier, J., M. C. Delisle, R. Quirion, I. Aubert, M. Farlow, D. Lahiri, S. Hui, P. Bertrand, J. Nalbantoglu, B. M. Gilfix, and et al. 1995. Apolipoprotein E4 allele as a predictor of cholinergic deficits and treatment outcome in Alzheimer disease. Proc Natl Acad Sci U S A 92(26):12260-4).

Poirier, J. 1999. Apolipoprotein E: a pharmacogenetic target for the treatment of Alzheimer's disease. Mol Diagn 4(4):335-41; Soininen, H., O. Kosunen, S. Helisalmi, A. Mannermaa, L. Paljarvi, S. Talasniemi, M. Ryynanen, and P. Riekkinen, Sr. 1995. A severe loss of choline acetyltransferase in the frontal cortex of Alzheimer patients carrying apolipoprotein epsilon 4 allele. Neurosci Lett 187(2):79-82).

In another embodiment, the nucleic acid analogs may be designed to detect HLA polymorphisms which can influence the response of Abacavir used as an antiviral and to treat HIV (see, e.g., Mallal, S., D. Nolan, C. Witt, G. Masel, A. M. Martin, C. Moore, D. Sayer, A. Castley, C. Mamotte, D. Maxwell, I. James, and F. T. Christiansen. 2002. Association between presence of HLA-B*5701, HLA-DR7, and HLA-DQ3 and hypersensitivity to HIV-1 reverse-transcriptase inhibitor abacavir. Lancet 359(9308):727-32; and Hetherington, S., A. R. Hughes, M. Mosteller, D. Shortino, K. L. Baker, W. Spreen, E. Lai, K. Davies, A. Handley, D. J. Dow, M. E. Fling, M. Stocum, C. Bowman, L. M. Thurmond, and A. D. Roses. 2002. Genetic variations in HLA-B region and hypersensitivity reactions to abacavir. Lancet 359(9312):1121-2).

The nucleic acid analogs may also be designed to detect Cholesterol ester transfer protein (CETP) polymorphisms which can influence the response of Statins used to treat coronary related disease, cholestrol, and atherosclerosis (see, e.g., Kuivenhoven, J. A., J. W. Jukema, A. H. Zwinderman, P. de Knijff, R. McPherson, A. V. Bruschke, K. I. Lie, and J. J. Kastelein. 1998. The role of a common variant of the cholesteryl ester transfer protein gene in the progression of coronary atherosclerosis. The Regression Growth Evaluation Statin Study Group. N Engl J Med 338(2):86-93; Rump, P., R. P. Mensink, and G. Homstra. 2002. Interaction between a common variant of the cholesteryl ester transfer protein gene and the apolipoprotein E polymorphism: effects on plasma lipids and lipoproteins in a cohort of 7-year-old children. Nutr Metab Cardiovasc Dis 12(6):317-24; and van Venrooij, F. V., R. P. Stolk, J. D. Banga, T. P. Sijmonsma, A. van Tol, D. W. Erkelens, and G. M. Dallinga-Thie. 2003. Common cholesteryl ester transfer protein gene polymorphisms and the effect of atorvastatin therapy in type 2 diabetes. Diabetes Care 26(4):1216-23).

In another embodiment, the nucleic acid analogs may be designed to detect Ion Channel associated polymorphisms which can influence the response of Erythromycin, an antibiotic used to treat bacterial infections (see, e.g., Sesti, F., G. W. Abbott, J. Wei, K. T. Murray, S. Saksena, P. J. Schwartz, S. G. Priori, D. M. Roden, A. L. George, Jr., and S. A. Goldstein. 2000. A common polymorphism associated with antibiotic-induced cardiac arrhythmia. Proc Natl Acad Sci U S A 97(19):10613-8; Abbott, G. W., F. Sesti, I. Splawski, M. E. Buck, M. H. Lehmann, K. W. Timothy, M. T. Keating, and S. A. Goldstein. 1999. MiRP1 forms IKr potassium channels with HERG and is associated with cardiac arrhythmia. Cell 97(2):175-87).

The nucleic acid analogs may further be designed to detect Methylguanine methyltransferase polymorphisms which can influence the response of Carmustine used to treat glioma (see, e.g., Esteller, M., J. Garcia-Foncillas, E. Andion, S. N. Goodman, O. F. Hidalgo, V. Vanaclocha, S. B. Baylin, and J. G. Herman. 2000. Inactivation of the DNA-repair gene MGMT and the clinical response of gliomas to alkylating agents. N Engl J Med 343(19):1350-4; Gerson, S. L. 2002. Clinical relevance of MGMT in the treatment of cancer. J Clin Oncol 20(9):2388-99).

Nucleic acid analogs may be designed to detect Parkin polymorphisms which can influence the response of Levodopa used to treat parkinson's disease (see, e.g., Lucking, C. B., A. Durr, V. Bonifati, J. Vaughan, G. De Michele, T. Gasser, B. S. Harhangi, G. Meco, P. Denefle, N. W. Wood, Y. Agid, and A. Brice. 2000. Association between early-onset Parkinson's disease and mutations in the parkin gene. French Parkinson's Disease Genetics Study Group. N Engl J Med 342(21):1560-7; Bonifati, V., G. De Michele, C. B. Lucking, A. Durr, E. Fabrizio, G. Ambrosio, N. Vanacore, M. De Mari, R. Marconi, L. Capus, M. M. Breteler, T. Gasser, B. Oostra, N. Wood, Y. Agid, A. Filla, G. Meco, and A. Brice. 2001. The parkin gene and its phenotype. Italian PD Genetics Study Group, French PD Genetics Study Group and the European Consortium on Genetic Susceptibility in Parkinson's Disease. Neurol Sci 22(1):51-2).

Nucleic acid analogs may be designed to detect Prothrombin and factor V polymorphisms which can influence the response of oral contraceptives and indicate potential risk for development of thrombosis (see, e.g., Tassin, J., A. Durr, A. M. Bonnet, R. Gil, M. Vidailhet, C. B. Lucking, J. Y. Goas, F. Durif, M. Abada, B. Echenne, J. Motte, A. Lagueny, L. Lacomblez, P. Jedynak, B. Bartholome, Y. Agid, and A. Brice. 2000. Levodopa-responsive dystonia. GTP cyclohydrolase I or parkin mutations? Brain 123(Pt 6):1112-21; Martinelli, I., E. Sacchi, G. Landi, E. Taioli, F. Duca, and P. M. Mannucci. 1998. High risk of cerebral-vein thrombosis in carriers of a prothrombin-gene mutation and in users of oral contraceptives. N Engl J Med 338(25):1793-7; and Martinelli, I., E. Taioli, P. Bucciarelli, S. Akhavan, and P. M. Mannucci. 1999. Interaction between the G20210A mutation of the prothrombin gene and oral contraceptive use in deep vein thrombosis. Arterioscler Thromb Vasc Biol 19(3):700-3).

The nucleic acid analogs may also be designed to detect Stromelysin-1 polymorphisms which can influence the response of Statin used to treat atherosclerosis (see, e.g., Legnani, C., G. Palareti, G. Guazzaloca, B. Cosmi, B. Lunghi, F. Bernardi, and S. Coccheri. 2002. Venous thromboembolism in young women; role of thrombophilic mutations and oral contraceptive use. Eur Heart J 23(12):984-90; Liggett, S. B. 2000. beta(2)-adrenergic receptor pharmacogenetics. Am J Respir Crit Care Med 161(3 Pt 2):S197-201; Humphries, S. E., L. A. Luong, P. J. Talmud, M. H. Frick, Y. A. Kesaniemi, A. Pasternack, M. R. Taskinen, and M. Syvanne. 1998. The 5A/6A polymorphism in the promoter of the stromelysin-1 (MMP-3) gene predicts progression of angiographically determined coronary artery disease in men in the LOCAT gemfibrozil study. Lopid Coronary Angiography Trial. Atherosclerosis 139(1):49-56). Humphries, S., C. Bauters, A. Meirhaeghe, L. Luong, M. Bertrand, and P. Amouyel. 2002. The 5A6A polymorphism in the promoter of the stromelysin-1 (MMP3) gene as a risk factor for restenosis. Eur Heart J 23(9):721-5; and de Maat, M. P., J. W. Jukema, S. Ye, A. H. Zwinderman, P. H. Moghaddam, M. Beekman, J. J. Kastelein, A. J. van Boven, A. V. Bruschke, S. E. Humphries, C. Kluft, and A. M. Henney. 1999. Effect of the stromelysin-1 promoter on efficacy of pravastatin in coronary atherosclerosis and restenosis. Am J Cardiol 83(6):852-6).

### K. Genetically Modified Organisms

The methods disclosed herein also provide a rapid and sensitive diagnostic test for the presence genetically modified organisms (GMOs). Examples of GMOs include, but are not limited to, organisms in which one or more genes have been modified, added, or deleted. GMOs may be characterized by the presence of one or more specific gene, absence of one or more specific genes, specific alteration, or altered expression of one or more specific genes. Nucleic acid analogs may be designed to complement target polynucleotides characteristic of the of the GMOs. The presence and number of GMOs may be measured using the methods of the reaction.

### L. Non-Indigenous Fluora and Fauna

The methods disclosed herein also provide a rapid and sensitive diagnostic test for the presence and enumeration of non-indigenous fluora and fauna. Organisms that are not indigenous to a particular region present environmental and biological hazards to indigenous fluora and fauna. Nucleic acid analogs may be designed to complement target polynucleotides characteristic of the non-indigenous fluora and fauna. The presence and number of non-indigenous fluora and fauna may be measured using the methods of the reaction.

### M. Agricultural Applications

The methods disclosed herein also provide a rapid and sensitive diagnostic test for the presence and enumeration of specific plants and plant varieties. Nucleic acid analogs may be designed to complement target polynucleotides characteristic of the specific plants or plant varieties. The presence and number of the plants and varieties may be measured using the methods of the reaction.

### N. Veterinary Applications

The methods, compositions, and assays disclosed herein also provide a rapid and sensitive diagnostic test in veterinary applications. Nucleic acid analogs may be designed to complement target polynucleotides that correspond to animal based infections or to genetic diseases or disorders.

In one embodiment, the methods, compositions, and assays may be used to detect the presence of or infection by rabies virus. Nucleic acid analogs may be designed to have the sequences of primers used for PCR-based detection of rabies. Examples of primers used to identify rabies virus by PCR based assays are known in the art (see, *e.g*., Ito M, Itou T, Shoji Y, Sakai T, Ito FH, Arai YT, Takasaki T, Kurane I. Discrimination between dog-related and vampire bat-related rabies viruses in Brazil by strain-specific reverse transcriptase-polymerase chain reaction and restriction fragment length polymorphism analysis. J Clin Virol. 2003 Apr;26(3):317-30; Black EM, Lowings JP, Smith J, Heaton PR, McElhinney LM. A rapid RT-PCR method to differentiate six established genotypes of rabies and rabies-related viruses using TaqMan technology. J Virol Methods. 2002 Aug;105(1):25-35; David D, Yakobson B, Rotenberg D, Dveres N, Davidson I, Stram Y. Rabies virus detection by RT-PCR in decomposed naturally infected brains. Vet Microbiol. 2002 Jun 20;87(2):111-8; Ito M, Itou T, Sakai T, Santos MF, Arai YT, Takasaki T, Kurane I, Ito FH. Detection of rabies virus RNA isolated from several species of animals in Brazil by RT-PCR,J Vet Med Sci. 2001 Dec;63(12):1309-13).

In another embodiment, the methods, compositions, and assays may be used to detect Porcine Stress Syndrome. Porcine Stress Syndrome is caused by a mutation of the ryanodine-receptor (RYR-1)-gene--homozygous or heterozygous. Nucleic acid analogs may be designed to have the sequences of primers used for PCR-based detection of Porcine Stress Syndrome. Examples of primers used to identify Porcine Stress Syndrome by PCR based assays are known in the art (see, *e.g*., Lee SH, Cho KK, Kang SK, Kim CW, Park HC, Choy YH, Choi YJ. Detection of pigs resistant to post-weaning diarrheaea, oedema disease and porcine stress syndrome by allele-specific polymerase chain reaction. Anim Genet. 2002 Jun;33(3):237-9, Bu H, Liu J, Li SF. The RYR1 genotype of Chinese inbred pigs. Zhongguo Xiu Fu Chong Jian Wai Ke Za Zhi. 2000 Sep;14(5):311-4).

The methods, compositions, and assays may be used to detect Hyperkalemic Periodic Paralysis Disease (HyPP). HyPP is a genetic disease in American Quarter horses or crossbreds. Data imply that HyPP is inherited as a codominant genetic defect, because the homozygotes showed more severe clinical signs of disease than heterozygotes. Nucleic acid analogs may be designed to have the sequences of primers used for PCR-based detection of HyPP.

In another embodiment, the methods, compositions, and assays may be used to detect FeCV (Feline Coronavirus). Nucleic acid analogs may be designed to have the sequences of primers used for PCR-based detection of Feline Coronavirus. Examples of primers used to identify Feline Coronavirus by PCR based assays are known in the art (see, e.g., Kennedy M, Citino S, McNabb AH, Moffatt AS, Gertz K, Kania S. Detection of feline coronavirus in captive Felidae in the USA. J Vet Diagn Invest. 2002 Nov;14(6):520-2; Addie DD, Jarrett O. Use of a reverse-transcriptase polymerase chain reaction for monitoring the shedding of feline coronavirus by healthy cats. Vet Rec. 2001 May 26;148(21):649-53; Herrewegh AA, Mahler M, Hedrich HJ, Haagmans BL, Egberink HF, Horzinek MC, Rottier PJ, de Groot RJ. Persistence and evolution of feline coronavirus in a closed cat-breeding colony. Virology. 1997 Aug 4;234(2):349-63).

In another embodiment, the methods, compositions, and assays may be used to detect the presence of or infection by FIP virus, a mutant of the ubiquitous feline enteric coronavirus (FECV). Nucleic acid analogs may be designed to have the sequences of primers used for PCR-based detection of FIP virus. Examples of primers used to identify FIP virus by PCR based assays are known in the art (see, e.g., Gunn-Moore DA, Gruffydd-Jones TJ, Harbour DA. Detection of feline coronaviruses by culture and reverse transcriptase-polymerase chain reaction of blood samples from healthy cats and cats with clinical feline infectious peritonitis. Vet Microbiol. 1998 Jul;62(3):193-205; Kennedy M, Boedeker N, Gibbs P, Kania S.Deletions in the 7a ORF of feline coronavirus associated with an epidemic of feline infectious peritonitis. Vet Microbiol. 2001 Aug 8;81(3):227-34).

The methods, compositions, and assays may also be used to detect the presence of or infection by Equine infectious anemia virus (EIAV). Nucleic acid analogs may be designed to have the sequences of primers used for PCR-based detection of EIAV. Examples of primers used to identify EIAV by PCR based assays are known in the art (see, e.g., Cook RF, Cook SJ, Li FL, Montelaro RC, Issel CJ. Development of a multiplex real-time reverse transcriptase-polymerase chain reaction for equine infectious anemia virus (EIAV). J Virol Methods. 2002 Aug;105(1):171-9; Langemeier JL, Cook SJ, Cook RF, Rushlow KE, Montelaro RC, Issel CJ. Detection of equine infectious anemia viral RNA in plasma samples from recently infected and long-term inapparent carrier animals by PCR. J Clin Microbiol. 1996 Jun;34(6):1481-7; Nagaraj an MM, Simard C. Detection of horses infected naturally with equine infectious anemia virus by nested polymerase chain reaction. J Virol Methods. 2001 May;94(1-2):97-109.

The methods, compositions, and assays may also be used to detect the presence of or infection by Brucella ovis. Nucleic acid analogs may be designed to have the sequences of primers used for PCR-based detection of Brucella ovis. Examples of primers used to identify Brucella ovis by PCR based assays are known in the art (see, e.g.,Manterola L, Tejero-Garces A, Ficapal A, Shopayeva G, Blasco JM, Marin CM, Lopez-Goni I. Evaluation of a PCR test for the diagnosis of Brucella ovis infection in semen samples from rams., Vet Microbiol. 2003 Mar 20;92(1-2):65-72; Hamdy ME, Amin AS. Detection of Brucella species in the milk of infected cattle, sheep, goats and camels by PCR. Vet J. 2002 May;163(3):299-305; Bricker BJ, Halling SM. Differentiation of Brucella abortus bv. 1, 2, and 4, Brucella melitensis, Brucella ovis, and Brucella suis bv. 1 by PCR.J Clin Microbiol. 1994 Nov;32(11):2660-6).

The foregoing examples are only exemplary. Target polynucleotides may include polynucleotides corresponding to other antibiotic resistant conferring genes known in the art.

### VIII. Kits

In one aspect, the present application provides a kit for detecting target polynucleotides. A kit may include one or more reagents useful in the present invention, for example, dyes, nucleic acid anlogs, sources of light stimulus, buffers, standards used for controls, keys showing positives and negatives of control samples for interpreting reaction results. Optionally, one or more buffers are provided. The kits may further include suitable packaging of the respective compositions, instructions, and/or other optional components as disclosed below.

### A. Dyes

The kits provided herein include one or more dyes.

The dyes can be provided in pre-packages amounts, or can be provided in a single tube from which aliquots can be apportioned.

The dyes may be further packaged in any suitable packaging for segregation from other components of the kit and to facilitate dispensing of the composition.

### B. Nucleic Acid Analogs

The kits may also include one or more nucleic acid analogs.

The nucleic acid analog may be any nucleic acid analog, as described herein. In one embodiment, the nucleic acid analog is an LNA. In another embodiment, the nucleic acid analog is a PNA.

The nucleic acid analog may have any sequence that is complementary or fully complementary to a target nucleic acid sequence. The sequence may be any sequence known in the art. In one embodiment, the nucleic acid analog has a sequence disclosed herein.

The nucleic acid analog may be provided in any suitable container, and may be prealiquoted into usable amounts, or in a single tube to be apportioned. The container may be further packaged in any suitable packaging for segregation from other components of the kit and to facilitate dispensing of the cleansing composition. In another embodiment, two or more the nucleic acid analog sequences may be contained in the same package.

The kits may also include a vehicle to facilitate effective hybridization of the nucleic acid analog to the target polynucleotide, such as salmon sperm DNA.

### C. Source of Light Stimulus

The kits may optionally further include a source of light stimulus. Non-limiting examples of light sources include the Sylvania Cool White T8-CW, General Electric T8 - C50, and Fritz Aurora 50/50, a Sylvania dulux S9W CF9DS/blue and a Osram F9TT/50K.

### D. Polynucleotide Manipulating Components

The kits may also include components used to manipulate polynucleotides, such as buffers, enzymes, columns, and other materials.

The buffers, enzymes, columns, and other materials can include those that are used to lyse cells or extract DNA or RNA from a cell. The buffers, enzymes, columns, and other materials can also include components used to manipulate polynucleotides, including DNA and RNA. Such components include, for example, those disclosed in Molecular Cloning: A Laboratory Manual, third edition (Sambrook et al., 2000) Cold Spring Harbor Press, or any other reference disclosed herein.

### E. Instructions.

Kits may further include instructions for performing the methods described herein. Instructions may be included as a separate insert and/or as part of the packaging or container, e.g., as a label affixed to a container or as writing or other communication integrated as part of a container. The instructions may inform the user of methods for application and/or removal of the contents of the kit, precautions and methods concerning handling of materials, expected results, warnings concerning improper use, and the like.

### F. Additional optional components of the kits.

Kits may further contain components useful in the practicing the methods disclosed herein. Exemplary additional components include chemical-resistant disposal bags, tubes, diluent, gloves, scissors, marking pens and eye protection.

### EXAMPLES

The following non-limiting examples serve to more fully describe the manner of using the above-described invention. It is understood that these examples in no way serve to limit the scope of this invention, but rather are presented for illustrative purposes.

All nucleic acid analog and DNA stock solutions in the following Examples were made in 5 mM phosphate buffer, pH 7.5 unless otherwise noted. The stock dye was made in methanol or DMSO.

### EXAMPLE 1

### Liquid-Based Assay System for Detection of PNA/polynucleotide Binding

The formation of a PNA/polynucleotide complex resulted in a color change of the indicator dye. Cauliflower mosaic virus 35S promoter DNA was used as the specific target polynucleotide. The dye was 3,3'-diethylthiacarbocyanine.

In a test tube, 15 µM PNA molecules complementary to cauliflower mosaic virus 35S promoter (35SPNA was added to 5 µM 35S promoter DNA (35SDNA). The PNA sequence was CCCACCCACGAGG-LYS. (SEQ ID NO: 11) 150 µM dye in 5mM phosphate buffer, pH 7.5, was added. The 35SPNA, and dye, are present in excess in the system.

The color change indicated the presence of the polynucleotide.

The liquid based system was tested using lul of 2.5mM dye diluted in a 50ul total volume of 5mM PO4 reaction buffer. The system and methods were tested and successful in the pH range of 4-10, however the system performed better in systems above the pH of 5. At time zero reactions across the 4-10 pH range looked nearly identical to each other but less vibrant than controls lacking the PNA/target polynucleotide hybrid. Upon exposure to light stimulus, the reactions began to become clear. Upon further exposure to light stimulus reactions with PNA/polynucleotide hybrid became completely clear at the same rate between pH 6-10. Reactions with PNA/polynucleotide hybrid from pH 4-5 had residual dye at various shades of pink..

A number of buffers at 10mM concentrations have been used. Some of the buffers were tested with various pHs. NaPO4 buffers were tested up to 0.5M concentration. Buffers and salts which performed optimally include NaPO4, NaHSO4, K2HPO4, K2SO4, and CaSO4. Buffers and salts which did not allow successful performance of the methods include Na Citrate, NaCl, CaH2PO4, FeSO4, and MgSO4. The method also may use pure water, 0.1% SDS, 0.1% Triton X, 0.1% Tween-20, 3% butanol, 10% methanol, 10% isoporpanol, or 10% DMSO, 1X blood lysis buffer (0.15M NH₄Cl, 10mM NaHCO₃, 1mM EDTA) pH 7.4 or sucrose lysis buffer (0.32M sucrose, 10m MTris, 1% Trition X-100, 5mM MgCl₂).

Sodium phosphate buffer was tested at pH 4, 5, 5.5, 6, 7, 8, 9, 10. All other buffers were tested at pH 7.5. Sodium phosphate buffer pH 7 gave the fastest reaction.

300 target polynucleotide per microliter of reaction were detected. Concentrations of 1.5 X 10¹⁴ target polynucleotides/reaction (3 X 10¹² target polynucleotide/ul of reaction); 10⁸ amplified target polynucleotides /reaction (2 X 10⁶ amplicon target polynucleotides/ul of reaction) were also detected.

### EXAMPLE 2

### Determination of the Effect of PNA molecules on PCR Reactions

This Example shows the effects of various PNA molecules on PCR reactions. The PCR components were as follows:

| **Component** | **Volume** | **Final Concentration** |
|---|---|---|
| Water | 38 ul | N/A |
| Upstream primer, 50µM | 0.5 ul | 25uM |
| Downstream primer, 50µM | 0.5 ul | 25uM |
| MgCl₂, 25Mm | 3 ul | 1.5mM |
| 10X Reaction Buffer (Promega) | 5 ul | 1X |
| PCR Nucleotide Mix (Promega), 10mM each dNTP | 1 ul | 800uM |
| Taq DNA Polymerase in Storage Buffer B (Promega), 5u/µl | 0.5 ul | 0.05 u/ul |
| BSA | 0.5 ul | 10 mg/ml |

| **Composition of 10X Reaction Buffer:** | |
|---|---|
| Tris-HCl | 100mM |
| KCl | 500mM |
| Triton X-100 | 1% |

The primer sequences were as follows:

| **Primer** | **Primer Sequence** |
|---|---|
| OI 17 | 5' GCTCCTACAAATGCCATCA (SEQ ID NO: 12) |
| OI 18 | 5' GATAGTGGGATTGTGCGTCA (SEQ ID NO: 13) |
| PNA | 5' CCCACCCACGAGGAACATC (SEQ ID NO: 14) |

| **Thermal cycler program** (MJ Research PTC-200) | |
|---|---|
| | 95° for 1 minute |
| 42 cycles of: | |
| | 94° for 10 seconds |
| | 53° for 10 seconds |
| | 72° for 20 seconds |
| Hold at 4° | |

The following samples were analyzed by PCR to determine if PNA molecules inhibit PCR reactions;

1. 1 ul Bt/RR corn DNA (Bt/RR corn has been genetically modified))

2. 1 ul Bt/RR corn DNA, 1 ul PNA (100mM)

3. 1 ul Bt/RR corn DNA, 5 ul PNA (100mM)

4. 1 ul Water

The PCR results were visualized using 2% TBEE agarose gel electrophoresis.

The following samples were analyzed to determine whether the presence of PNA inhibits PCR. In addition, the effects of PNA and methanol on PCR performance were determined.

1. 1 ul Bt/RR corn DNA

2. 1 ul Bt/RR corn DNA, 1 ul PNA (100mM)

3. 1 ul Bt/RR corn DNA, 5 ul PNA (100mM)

4. 1 ul Bt/RR corn DNA, 1 ul methanol

5. 1 ul Bt/RR corn DNA, 5 ul methanol

6. 1 ul Water

The PCR results were visualized using 2% TBEE agarose gel electrophoresis.

The results indicate that PNA did not inhibit PCR when 1 µl PNA was added to the reaction (Tube 2). PNA did inhibit PCR when 5 µl PNA was added to the reaction (Tube 3). Methanol did not inhibit PCR when 1 µl PNA was added to the reaction (Tube 4). Methanol did inhibit PCR when 5 µl PNA was added to the reaction (Tube 5).

### Example 3

The time course of light stimulated change in an optical property of 3,3'-diethylthiacarbocyanine iodide for different concentrations of DNA molecule was determined for a specific target polynucleotide sequence, and PNA sequence.

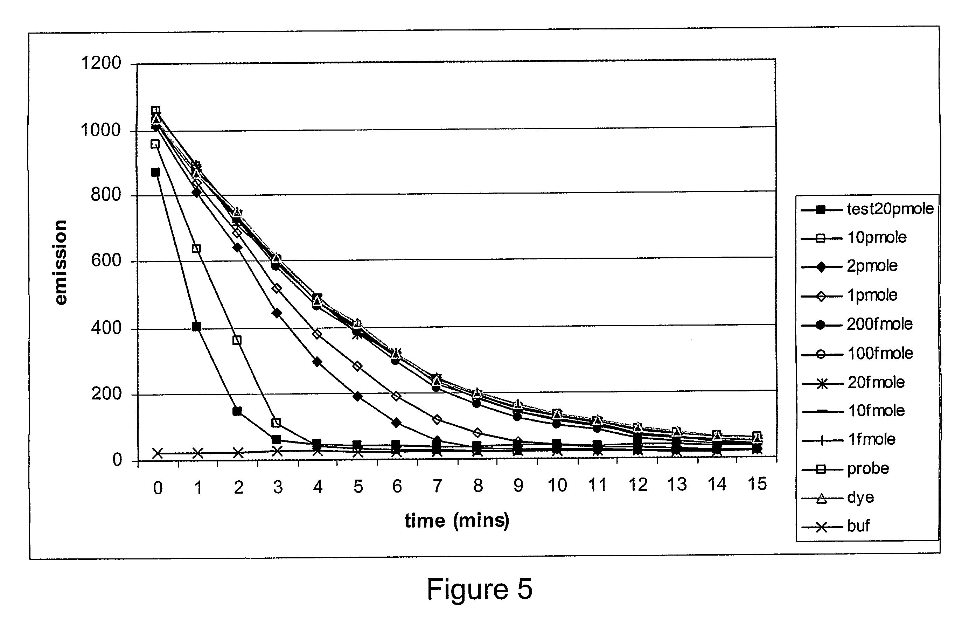

The target polynucleotide was 5' CTACGGGAGGCAGCAGTG 3' (SEQ ID NO: 2) and complementary the PNA molecule was N' CACTGCTGCCTCCCCGTAG-Lys. (SEQ ID NO: 1) The target polynucleotide concentrations were those that are listed in the legend of Figure 5. The PNA concentration was 14.4 pmole/reaction. The dye concentration was 6nmole/reaction.

Figure 5 depicts the time course of 3,3'-diethylthiacarbocyanine iodide dye emission after exposure to light stimulus for different concentrations of DNA. Assay sensitivity with varying test DNA concentration using mixed wavelength light source to stimulate the change. Varying concentrations of polynucleotide (in this case, DNA) are depicted in the Figure legend. Legend concentration represents the final DNA concentration in each reaction. (□) represents samples containing probe and dye only; (Δ) represents samples containing dye only; (x) represents samples containing buffer only.

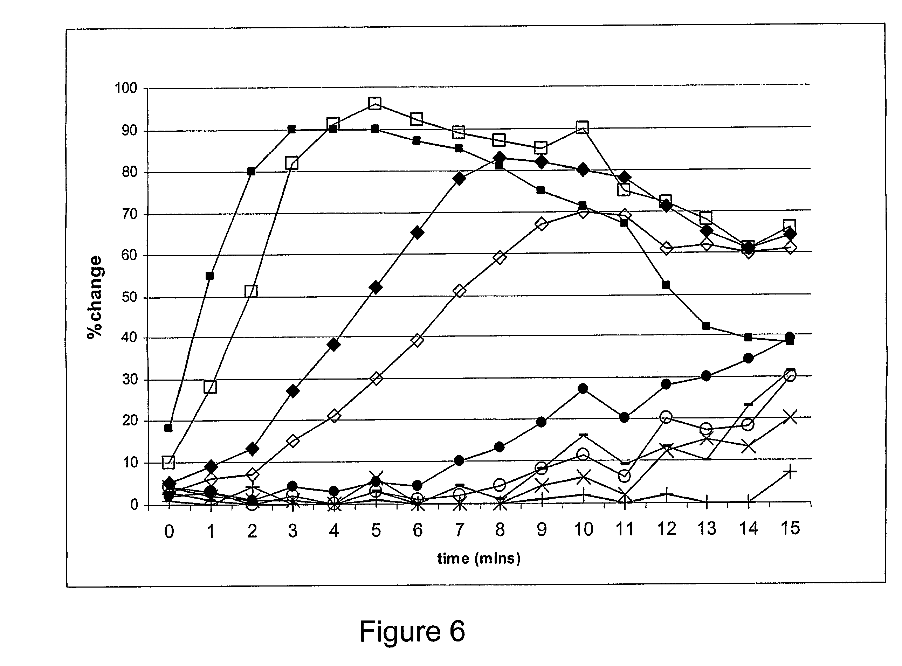

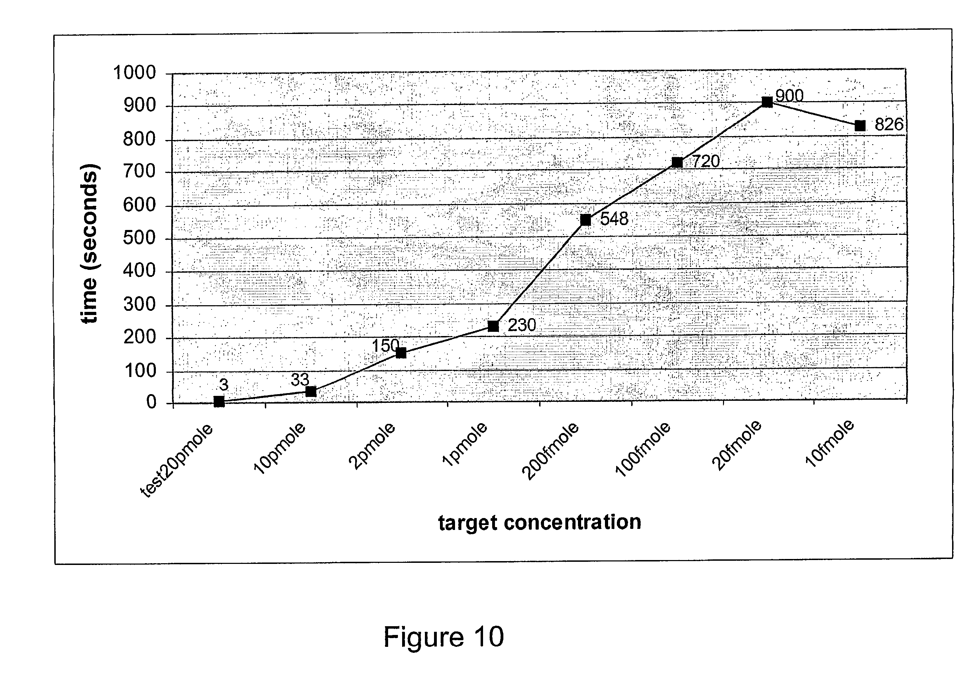

Figure 6 depicts the percent change in emission at different DNA concentrations. The dye had a higher rate of change of emission at higher DNA concentrations. By comparing the rate of change in an optical property of the dye at higher PNA concentrations to the rate of change in an optical property at lower PNA concentrations (or in the absence of PNA), the presence of the target polynucleotide was detected and could be quantitated as shown in Figure 10. Polynucleotide concentrations are the same as depicted in the legend of Figure 5.

### Example 4

This example illustrates that rates of change in optical properties of dyes can differ for different wavelengths of light stimulus.

A target polynucleotide having the sequence 5' CTACGGGAGGCAGCAGTG 3' (SEQ ID NO: 2) at 20 pmole/reaction and the PNA molecule having the sequence N' CACTGCTGCCTCCCCGTAG-Lys (SEQ ID NO: 1) at 14.4 pmole/reaction were combined with 3,3'-diethylthiacarbocyanine iodide dye to form a mixture. The mixture was then exposed to the light stimulus produced by different light spectrum over 10 minutes, and the percent change in dye emission was measured over time.

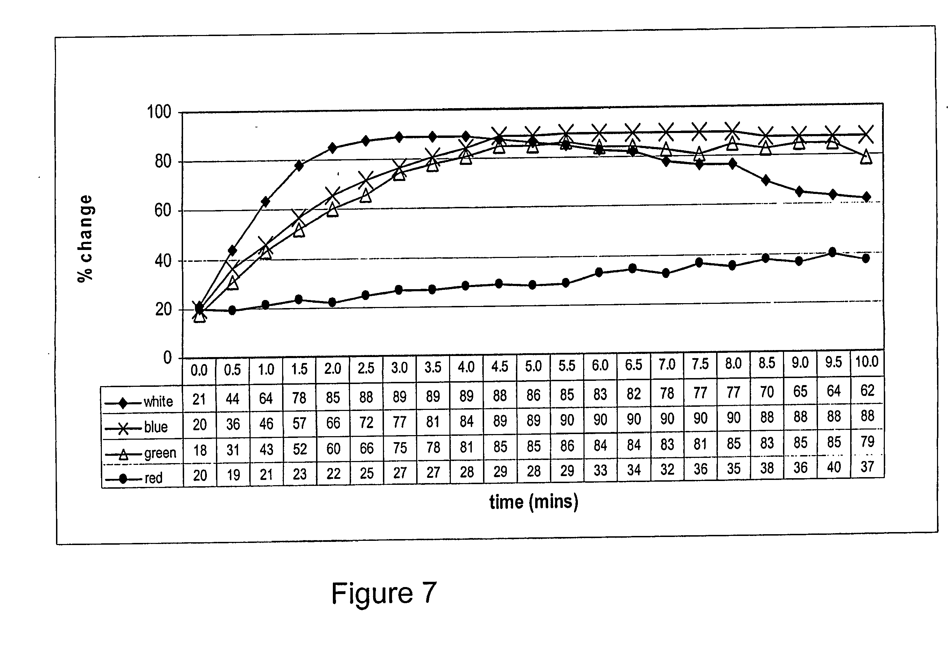

Figure 7 depicts the results of this experiment. White light stimulus was generated using the Fritz Aurora 50/50 visual spectrum, and generated wavelengths from 400 nm to 700 nm. Blue light stimulus was generated using a blue filter. The resulting blue light was from 450 nm to 480 nm. Green light stimulus was generated using a green filter. The resulting green light was from 450 nm to 480 nm. Red light stimulus was generated using a red filter. The resulting red light was from 630 nm to 720 nm.

White light stimulus resulted in the greatest percent change in emission. Blue and green wavelengths resulted in initially slower percent changes in emission. Red wavelengths showed a very slow percent change in emission.

### Example 5

This Example demonstrates that the methods may also be used to detect differences in nucleic acid sequence.

Two samples were prepared. The first sample contained a mixture of PNA complementary to a GMO sequence found in GMO soy polynucleotide, GMO soy polynucleotide, and 3,3'-diethythiacarbocyanine iodide. The second sample contained a mixture of PNA complementary to a GMO sequence found in GMO soy polynucleotide, non-GMO soy polynucleotide, and 3,3'-diethylthiacarbocyanine iodide. Upon exposure to light stimulus, rate of change in emission over time was observed for both samples. The soy polynucleotides were obtained from soy leaf for both mixtures. In both mixtures, 1µl of isolated DNA was used in a 100 µl reaction volume at a DNA concentration was 25 ng/ul.

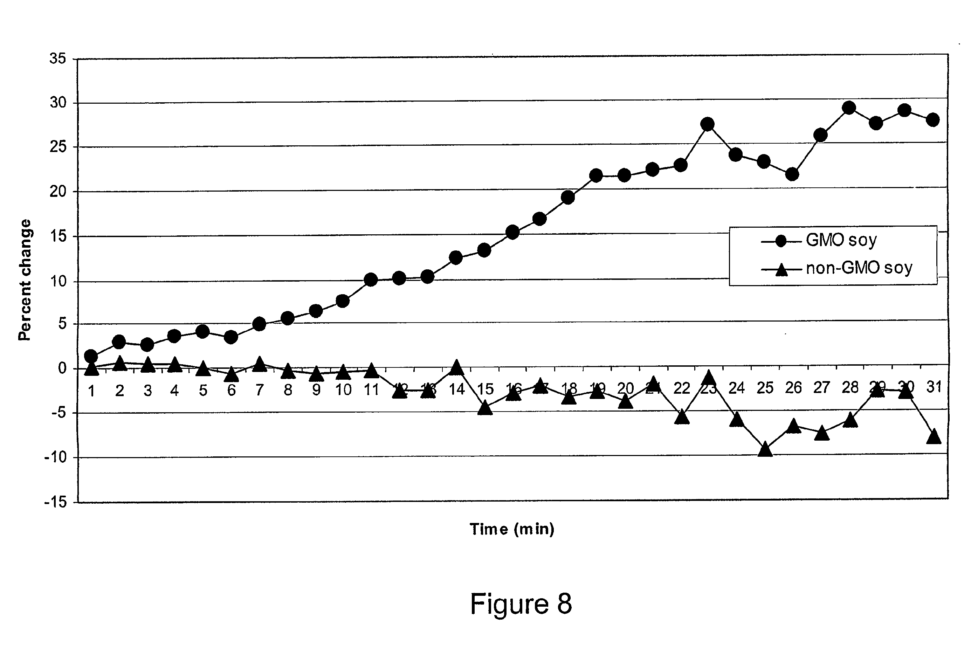

The resulting rate of change in emission over time is depicted in Figure 8. The percent change in emission of 3,3'-diethythialcarbocyanine iodide was measured over time for a sample containing 3,3'-diethylthiacarbocyanine iodide, a PNA molecule complementary to a region of genetically modified organism (GMO) soy 35S polynucleotide), and either a genetically modified organism (GMO) soy polynucleotide or non-GMO polynucleotide. The change in emission of the dye results from the hybridization of the PNA to the GMO soy, and not to the non-GMO soy. The method detected the GMO soy, but not the non-GMO soy. The present methods provide means to detect differences in polynucleotide sequences present in DNA.

### Example 6

This example shows that the quantity of polynucleotide in a sample may be determined by measuring the rate of change in an optical property of the dye in a sample having an unknown polynucleotide concentration and correlating the rate of change in the optical property to a curve of known rates of change in the optical property. The time may be fixed and the change may be measured at a particular time of reaction. The determination of the rate may also include determining the change in optical property at a single time and correlating the change in optical property to the concentration of one or more samples having a known concentration.

The time necessary for a 20% change in emission at a series of polynucleotide concentrations was calculated. The target polynucleotide sequence was 5' CTACGGGAGGCAGCAGTG 3', (SEQ ID NO: 2) and the PNA molecule sequence was N' CACTGCTGCCTCCCCGTAG-Lys. (SEQ ID NO: 1) The time required for a 20% reduction as a function of target polynucleotide concentration is depicted in Figure 10. The data correspond to the percent change data in Figure 6. For one version of the assay, the point at which there is a 20% change in emission is measured for a sample having an unknown concentration. The quantity of polynucleotide in a given sample may be determined by extrapolating to a standard curve.

### Example 7

This example demonstrates the target polynucleotide may be RNA.

A PNA, RNA, and 3,3'-diethylthiacarbocyanine iodide dye were combined. The target RNA sequence was 5' CUACGGGAGGCAGCAGUG 3', (SEQ ID NO: 15) and the PNA sequence was a universal bacterial PNA probe having the complementary sequence to the RNA sequence. The mixtures were exposed to light stimulus, and the percent change in emission of the dye over time was measured, and compared to a sample including a DNA polynucleotide sequence instead of an RNA polynucleotide sequence.

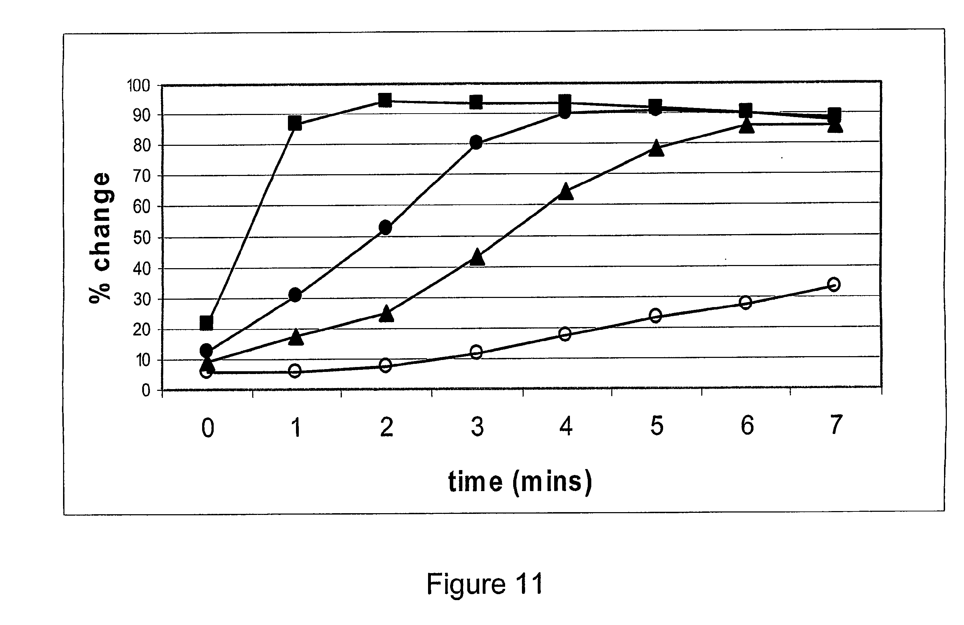

The resulting percent change in the optical property over time is depicted in Figure 11. Comparison of 20 pmole of target DNA per reaction (■), and target RNA (●) at RNA concentrations of 20 pmole/reaction, 10 pmole/reaction, and 2 pmole/reaction. The Y-axis represents the percent change in fluorescence with time compared to probe and dye alone. RNA polynucleotides produced a change in emission of the dye over time. The presence of RNA was detected based on the rate of change in the optical property of the dye.

### Example 8

This example demonstrates that the method works in the presence of a contaminating background.

The percent change in emission over time for 3,3'-diethylthiacarbocyanine iodide in a sample in a contaminating background of deli meat juices was determined. 3,3'-diethylthiacarbocyanine iodide dye, and a PNA molecule were prepared. The target polynucleotide sequence was 5' CTACGGGAGGCAGCAGTG 3', (SEQ ID NO: 2) and the PNA sequence was CACTGCTGCCTCCCCGTAG-Lys. (SEQ ID NO: 1)

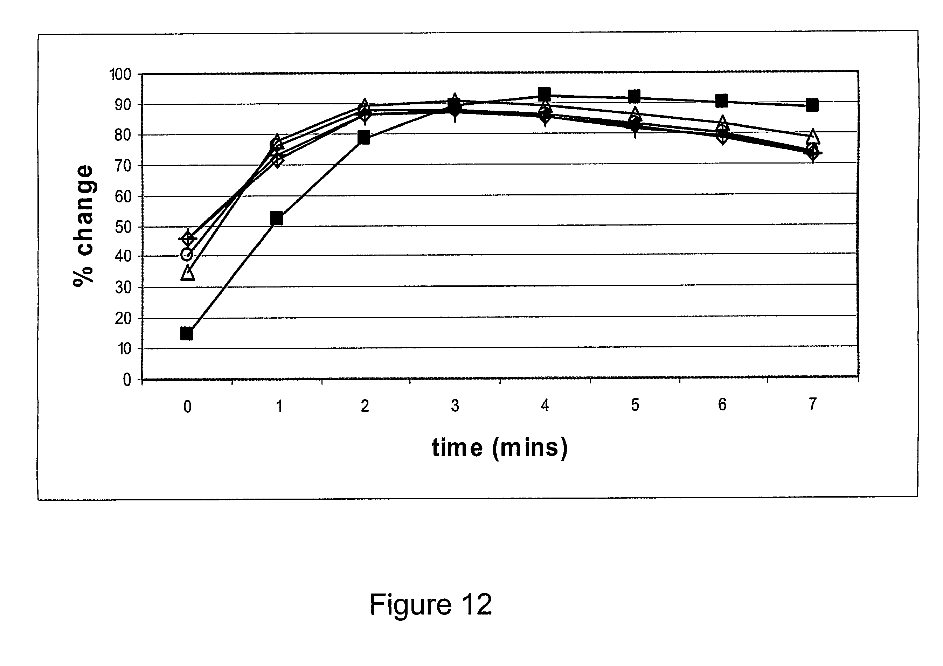

The results are depicted in Figure 12. Percent changes in (■) an uncontaminated, clean system and in samples contaminated in a background of 1 □1,2 □1, and 4 □1 concentrations of deli meat wash are depicted (open symbols). The example demonstrates that the presence of a polynucleotide was detected in a contaminated background.

### Example 9

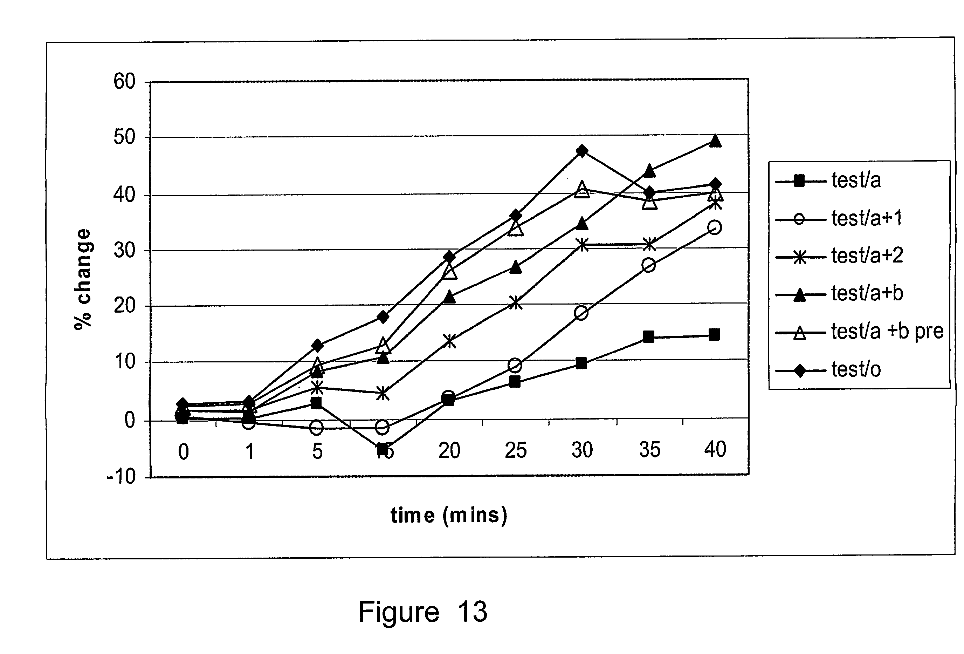

Figure 13 depicts the addition of PNA "wedges" in the system. Biotinylated PNA (5'bio-oo-gatagtgggattgtgcgt) (SEQ ID NO: 16) is attached to a streptavidin well then reacted with a polynucleotide. After hybridization, the dye is added the system exposed to light stimulus and the percent change determined over time. The polynucleotide may be a synthesized polynucleotide or an amplicon target polynucleotide. "Test a" represents 35S PNA 5'bio-oo-gatagtgggattgtgcgt (SEQ ID NO: 16) and a 195bp amplicon target polynucleotide. "Test a+1" includes both the 35S PNA 5'bio-oo-gatagtgggattgtgcgt (SEQ ID NO: 16) with 195bp amplicon target polynucleotide and PNA 5'tcttctttttccacg-LYS. (SEQ ID NO: 17) "Test/a+2" includes 35S PNA 5'bio-oo-gatagtgggattgtgcgt (SEQ ID NO: 16) with a 195bp amplicon target polynucleotide and PNA 5'tcttctttttccacg-LYS. (SEQ ID NO: 17) "Test/a+b" includes 35S PNA 5'bio-oo-gatagtgggattgtgcgt (SEQ ID NO: 16) with a 195bp amplicon target polynucleotide, 5'tcttctttttccacg-LYS, (SEQ ID NO: 17) and 5'tcacatcaatccact-LYS. (SEQ ID NO: 18) "Test/a+b pre" includes 35S PNA 5'bio-oo-gatagtgggattgtgcgt (SEQ ID NO: 16) with a 195bp amplicon target polynucleotide, 5'tcttctttttccacg-LYS, (SEQ ID NO: 17) and 5'tcacatcaatccact-LYS, (SEQ ID NO: 18) where 5'tcttctttttccacg-LYS (SEQ ID NO: 17) and 5'tcacatcaatccact-LYS (SEQ ID NO: 18) are incubated with the amplicon target polynucleotide before hybridization with the attached PNA. "Test/o" represents 35S PNA 5'bio-oo-gatagtgggattgtgcgt (SEQ ID NO: 16) with a complementary polynucleotide. The linker -oo- was 8-amino-3,6-dioxaoctanoic acid. In this and other embodiments, linkers can be any chemical linking group known in the art. The addition of nucleic acid analogs to different sites on the target polynucleotide can alter the rate of change.

### Example 10

This Example depicts the rate of change in an optical property of the dye at different wavelengths.

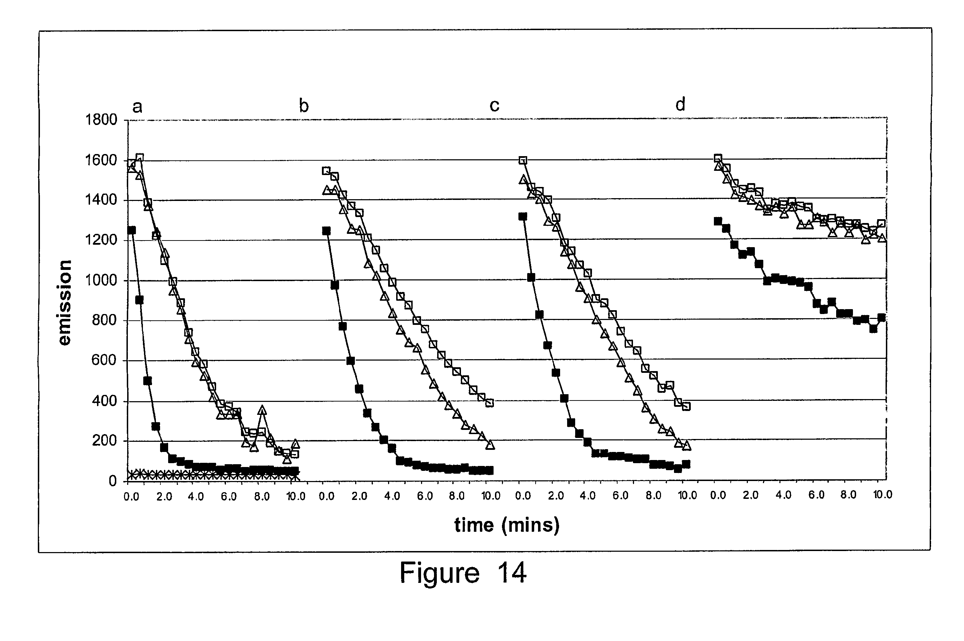

Figure 14 depicts the comparison of exposure to light stimulus of different wavelengths for a series of samples. A mixture of target polynucleotide, PNA, and 3,3'-diethylthiacarbocyanine iodide dye was prepared, and a change in an optical property of the dye was observed. The target polynucleotide was 5' CTACGGGAGGCAGCAGTG 3', (SEQ ID NO: 2) and the PNA molecule was CACTGCTGCCTCCCCGTAG-Lys. (SEQ ID NO: 1)

Figure 14 a)-d) depict the time course of dye emission over time at a series of wavelengths as follows: a) mixed light; b) blue light source, 450nm; c) green light source, 510nm; d) red light source 645nm. For each of a)-d), (■) depicts a positive control or test sample; (□) depicts the PNA probe only; (A) depicts the 3,3'-diethylthiacarbocyanine iodide dye only; (x) depicts a buffer background. The emission corresponds percent changes depicted in Figure 7.

### Example 11

This example shows that the methods may be used in a solid based format by preparing a PNA/polynucleotide hybrid either before or after binding to a solid support.

A universal bacterial PNA probe, and its complementary polynucleotide, were used in these experiments. The target polynucleotide was 5' CTACGGGAGGCAGCAGTG 3', (SEQ ID NO: 2) and the PNA molecule was 5' bio - CACTGCTGCCTCCCCGTAG3' (SEQ ID NO: 1). In a first sample, a biotinylated PNA probe was immobilized to well by streptavidin-biotin binding. The polynucleotide and 3,3'-diethylthiacarbocyanine iodide dye were then added. The sample was exposed to light stimulus, and the percent change in fluorescent emission of the dye was measured.

In a second sample, a biotinylated probe and a target polynucleotide were allowed to hybridize together to form a PNA/polynucleotide hybrid. The PNA/polynucleotide hybrid was then added to a streptavidin coated well, and the hybrid was allowed to bind to the well surface.

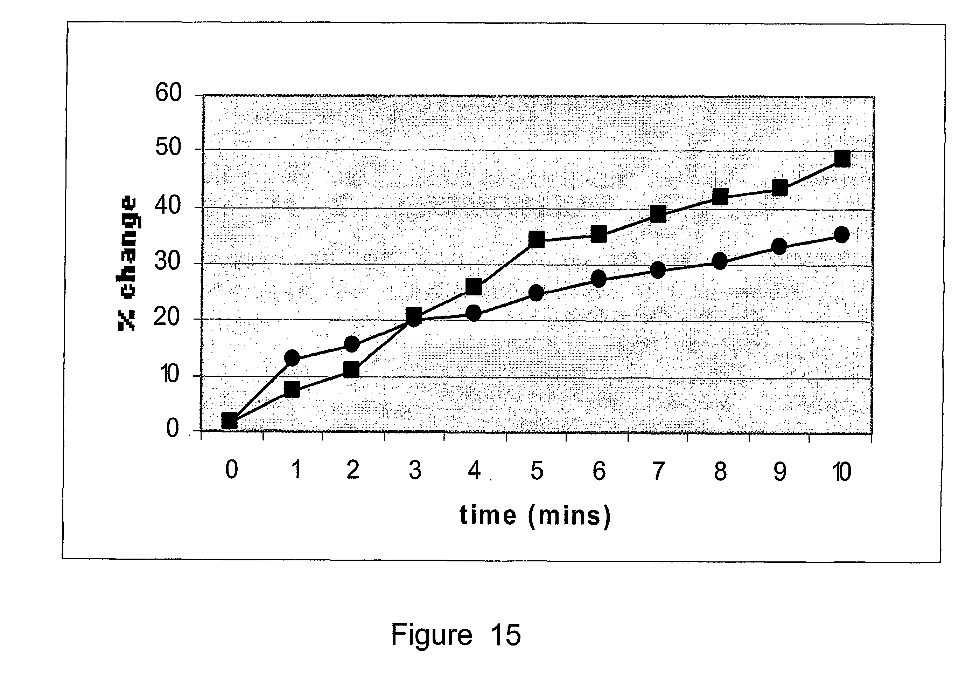

Figure 15 depicts the percent change in emission over time observed in the first and second samples. In test 1 (■), the probe-bio immobilized to well by streptavidin-biotin binding before reacting to the target DNA and dye. In test 2 (●), the PNA molecule and target was allowed to hybridize together in solution before adding to the well. Y-axis represents the percent change in fluorescence with time compared to probe alone.

### Example 12

This example demonstrates that the methods may be used to identify and/or quantify a target polynucleotide obtained from a blood sample.

Human blood was collected by venipuncture into EDTA tubes. 10µl of male blood and 10µl of female blood were added into a microfuge tube containing 180 µl of lysis buffer that was 6M guanidinium thiocyanate; 20mM EDTA; 10mM TRIS.HCl (pH 6.5); 40g/L Triton X; 10g/L dithiothreitol. Prior to its addition to the blood the buffer was heated at 60°C until dissolved. 10µl of female blood was added into a microfuge tube containing 190µl of the same lysis buffer. The reactions were allowed to incubate at room temperature for 10 minutes.

The reactions were centrifuged for 5 mins at 4000rpm. Supernatant was discarded, and the pellet and wash with 500µl of the lysis buffer. The pellet was centrifuged again for 2 mins at max speed. The supernatant was discarded, and the supernatant was washed with 500µl of wash buffer (25% ethanol; 25% isopropanol; 100mM NaCl; 10mM Tris.HCl (pH8.0)). The reactions were centrifuged for 2 mins at maximum speed, and the supernatant was discarded. The reactions were washed twice with 5mM phosphate buffer used in the PNA hybridization reaction. After the final rinse, reactions were resuspended with 200µl of 5mM phosphate buffer.

The following table shows the test conditions for reaction in microwell. 2.5 µl of whole blood was used in a 50µl reaction volume. This equates to 300 targets/µl of reaction and 1ng of DNA. PNA sequence 5' Bio-OO-TGAGTGTGTGGCTTTCG 3' (SEQ ID NO: 19).

| | Test | Neg control | No PNA control |
|---|---|---|---|
| Sample | 48 µl of test | 48 µl of negative | 48 µl of negative |
| PNA | 1 µl | 1 µl | 0 |
| Dye | 1 µl | 1 µl | 1 µl |

Emission data was collected using a Genios spectrophotometer at an excitation wavelength of 535 nm and an emission wavelength of 590nm. After an initial zero minute read, the samples were exposed to light stimulus from the Aurora 50/50 for 30 seconds. The fluorescence was measured every 30 seconds for 10 minutes.

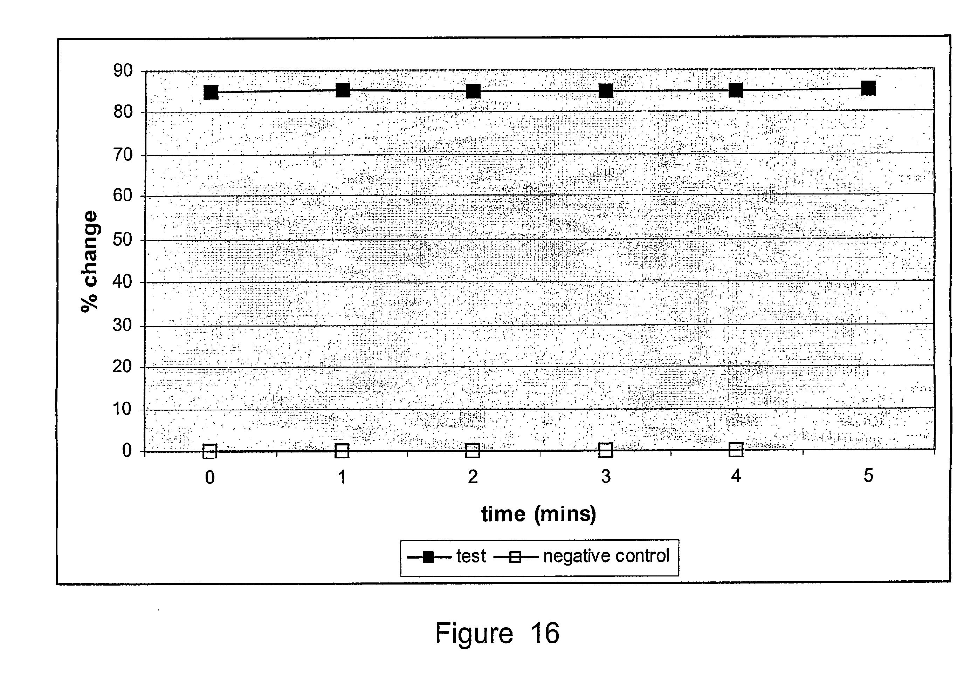

Figure 16 shows that the sample of target male blood, depicted by (■), was detected by the assay, but the female blood depicted by (□) as a negative control was not detected.

### EXAMPLE 14

A number of nucleic acid sequences were or can be used to detect target polynucleotides. These sequences are listed below.

| PNA name | Seq | |
|---|---|---|
| PPL101bio | 5'bio-oo-tgagtgtgtggctttcg (SEQ ID NO: 19) | huSRY |
| | | |
| PPI2024 | 5'cactgctgcctcccgtag-LYS (SEQ ID NO: 1) | 16S |
| PPI2024bio | 5'bio-OO-actgctgcctcccgtag (SEQ ID NO: 20) | |
| PPI2025bio4 | 5'bio-OOOO-tgcctcccgtag (SEQ ID NO: 21) | |
| PPI2025bio6 | 5'bio-OOOOOO-tgcctcccgta (SEQ ID NO: 22) | |
| PPI2025bio8 | 5'bio-OOOOOOOO-tgcctcccgta (SEQ ID NO: 22) | |
| PPI2025bio10 | 5'bio-OOOOOOOOOO-tgcctcccgtag (SEQ ID NO: 21) | |
| PPI2025 UL | 5'tgcctcccgtag (SEQ ID NO: 21) | |
| | | |
| PPI18bio | 5`bio-oo-gatagtgggattgtgcgt (SEQ ID NO: 16) | 35S |
| PPI359 | 5'cccacccacgagg-LYS SEQ ID NO: 11) | |
| PPI485 | 5'tcttctttttccacg-LYS (SEQ ID NO: 17) | |
| PPI486 | 5'tcacatcaatccact-LYS (SEQ ID NO: 18) | |
| | | |
| PPI2202 | bio-(O)₁₀-ctcattgatggt (SEQ ID NO: 23) | HIV |
| PPI911 | bio-(O)₁₀-cgcagaccacta (SEQ ID NO: 24) | HCV |

### EXAMPLE 15

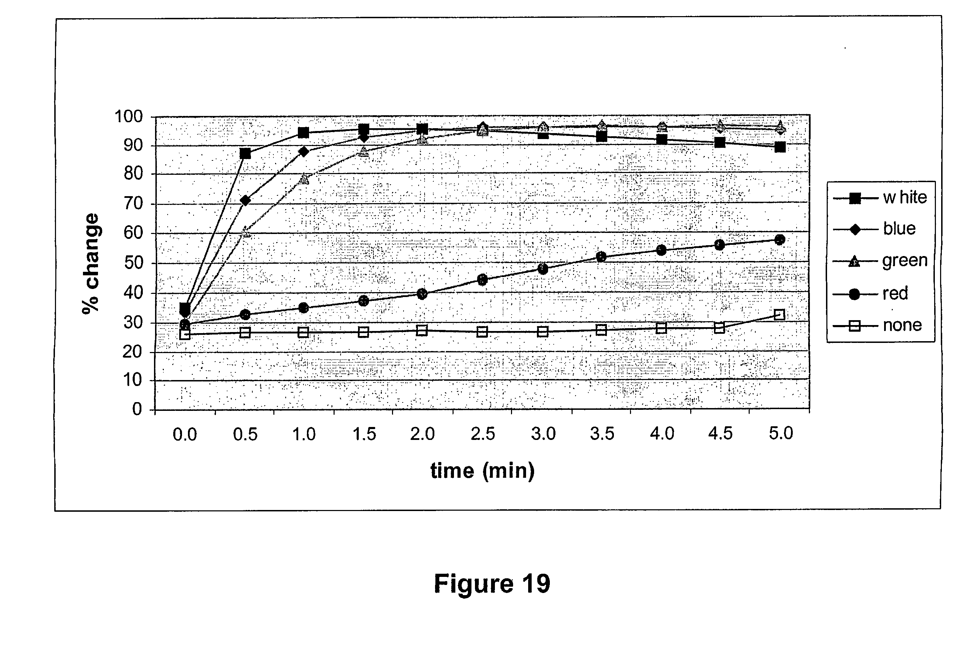

Figure 19 depicts the effect of exposure to different wavelengths of light stimulus.

A mixture including 16S PNA (5' cactgctgcctcccgtag-LYS) (SEQ ID NO: 1), a polynucleotide (5' ctacgggaggcagcagtg) (SEQ ID NO: 2), and 3,3'-diethylthiacarbocyanine iodide dye were exposed to a white light stimulus having all visible wavelengths, a blue light source (450nm), green light source (510nm), a red light source (645nm), and no light stimulus exposure. Light source for the mixed light was an Aurora 50/50 from FITZ. For blue, green and red, filters were placed on the Aurora 50/50. Reading was done on a Safire multiwell plate reader (produced by Tecan).

Mixtures exposed to light stimulus resulted in a different rate of change in an optical property of the dye compared to a reference value. Mixtures that were not exposed to light stimulus resulted in no measurable change over the time measurement. Different wavelengths resulted in different rates of change in optical properties. This example shows that light radiation may be used to cause different rates of change in an optical property of a dye.

### EXAMPLE 16

The PNA was immobilized on a solid substrate, and the amount of DNA was detected based on the change in rate of fluorescence of a dye.

The following reactants were used in this example: a)10 µM probe-BIO (from Example 14); b) 2 mM dye Diethylthiacarbocyanine iodide (100mM stock in DMSO; 2mM working concentration in 5mM buffer); c) 5 mM buffer (pH 5.5) (1ml of 100mM Na₂HPO₄.7H₂O + 24ml of 100mM Na₂H₂PO₄.H₂O + 475ml H₂O pH to 5.5); d) 1XPBS + 0.05% Tween 20 (PBST) (1X PBS = 0.137M NaCl + 2.68mM KCl + 4.3mM NaH₂PO₄ + 1.47mM KH₂PO₄); e) Streptavidin microtiter plates (NUNC); and f) test samples containing or lacking a target polynucleotide.

The number of wells was calculated. Each well was prepared by pre-washing wells 3X with 200µl 1XPBST. 1ul of PNA probe was used in 50ul total reaction. 3ul of probe was added to 147ul PBST.

The PNA probes were attached to the solid surface of microtiter wells. The mixture was incubated 1 hour at room temperature shaking gently.

| + control | Test sample | Probe only | Buffer only |
|---|---|---|---|
| 50ul | 50ul | 50ul | 50ul buffer |

The wells were then washed with 3X with 100ul of PBST. The liquid was removed. The wells were then washed with 3X with 5mM phosphate buffer. For a positive control, 1ul of the reaction was added in 49ul of 5mM phosphate buffer, then added to the positive control well. 1ul of the sample was diluted in 49ul of phosphate buffer then added to the test sample well. To probe and buffer only wells, 50ul of phosphate buffer was added. All wells were incubated for 30 minutes at room temperature with gentle shaking. The wells were washed 5X with 100ul of phosphate buffer as described above.

A dye solution was made by using 1ul of 2mM dye to 49ul of phosphate buffer/well. The dye solution was added to all wells except the negative control buffer only well. 50ul of phosphate buffer was added to the buffer only well.

The fluorescence of the dye was observed. An initial fluorescence reading was detected prior to providing exposure to light stimulus from an Aurora 50/50 light source. Excitation was at 535 nm and emission at 590 nm was observed every 2 minutes using a Genios multiwell plate reader.

### EXAMPLE 17

A. The following protocol was followed using a liquid PNA sample.

Sufficient wells for the number of samples to be tested plus 3 for controls were prepared. The DNA or RNA was isolated.

Each test reaction contained: 1 µL PNA probe, 47 µL buffer (5 mM 2ml 100mM Na₂HPO₄.7H₂O + 48ml NaH₂PO₄.H₂O/ L (pH 5.5)), and 1 µL test sample. The samples were loaded into a Greiner 96-well strip plates (#705070).

The probe, dye and buffer were combined to form a mixture, and the wells were set up as follows:

| + control | Test sample | PNA only | Buffer only |
|---|---|---|---|
| 1 µL + control | 1 µL sample | 1 µL buffer | 50 µL buffer |

49 µL of the mixture was added to all wells EXCEPT buffer only well. The solution was gently mixed.

The absorbance or fluorescence without exposure to a light stimulus was determined at the following wavelengths: absorbance: 562nm; fluorescence emission: 535nm; and fluorescence excitation 590nm.

The samples were exposed to light stimulus using an Aurora 50/50 and the absorbance or fluorescence was determined after every 30 seconds of exposure to a light stimulus.

B. The following protocol was conducted using PNA molecules that were immobilized on a solid surface.

The following items were used in the protocol: a) 10 µM PNA (ABI); b) 2 mM 3,3'-diethylthiacarbocyanine iodide (Sigma-Aldrich, catalog #173738) dye; c) 5 mM 1X PBS (0.137M NaCl, 2.68mM KCl, 4.3mM NaH₂PO₄, 1.47mM KH₂PO₄) + 0.05% Tween-20; d) 1X PBS (0.137M NaCl, 2.68mM KCl, 4.3mM NaH₂PO₄, 1.47mM KH₂PO₄) + 0.05% Tween-20; e) test samples including a target polynucleotide; and f) Streptavidin coated plates (Nuncbrand Immobilizer™ (Catalog No. 436014)).

In one variation, the PNA was immobilized before introducing the polynucleotide samples. Enough wells were prepared for samples (*n*) to be tested plus 3 extra wells for controls by washing 3X with 300 µl of 1X PBST. The DNA or RNA to be tested was isolated. The biotinylated PNA was immobilized to the streptavidin coated wells by adding 1 µl of 10 µM PNA stock into 49µl of PBST.

A PNA master mix was made that included (*n* +2)X 1 µl of 10 µM PNA stock, and (*n* +2) X 49 µl of PBST. 50µl of PNA mix was added to all wells EXCEPT buffer only well.

The mixture was covered and incubated for 1 hour at room temperature on a gentle shaker.

Each well was washed 3X with 200µl of PBST, then 3X with 5mM phosphate buffer, and the nucleic acid samples were added to the immobilized probe. 1 µl of sample was added to 49µl of 5mM phosphate buffer.

Control wells were prepared as diagramed below:

| | Test | + control | PNA only | Buffer only |
|---|---|---|---|---|
| Test sample | 1 µl | 1 µl | 0 | 0 |
| 5mM phosphate buffer | 49 µl | 49 µl | 50 µl | 50 µl |

The wells were covered at room temperature for 30 minutes and incubated by gentle shaking. Each well was then washed with 100µl 5mM phosphate buffer 6X. A dye solution was prepared by adding 1 µl of 2mM dye solution to 49µl of 5mM phosphate buffer per sample. 50µl of 5mM phosphate buffer was added to the buffer only well.

An initial read absorbance or fluorescence was detected prior to exposure to a light stimulus at the following wavelengths: absorbance of 562nm; fluorescence emission of 535nm, and fluorescent excitation of 590nm.

Samples were exposed to light stimulus at the using an Aurora 50/50. The Aurora 50/50 can also be used with different colored filters (blue, green, red) to specify the wavelength of interest every 20 minutes of exposure to a light stimulus.

In another variation, PNA was immobilized and target polynucleotides were hybridized at the same time. Enough wells for samples (*n*) were prepared, along with 3 extra wells for controls by washing 3X with 300µl of 1X PBST. The DNA or RNA was isolated.

A mixture of PNA probe and sample was prepared as diagramed below:

| | Test | + control | PNA only | Buffer only |
|---|---|---|---|---|
| PNA | 1 µl | 1 µl | 1 µl | 0 |
| Test sample | 1 µl | 1 µl | 0 | 0 |
| 5mM phosphate buffer | 48 µl | 48 µl | 49 µl | 50 µl |

The mixture was Incubate covered for 10-120 minutes (dependent on application) at room temperature gently shaking. The mixture was washed with 6X with 100µl 5mM phosphate buffer. A dye was prepared as described above. 50µl of the solution was added to each well except the buffer only well.

### EXAMPLE 18

The following protocol was conducted using plant DNA.

The line was RR 2701 soy. The DNA used was from a purified DNA extraction that contained 62ng/ul. Samples were serial diluted from 62ng/ul to 0.062ng/ul. One ul of this dilution was used in a 50ul reaction.

A streptavidin plate was washed 3X with 400ul of Phosphate buffered saline buffer (PBS) (+ 0.5% tween (PBST)) at room temperature (RT). All washing, loading and unloading was done using a pipette. To wash 400ul of solution was added directly to the well. The solution was then sucked off using the same pipette and tip.

PNA bio-18 (35S) was diluted 1/10 in water. (1ul of PNA stock to 9ul water). (BIO-OO-GATAGTGGGATTGTGCGT(SEQ ID NO: 16), where OO are two linkers.) 1 ul of the diluted PNA was added per 49ul of PBST. A master mix was made to include all wells to be bound plus one well in excess. Thus if we were to bind 10 wells enough mix was made for 11 wells. (11ul of the diluted PNA + 539ul PBST). 50ul of this mix was added to each well. The plate was incubated 1 hour at RT with gentle shaking on an orbital shaker. In the mean time, DNA was prepared by making serial dilutions (1/10, 1/100, 1/1000) of each DNA sample in water in micro-PCR tubes. Tubes were placed in a thermal cycler and a denature program was run (95C for 5 min). Upon completion tubes were placed on ice until needed. After incubation the plate was washed 3X with PBST as described above. The plate was then washed 3X with 5mM PO4 buffer (pH 5.7) as described above. One ul of DNA or diluted DNA sample was added to each well, (GM or non GM DNA, one sample per well). 49ul of PO4 buffer was added. Plate was gently mixed and incubated 30min at RT with gentle shaking on an orbital shaker. After incubation the plate was washed 5X with PO4 buffer as described above. 1 ul of 3,3'-diethylthiacarbocyanine iodide (3mM) was added to 49ul of PO4 buffer (A master mix was made to include all wells plus enough for one more well). 50ul of dye/buffer was added to each well using a pipette. The plate was then placed in Tecan scanner to monitor spectral emissions of the reaction at 535 nm excitation and 590 nm emission. An initial read was conducted at time zero. The plate was then exposed to light stimulus and read in intervals of 1 minute. Data as analyzed in Excel and plotted based on percent change vs time using the equation 100-(sample/PNA dye only) X100.

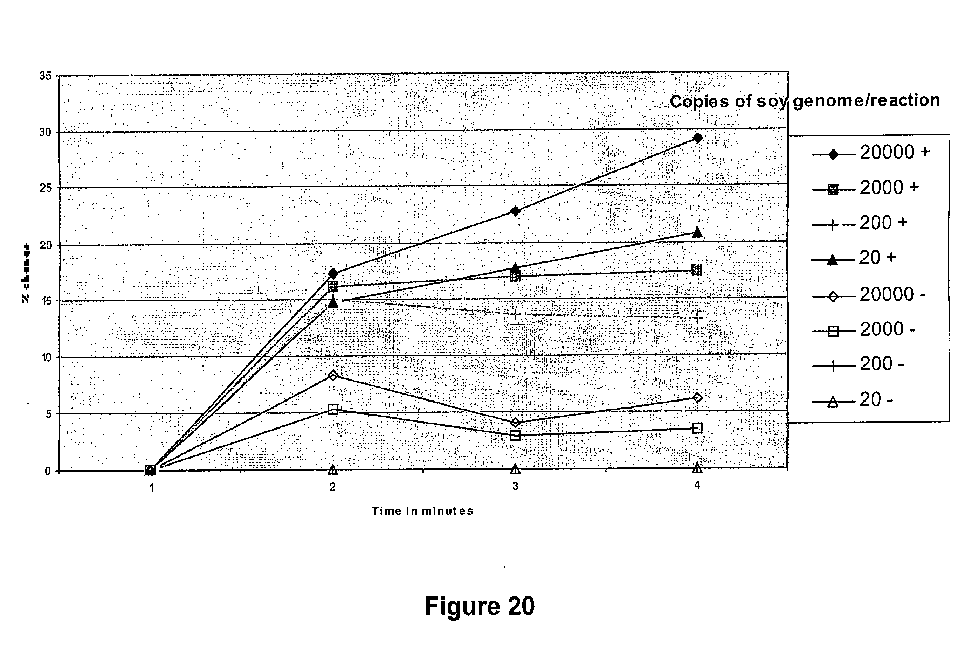

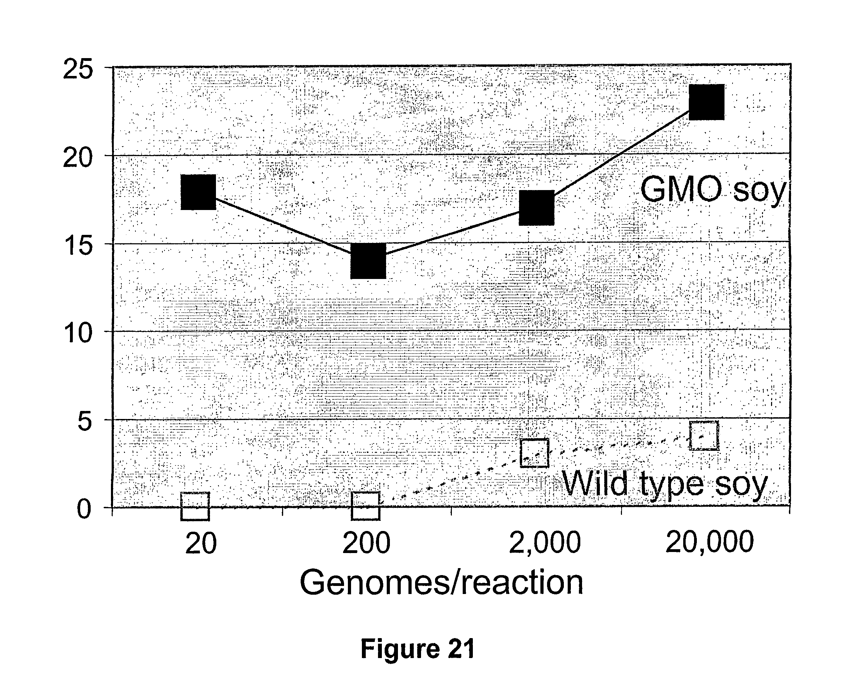

Figure 20 depicts the detection of different concentrations of soy DNA. These are indicated by the +. Soy DNA that did not contain the GMO sequence was close to background levels. These are indicated by the -. The method was able to detect 0.0625 ng GMO soy DNA, which corresponds to approximately 21 genomes. Figure 21 depicts the percent change in optical property versus the number of genomes of GMO positive soy and wild type soy that does not contain the PNA target sequence.

### EXAMPLE 19

The following reaction shows detection of a target polynucleotide in phosphate buffer alone and with various tween20 concentrations. Similar experiments were done with other nonionic detergents including NP-40 and triton X-100.

The following items were used in the protocol: a) 10 µM PNA (ABI); b) 2 mM 3,3'-diethylthiacarbocyanine iodide (Sigma-Aldrich, catalog #173738) dye; c) 5 mM buffer (pH 5.5) (2ml 100mM Na₂HPO₄.7H₂O + 48ml NaH₂PO₄.H₂O/ L); d) test samples.

Enough wells were prepared for the number of samples to be tested plus 3 for controls. The DNA or RNA was isolated. Each test reaction contained: 1 µL probe, 47 µL buffer, and 1 µL sample to form a mixture.

| + control | Test sample | PNA only | Buffer only |
|---|---|---|---|
| 1 µL + control | 1 µL sample | 1 µL buffer | 50 µL buffer |

To all wells EXCEPT buffer only well, add 49 µL of mixture was added, and the solution was mixed gently.

An initial fluorescence measurement was made without exposure to a light stimulus at an emission setting at 535nm and an excitation setting at 590nm. The samples were exposed to light stimulus using an Aurora 50/50 and the absorbance and/or fluorescence was measured after every 60 seconds of exposure to a light stimulus. The standard 5mM phosphate buffer (pH 5.5) was used and compared with reactions in phosphate buffer containing 0.05%, 0.1%. 0.5%. 1.0%, 1.5%, and 2% tween20.

Figure 22 depicts the emission using different concentrations of tween. (■) represents the reaction with target DNA, (□) represents probe alone, (Δ) represents dye in buffer, and (∗) represents buffer alone.

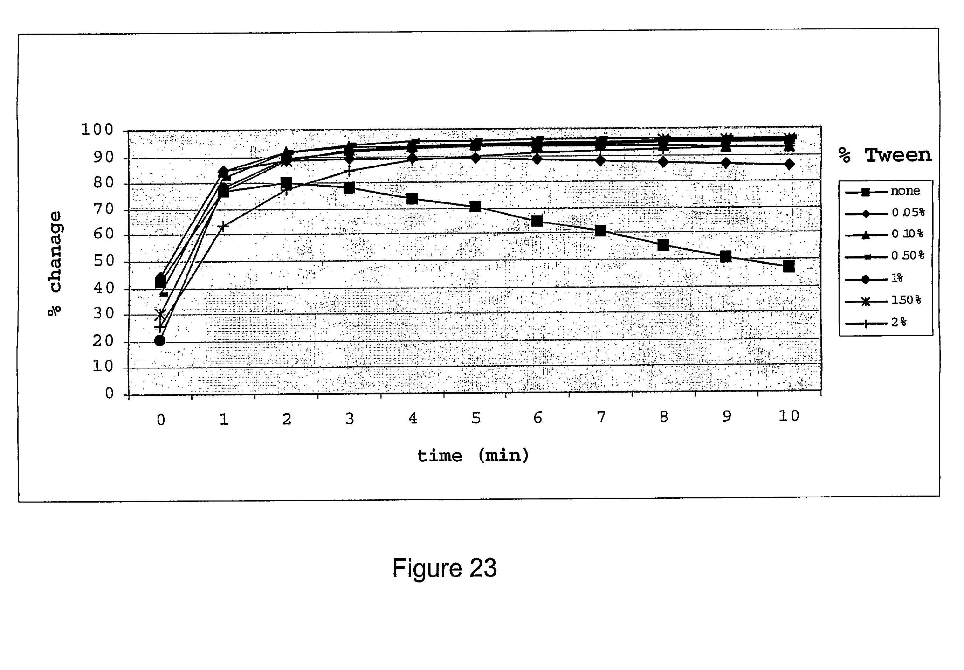

Figure 23 depicts the % change in emission for different concentrations of tween.

### EXAMPLE 20

This example demonstrates that the reaction can use nucleic acid analogs other than PNAs. The present reaction uses locked nucleic acids (LNAs). Various concentration of tween20 in the phosphate buffer was also tested.

The following LNA sequences were used:

LNA-A Tg^{m}c^{m}Ct^{m}c^{m}c^{m}CGTAG (SEQ ID NO: 25)

LNA-B TGC^{m}Ct^{m}Cc^{m}CGTAG (SEQ ID NO: 26)

LNA-C TGCCTCc^{m}CGTAG (SEQ ID NO: 27)

Lower case letter with ^{m} represents modified sequences.

Reactions were conducted in liquid form in accordance with the methods disclosed in Example 19, above.

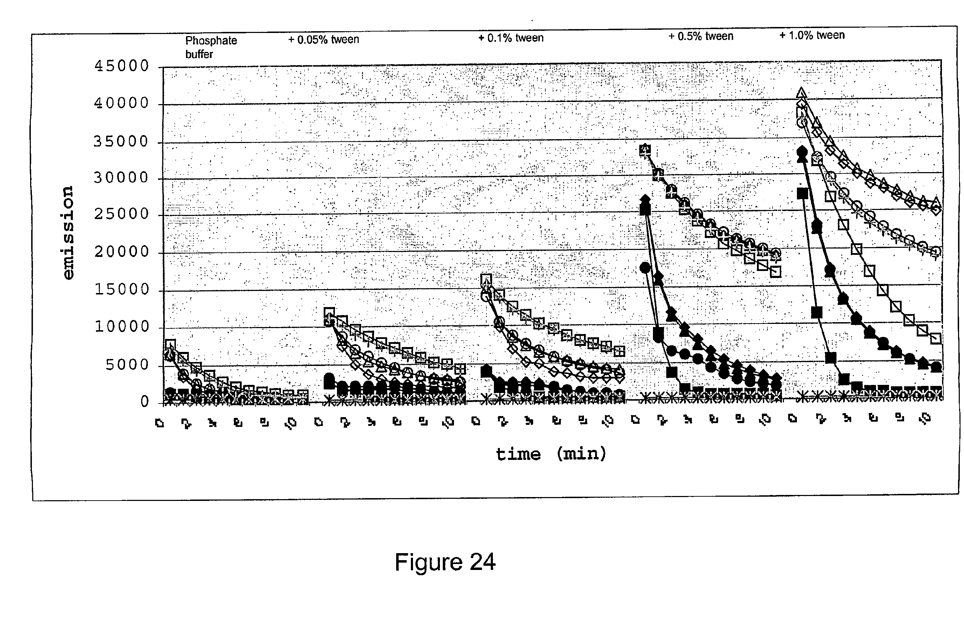

Figure 24 compares the raw data with PNA probes and with LNA probes in the liquid reaction. Standard reaction in phosphate buffer was compared to various concentrations of tween20 added to the phosphate buffer. Solid symbols represent test reaction while open symbols are the respective probes only. (■, □) PNA; (◆, ◊) LNA-A; (▲, Δ) LNA-B; (●, ○) LNA-C.

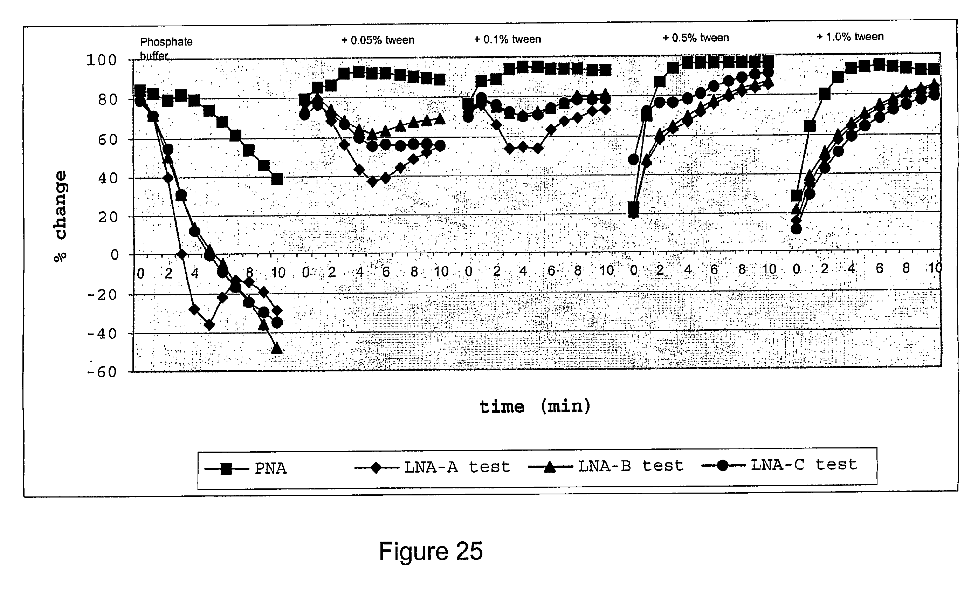

Figure 25 depicts the percent change in emission using PNAs versus LNAs. (■) represents the percent change in the reaction when compared to the PNA probe alone. (◆) represents the percent change in the LNA-A test reaction when compared to the LNA-A probe and dye alone. (▲) represents the percent change in the LNA-B test reaction when compared to the LNA-B probe alone. (●) represents the percent change in the LNA-C test reaction when compared to the LNA-C probe alone.

### EXAMPLE 21

The assay method may also be used to detect hepatitis C virus. Using standard methods hepatitis C virus RNA was isolated from plasma that had a known amount of hepatitis C virus present. Using this isolated RNA reactions were conducted in liquid form in accordance with the methods disclosed in Example 19, above.

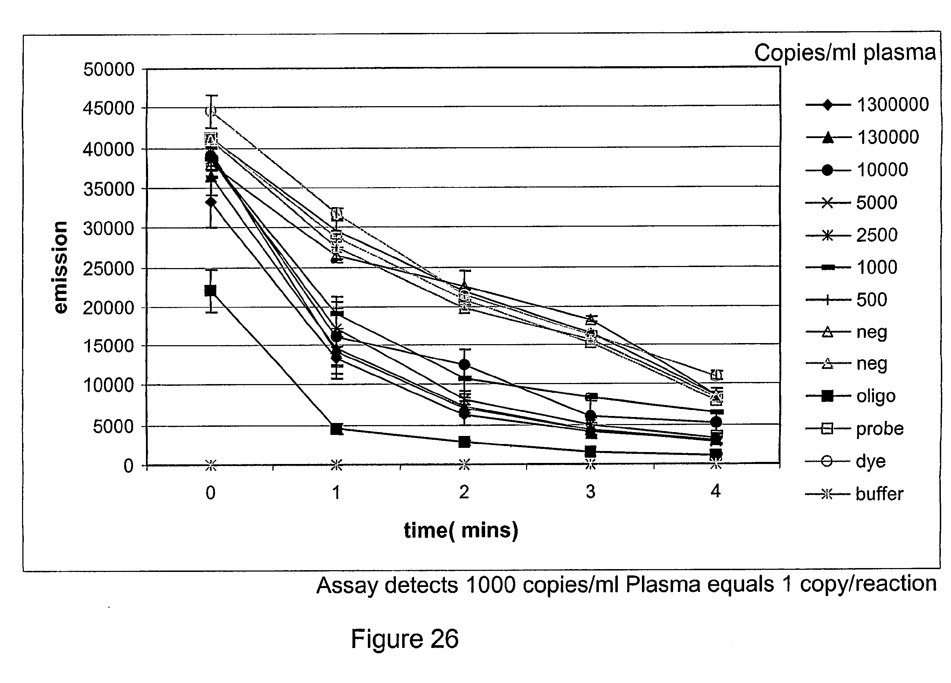

Figure 26 depicts the detection of hepatitis C virus probe using different quantities of plasma. The rate of change of optical property is different for viruses even in very low copy numbers.

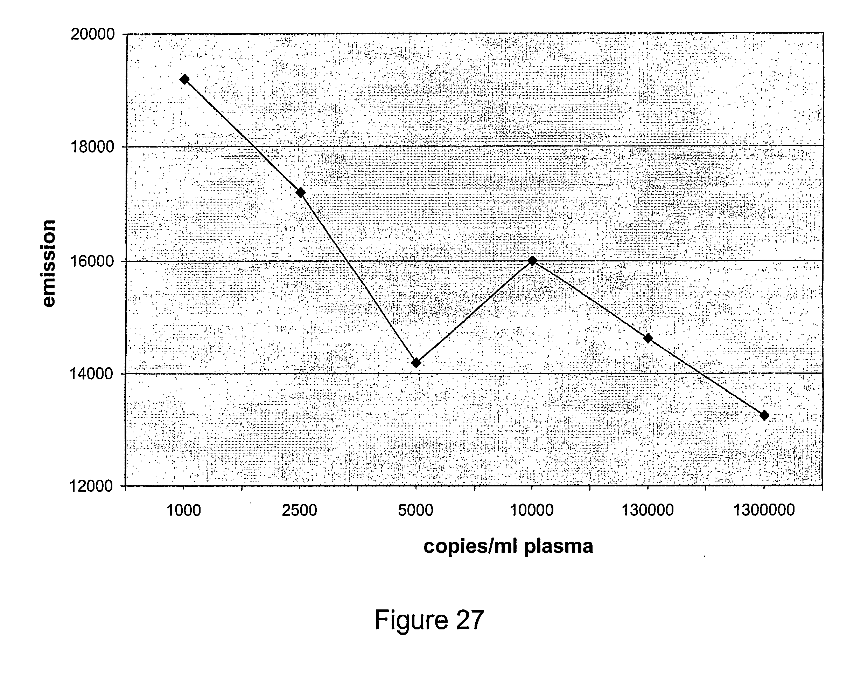

The copy number of hepatitis C virus can also be quantitated. Figure 27 depicts the emission of the dye obtained when different number of hepatitis C virus RNA were introduced into the assay. Lower numbers of virus RNA in the system generally have lower fluorescent emissions after one minute.

### EXAMPLE 22

This example demonstrates that the methods may be used to identify and/or quantify a target polynucleotide in bacteria.

500µl of bacterium (either E. coli or Bacillus cereus) culture grown overnight in TSB (tryptic soy broth) was pelleted by spinning for 5 minutes at 6000rpm then resuspended in 500µl1 of phosphate buffer then set at room temperature for 5 minutes. After this the PNA (either 5'bio-oo-gatagtgggattgtgcgt (SEQ ID NO: 16) for the 16S sequence or 5' Bio-OO-TGAGTGTGTGGCTTTCG 3' (SEQ ID NO: 19) for the non-specific HCV negative control sequence) and dye was added to the system, exposed to light stimulus and readings taken.

The following table shows the test conditions for reaction in microwell with a 50µl reaction volume.

| | P/O test | Probe only | Dye only | Bacteria test | Bacteria dye |
|---|---|---|---|---|---|
| PNA | 5µl | 5µl | | 5µl | |
| Oligo | 5µl | | | | |
| Bacterial culture | | | | 43 µl | 43µl |
| Dye | 2µl | 2µl | 2µl | 2µl | 2µl |
| Phosphate buffer | 38µl | 43µl | 48µl | | 5µl |

Emission data was collected using a Genios spectrophotometer at an excitation wavelength of 535 nm and an emission wavelength of 590nm. After an initial zero minute read, the samples were exposed to light stimulus from the Aurora 50/50 light for 60 seconds. The fluorescence was measured every 60 seconds for 10 minutes.

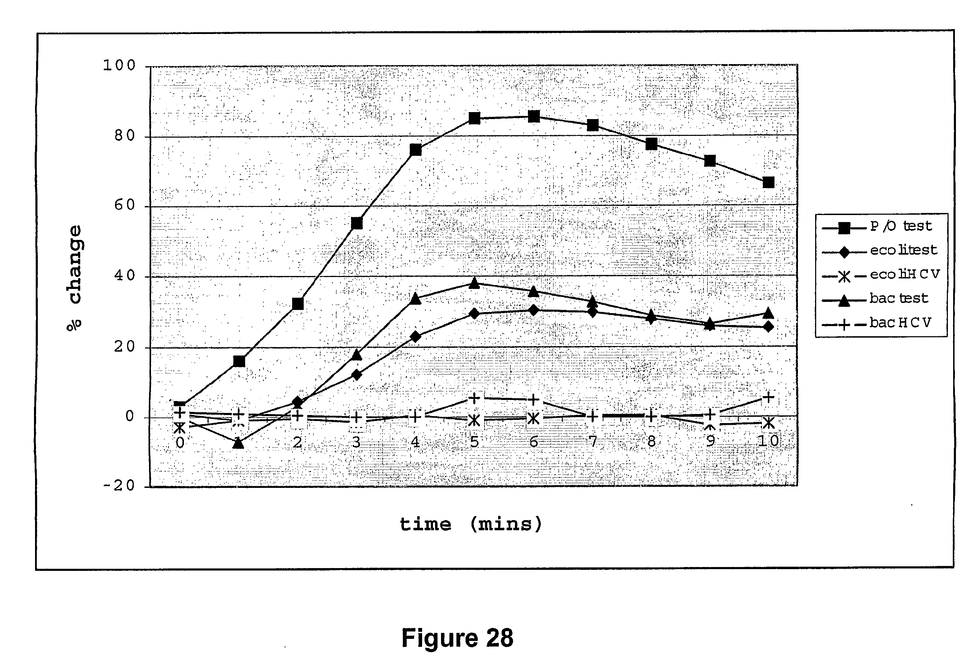

Figure 28 depicts the rate of change in the fluorescence compared to the absence of a nucleic acid analog/polynucleotide hybrid for each sample. The sample of target E. coli or B. cereus, depicted by (x, and +, respectively), was detected by the 16S PNA probe in the assay, but target E. coli or B. cereus, depicted by (◊ and ▲, respectively), was not detected by the viral HCV PNA probe in the assay. The (black square) shows the positive control with oligo to the 16S sequence as the target polynucleotide.

### EXAMPLE 23

Oligonucleotides (5' tgtgaacgca 3' and 5'tgcgttcaca 3') were mixed in annealing buffer (10mM Tris, pH 7.5 - 8.0, 50mum NaCl, 1mM EDTA) to 10mM and heated at 95C for 10 minutes in a thermal cycler. After heating the oligo mixture was allowed to cool at room temperature for 1 hour. One microliter of this mix was used per reaction.

PNAs N'Bio-OO-gatagtgggattgtgcgt, C(1)' and N' tcacatcaatccact-lys, C',(2) were used mixed of individually at 10mM in reaction buffer (5mMPO4 + 0.05% Tween). One microliter of each mix was used per reaction.

10mM, 3-3'- diethylthiacarbocyanine dye stock was diluted to 4mM in reaction buffer. One microliter of this mix was used per reaction.

### REFERENCES

1. Chandler, D.P., J.R. Stults., K.K. Anderson, S. Cebula, B.L. Schuck., and F.J. Brockman. 2000. Affinity capture and recovery of DNA at femtomolar concentrations with peptide nucleic acid probes. Anal. Biochem. 283:241-249.

2. Das, R., Cummings, C., Mendis, C., Neill, R., Ludwig, G., Hoover, D., Paranavitana, C., Yang, D. H. C., Lindler, L., Huang, X., Henchal, E., and Jett, M. (2001). Global Gene Analysis of Various Biological Threat and Infectious Agents Using PBMC: Implications for Therapy and Rapid Diagnostics. In: Dianne McQuestion (ed) Proceedings, Chemical and Biological Defense Symposium.

3. Demidov, V. V., Potaman, V. N., Frank-Kamenetskii, M. D., Egholm, M., Buchard, O., Sonnichsen, S. H., and Nielsen, P. E. (1994). Stability of peptide nucleic acids in human serum and cellular extracts. Biochem Pharmacol 48, 1310-3.

4. Haaima, G., Lohse, A., Buchardt, O. and Nielsen, P. E. (1996). Peptide Nucleic Acids (PNA) containing thymine monomers derived from chiral amino acids. Hybridization and solubility properties of d-lysine PNA. Ang Chem 35, 1939-1941.

5. Jett, M., Das, R., Cummings, C., Mendis, C., Neill, R., Hoover, D., Lindler, L., Paranavitana, C., Huang, X., Ludwig, G., Henchal, E., and Yang, D.H.C. (2001). Identification Of Changes In Gene Expression Induced By Toxic Agents: Implications for Therapy And Rapid Diagnosis. In: Proceedings of NATO Conference: Operational Issues in Chemical and Biological Defense Human Factors in Medicine Panel. Wade (ed).

6. Mikheikin, A. L., Zhuze, A. L., and Zasedatelev, A. S. (2000). Binding of symmetrical cyanine dyes into the DNA minor groove. J Biomol Struct Dyn 18, 59-72.

7. Nielsen, P. E., Egholm, M., Berg, R. H., and Buchardt, O. (1991). Sequence-selective recognition of DNA by strand displacement with a thymine-substituted polyamide. Science 254, 1497-500.

8. Nielsen, P. E. (2001). Peptide nucleic acid: a versatile tool in genetic diagnostics and molecular biology. Curr Opin Biotechnol 12, 16-20.

9. Ray, A., and Norden, B. (2000). Peptide nucleic acid (PNA): its medical and biotechnical applications and promise for the future. Faseb J 14, 1041-60.

10. Stefano, K. and Hyldig-Nielsen, J. J. (1997). Diagnostic Applications of PNA oligomers. In: Diagnostic Gene Detection and Quantification Technologies Minden (ed). pp. 19-39.

11. Wang, J., Palecek, E., Nielsen, P. E., Rivas, G., Cai, X., Shiraishi, H., Dontha, N., Luo, D., and Farias, P. A. M. (1996). Peptide Nucleic Acid Probes for Sequence-Specific DNA Biosensors. J Am Chem Soc 118, 7667-70.

12. Wilhelmsson, L. M., Norden, B., Mukherjee, K., Dulay, M. T., and Zare, R. N. (2002). Genetic screening using the color change of a PNA-DNA hybrid-binding cyanine dye. Nucleic Acids Res 30, E3.

13. Institute of Food Technologists (2002). Emerging Microbiological Food Safety Issues: Implications for Control in the 21st Century.

14. Wagner, SJ. 2002. Virus inactivation in blood components by photoactive phenothiazine dyes. Transfus. Med. Rev. 16:61-6.

15. Kapuscinski, J. 1995. DAPI: a DNA-specific fluorescent probe. Biotech. Histochem. 70:220-33.

16. Carreon, JR., Mahon, KP jr., Kelley, SO. 2004. Thiazole orange-peptide conjugates: sensitivity of DNA binding to chemical structure. Org. Lett. 6:517-9.

17. Morozkin, ES., Laldonov, PP., Rykova, EY., Vlassov, VV. 2003. Fluorometric quantification of RNA and DNA in solutions containing both nucleic acids. Anal. Biochem. 322:48-50.

18. Eriksson, M., Karlson, HJ., Westman, G., Akerman, B. 2003. Groove-binding unsymmetrical cyanine dyes for staining of DNA: dissociation rates in free solution and electrophoresis gels. Nucleic Acids Res. 31:6235-42.

19. Karlsson, HJ., Eriksson, M., Perzon, E., Akerman, B., Lincoln, P., Westman, G. 2003. Groove-binding unsymmetrical cyanine dyes for staining of DNA: syntheses and characterization of the DNA-binding. Nucleic Acids Res. 31:6227-34.

20. Schaberle, FA., Kuz'min, VA., Borissevitch, IE. 2003. Spectroscopic studies of the interaction of bichromophoric cyanine dyes with DNA. Effect of ionic strength. Biochim. Biophys. Acta. 1621:183-91.

21. Maiti, S., Chaudhury, NK., Chowdhury, S. 2003. Hoechst 33258 binds to G-quadruplex in the promoter region of human c-myc. Biochem. Biophys. Res. Commun. 310:505-12.

22. Tolun, G., Myers, RS. 2003. A real-time Dnase assay (ReDA) based on PicoGreen fluorescence. Nucleic Acids Res. 31:e111.

23. Sovenyhazy, KM., Bordelon JA., Petty, JT. 2003. Spectroscopic studies of the multiple binding modes of a trimethine-bridged cyanine dye with DNA. Nucleic Acids Res. 31:2561-9.

24. Prento, P., Lyon, HO. 2003. Methyl green-pyronin Y staining of nucleic acids: studies on the effects of staining time, dye composition and diffusion rates. Biotech. Histochem. 78:27-33.

25. Adhikary, A., Buschmann, V., Muller, C., Sauer, M. 2003. Ensemble and single-molecule fluorescence spectroscopic study of the binding modes of the bis-benzimidazole derivative Hoechst 33258 with DNA. Nucleic Acids Res. 31:2178-86.

26. Johnson, IM., Kumar, SG., Malathi, R. 2003. De-intercalation of ethidium bromide and acridine orange by xanthine derivatives and their modulatory effect on anticancer agents: a study of DNA-directed toxicity enlightened by time correlated single photon counting. J. Biomol. Struct. Dyn. 20:677-86.

27. Wang, Z., Zhang, Z., Liu, D., Dong, S. 2003. A temperature-dependent interaction of neutral red with calf thymus DNA. Spectrochim. Acta. A Mol. Biomol. Spectrosc. 59:949-56.

28. Karlsson, HJ., Lincoln, P., Westman, G. 2003. Synthesis and DNA binding studies of a new asymmetric cyanine dye binding in the minor groove of [poly(dA-dT)]2. Bioorg. Med. Chem. 11:1035-40.

29. Puckowska, A., Bielawski, K., Bielawska, A., Midura-Nowaczek, K. 2004. Aromatic analogs of DNA minor groove binders-synthesis and biological evaluation. Eur. J. Med. Chem. 39:99-105.

30. Reddy, PA., Santra, BK., Nethaji, M., Chakravarly, AR. 2004. Metal-assisted light-induced DNA cleavage activity of 2-(methylthio)phenylsalicylaldimine Schiff base copper(II) complexes having planar heterocyclic bases. J. Inorg. Biochem. 98:377-86.

31. Tanious, FA., Hamelberg, D., Bailly, C., Czarny, A., Boykin, DW., Wilson, WD. 2004. DNA sequence dependent monomer-dimer binding modulation of asymmetric benzimidazole derivatives. J. Am. Chem. Soc. 126:143-53.

32. Ma, DL., Che, CM. 2003. A bifunctional platinum(II) complex capable of intercalation and hydrogen-bonding interactions with DNA: binding studies and cytotoxicity. Chemistry. 9:6133-44.

33. Wakelin, LP., Bu, X., Eleftheriou, A., Parmar, A., Hayek, C., Stewart, BW. 2003. Bisintercalating threading diacridines: relationships between DNA binding, cytotoxicity, and cell cycle arrest. J. Med. Chem. 46:5790-802.

34. Tarasov, SG., Casas-Finet, JR., Cholody, WN., Kosakowska-Cholody, T., Gryczynski, ZK., Michejda, CJ. 2003. Bisimidazoacridones: 2. Steady-state and time-resolved fluorescence studies of their diverse interactions with DNA. Photochem. Photobiol. 78:313-22.

35. Tawar, U., jain, AK., Chandra, R., Singh, Y., Dwarakanath, BS., Chaudhury, NK., Good, L., Tandon, V. 2003. Minor groove binding DNA ligands with expanded A/T sequence length recognition, selective binding to bent DNA regions and enhanced fluorecent properties. Biochemistry. 18:13339-46.

36. Boutorine, AS., Ryabinin, VA., Novopashima, DS., Venyaminova, AG., Helene, C., Sinyakov, AS. 2003. Stabilization of DNA double and triple helices by conjugation of minor groove binders to oligonucleotides. Nucleosides Nucleotides Nucleic Acids. 22:1267-72.

37. Novopashima, D., Sinyakov, A., Ryabinin, V., Venyaminova, A., Boutorine, A., 2003. Conjugates of oligo(2'-O-methylribonucleotides) with minor groove binders as new sequence-specific agents recognizing both grooves of double-stranded DNA. Nucleosides Nucleotides Nucleic Acids. 22:1179-82.

38. Dhar, S., Senapati, D., Das, PK., Chattopadhyay, P., Nethaji, M., Chakravarty, AR. 2003. Ternary copper complexes for photocleavage of DNA by red light: direct evidence for sulfur-to-copper charge transfer and d-d band involvement. J. Am. Chem. Soc. 125:12118-24.

39. Hiraku, Y., Oikawa, S., Kawanishi, S. 2002. Distamycin A, a minor groove binder, changes enediyne-induced DNA cleavage sites and enhances apoptosis. Nucleic Acids Res. Suppl. 2002:95-6.

40. Dervan, PB., Edelson, BS. 2003. Recognition of the DNA minor groove by pyrrole-imidazole polyamides. Curr. Opin. Struct. Biol. 13:284-99.

41. Reddy, PM., Jindra, PT., Satz, AL., Bruice, TC. 2003. Sequence selective recognition in the minor groove of dsDNA by pyrrole, imidazole-substituted bis-benzimidazole conjugates. J. Am. Chem. Soc. 125:7843-8.

42. Briehn, CA., Weyermann, P., Dervan, PB. 2003. Alternative heterocycles for DNA recognition: the benzimidazole/imidazole pair. Chemistry. 9:2110-22.

43. Renneberg, D., Dervan, PB. 2003. Imidiazopyridine/Pyrrole and hydroxybenzimidazole/pyrrole pairs for DNA minor groove recognition. J. Am. Chem. Soc. 125:5707-16.

44. Kuwabara, T., Noda, T., Ohtake, H., Ohtake, T., Toyama, S., Ikariyama, Y. Classification of DNA-binding mode of antitumor and antiviral agents by the electrochemiluminescence of ruthenium complex. Anal. Biochem. 314:30-7.

45. Ouameur, AA., Nafisi, Sh., Mohajerani, N., Tajmir-Riahi, HA. 2003. Thallium-DNA complexes in aqueous solution. Major or minor groove binding. J. Biomol. Struct. Dyn. 20:561-5.

46. Carrasco, C., Helissey, P., Haroun, M., Baldeyrou, B., Lansiaux, A., Colson, P., Houssier, C., Giorgi-Renault, S., Bailly, C. 2003. Design of a composite ethidium-netropsin-anilinoacridine molecule for DNA recognition. Chembiochem. 3:50-61.

47. Nguyen, B., Lee, MP., Hamelberg, D., Joubert, A., Bailly, C., Brun, R., Neidle, S., Wilson, WD. J. Am. Chem. Soc. 124:13680-1.

hh) Barcelo, F., Capo, D., Portugal, J. 2002. Thermodynamic characterization of the multivalent binding of chartreusin to DNA. Nucleic Acids Res. 30:4567-73.

48. Silverman, AP., Bu, W., Cohen, SM., Lippard, SJ. 2002. 2.4-A crystal structure of the asymmetric platinum complex [Pt(ammine)(cyclohexylamine)]2+ bound to a dodecamer DNA duplex. J. Biol. Chem. 277:49743-9.

49. Viola, G., Dall'Acqua, F., Gabellini, N., Moro, S., Vedaldi, D., Ihmels, H. 2002. Indolo[2.3-b]-quinolizinium bromide: an efficient intercalator with DNA-photodamaging properties. Chembiochem. 3:550-8.

50. Anthoney, DA., Twelves, CJ. 2001. DNA: still a target worth aiming at? A review of new DNA-interactive agents. Am. J. Pharmacogenomics. 1:67-81.

51. Shim, YH., Arimondo, PB., Laigle, A., Garbesi, A., Lavielle, S. 2004. Relative DNA binding affinity of helix 3 homeodomain analogs, major groove binders, can be rapidly screened by displacement of prebound ethidium bromide. A comparative study. Org. Biomol. Chem. 2:915-21.

52. Varadarajan, S., Shah, D., Dande, P., Settles, S., Chen, FX., Fronza, G., Gold, B. 2003. DNA damage and cytotoxicity induced by minor groove binding methyl sulfonate esters. Biochemistry. 42:14318-27.

53. Guelev, V., Lee, J., Ward, J., Sorey, S., Hoffman, DW., Iverson, BL. 2001. Peptide bis-intercalator binds DNA via threading mode with sequence specific contacts in the major groove. Chem. Biol. 8:415-25.

54. Huang, X., Suleman, A., Skibo, EB. 2000. Rational design of pyrrolo. Bioorg. Chem. 28:324-37.

55. Proudfoot, EM., Mackay, JP., Karuso, P. 2001. Probing site specificity of DNA binding metallointercalators by NMR spectroscopy and molecular modeling. Biochemistry. 40:4867-78.

56. Kondo, S., Kinjo, T., Yano, Y. 2004. Synthesis of a novel intercalator based on 2,2'-binaphthalene bearing dimethylammonium groups. Bioorg. Med. Chem. Lett. 14:1641-3.

57. Nielsen, CB., Petersen, M., Pedersen, EB., Hansen, PE., Christensen, UB. 2004. NMR structure determination of a modified DNA oligonucleotide containing a new intercalating nucleic Acid. Bioconjug. Chem. 15:260-9.

58. Liu, F., Wang, K., Bai, G., Zhang, Y., Gao, L. 2004. The pH-Induced Emission Switching and Interesting DNA-Binding Properties of a Novel Dinuclear Ruthenium(II) Complex. Inorg. Chem. 43:1799-806.

59. Biver, T., Secco, F., Tine, MR., Venturini, M. 2004. Kinetics and equilibria for the formation of a new DNA metal-inetercalator: the cyclic polyamine Neotrien/copper(II) complex. J. Inorg. Biochem. 98:33-40.

60. Lauretti, F., Lucas de Melo, F., Benati, FJ., de Melto Volotao, E., Santos, N., Linhares, RE., Nozawa, C. 2003. Use of acridine orange staining for the detection of rotavirus RNA in polyacrylamide gels. J. Virol. Methods. 114:29-35.

61. Wong, EL., Gooding, JJ. 2003. Electronic detection of target nucleic acids by a 2,6-disulfonic acid anthraquinone intercalator. Anal. Chem. 75:3845-52.

62. Luedtke, NW., Liu, Q., Tor, Y. 2003. RNA-ligand interactions: affinity and specificity of aminoglycoside dimers and acridine conjugates to the HIV-1 Rev response element. Biochemistry. 42:11391-403.

63. Yamana, K., Kawakami, N., Ohtsuka, T., Sugie, Y., Nakano, H., Saito, I.2003. Electrochemical detection of single-base mismatches in DNA by a redox-active intercalator conjugated oligonucleotide. Nucleic Acids Res. Suppl. 2003:89-90.

64. Takanaka, S., Ohtuka, K., Miyahara, H., Nojima, T., Takagi, M. 2002. An anthracene derivative carrying ferrocenyl moieties at its 9 and 10 positions as a new electrochemically active threading intercalator. Nucleic Acids Res. Suppl. 2002:291-2.

65. Xiao, Y., Kharitonov, AB., Patolsky, F., Weizmann, Y., Willner, I. 2003. Electrocatalytic intercalator-induced winding of double-stranded DNA with polyaniline. Chem. Commun. (Camb). 7:1540-1.

66. Baruah, H., Bierbach, U. 2003. Unusual intercalation of acridin-9-ylthiourea into the 5'GA/TC DNA base step from the minor groove: implications for the covalent DNA adduct profile of a novel platinum-intercalator conjugate. Nucleic Acids Res. 31:4138-46.

67. Nojima, T., Kondoh, Y., Takenaka, S., Ichilhara, T., Takagi, M., Tashiro, H., Matsumoto, K. 2001. Detection of DNA hybridization by use of a lanthanide fluorescent intercalator that specifically binds to double stranded DNA. Nucleic Acids Res. Suppl. 2001:105-6.

68. Wang, S., Peng, T., Yang, CF. 2003. Electrochemical determination of interaction parameters for DNA and mitoxantrone in an irreversible redox process. Biophys. Chem. 104:239-48.

69. Guittat, L., Alberti, P., Rosu, F., Van Miert, S., Thetiot, E., Pieters, L., Gabelica, V., De Pauw, E., Ottaviani, A., Riou, JF., Mergny, JL. 2003. Interactions of cryptolepine and neocryptolepine with unusual DNA structures. Biochimie. 85:535-47.

70. Barry, CG., Turney, EC., Day, CS., Saluta, G., Kucera, GL., Bierbach, U. 2002. Thermally inert metal ammines as light-inducible DNA-targeted agents. Synthesis, photochemistry, and photobiology of a prototypical rhodium(III)-intercalator conjugate. Inorg. Chem. 41:7159-69.

71. Christensen, UB., Pedersen, EB. 2002. Intercalating nucleic acids containing insertions of 1-O-(1-pyrenylmethyl)glycerol: stabilization of dsDNA and discrimination of DNA over RNA. Nucleic Acids Res. 30:4918-25.

72. Patolsky, F., Katz, E., Willner, I. 2002. Amplified DNA detection by electrogenerated biochemiluminescence and by the catalyzed precipitation of an insoluble product on electrodes in the presence of the doxorubicin intercalator. Angrew. Chem. Int. Ed. Engl. 41:3398-402.

73. Woods, CR., Ishii, T., Boger, DL. 2002. Synthesis and DNA binding properties of iminodiacetic acid-linked polyamides: characterization of cooperative extended 2:1 side-by-side parallel binding. J. Am. Chem. Soc. 124:10676-82.

74. Woods, CR., Faucher, N., Eschgfaller, B., Bair, KW., Boger, DL. 2002. Synthesis and DNA binding properties of saturated distamycin analogs. Bioorg. Med. Chem. Lett. 12:2647-50.

75. Brana, MF., Dominguez, G., Saez, B., Romerdahl, C., Robinson, S., Barlozzari, T. 2002. Synthesis and antitumor activity of new dendritic polyamines-(imide-DNA-intercalator) conjugates: potent Lck inhibitors. Eur. J. Med. Chem. 37:541-51.

76. Onfelt, B., Gostring, L., Lincoln, P., Norden, B., Onfelt, A. 2002. Cell studies of the DNA bis-iintercalator Delta-Delta [mu-C4 (cpdppz)(2)-(phen)Ru(2)](4+): toxic effects and properties as a light emitting DNA probe in V79 Chinese hamster cells. Mutagenesis. 17:317-20.

77. Berge, T., Jenkins, NS., Hopkirk, RB., Waring, MJ., Edwardson, JM., Henderson, RM. 2002. Structural perturbations in DNA caused by bis-intercalation of ditercalinium visualized by atomic force microscopy. Nucleic Acids Res. 30:2980-6.

78. Arabzadeh, A., Bathaie, SZ, Farsam, H., Amanlou, M., Saboury, AA., Shockravi, A., Moosavi-Movahedi, AA. 2002. Studies on mechanism of 8-methoxypsoralen-DNA interaction in the dark. Int. J. Pharm. 237:47-55.

79. Reha, D., Kabekac, M., Ryjacek, F., Sponer, J., Sponer, JE., Elstner, M., Suhai, S., Hobza, P. 2002. Intercalators. 1. Nature of stacking interactions between intercalators (ethidium, daunomycin, ellipticine, and 4',6-diaminide-2-phenylindole) and DNA base pairs. Ab initio quantum chemical, density functional theory, and empirical potencial study. J. Am. Chem. Soc. 124:3366-76.

80. Guelev, V., Sorey, S., Hoffman, Dw., Iverson, BL. 2002. Changing DNA grooves-a 1,4,5,8-naphthalene tetracarboxylic diimide bis-intercalator with the linker (beta-Ala)(3)-Lys in the minor groove. J. Am. Chem. Soc. 124:2864-5.

81. Lisgarten, JN., Coll, M., Portugal, J., Wright, CW., Aymami, J. 2002. The antimalarial and cytotoxic drug cryptolepine intercalates into DNA at cytosine-cytosine sites. Nat. Struct. Biol. 9:57-60.

82. Ferry, DM., Van Zijl, PL., Wilson, WR. 2001. Sensitive liquid chromatographic assay for the basic DNA intercalator (N, N-dimethylaminoethyl)-9-amino-5-methylacridine-4-

83. carboxamide and its nitroarylmethyl quaternary prodrugs in biological samples. J. Chromatogr. B. Biomed. Sci. Appl. 763:149-56.

84. Tok, JB., Fenker, J. 2001. Novel synthesis and RNA-binding properties of aminoglycoside dimers conjugated via a naphthalene diimide-based intercalator. Bioorg. Med. Chem. Lett. 11:2987-91.

85. Dienes, Z., Vogel, P. 1996. Asymetric Synthesis and DNA Intercalation of (-)-6-[[(Aminoalkyl)oxy]methyl]-4-demethoxy-6,7-dideoxydaunomycinones(I)(,). J. Org. Chem. 61:6958-6970.

86. Papadopoulou, MV., Ji, M., Rao, MK., Bloomer, WD. 2000. 4-[3-(2-Nitro-1-imidazolyl)propylamino-7-chloroquinoline hydrochloride (NLCQ-1), a novel bioreductive agent as radiosensitizer in vitro and in vivo: comparison with tirapazamine. Oncol. Res. 12:325-33.

87. Boger, DL, Tse, WC. 2001. Thiazole orange as the fluorescent intercalator in a high resolution fid assay for determining DNA binding affininty and sequence selectivity of small molecules. Bioorg. Med. Chem. 9:2511-8.

88. Gianolio, DA., McLaughlin, LW. 2001. Tethered naphthalene diimide intercalators enhance DNA triplex stability. Bioorg. Med. Chem. 9:2329-34.

89. Onfelt, B., Lincoln, P., Norden, B. 2001. Enantioselective DNA threading dynamics by phenazine-linked. J. Am. Chem. Soc. 123:3630-7.

90. Wong, M., Kong, S., Dragowska, WH., Bally, MB. 2001. Oxazole yellow homodimer YOYO-1-labeled DNA: a fluorescent complex that can be used to asses structural changes in DNA following formation and cellular delivery of cationic lipid DNA complexes. Biochim. Biophys. Acta. 1527:61-72.

91. Hicks, KO., Pruijn, FB., Baguley, BC., Wilson, WR. 2001. Extravascular transport of the DNA intercalator and topoisomerase poison N-[2-(Dimethylamino)ethyl]acridine-4-carboxamide (DACA): diffusion and metabolism in multicellular layers of tumor cells. J. Pharmacol. Exp. Ther. 297:1088-98.

92. Kisko, JL, Barton, JK. 2000. Recognition of DNA base pair mismatches by a cyclometalated RH(III) intercalator. Inorg. Chem. 39:4942-9.

93. Dees, EC., Whitfield, LR., Grove, WR., Rummel, S., Grochow, LB., Donehower, RC. 2000. A phase I and pharmacologic evaluation of the DNA intercalator CI-958 in patients with advanced solid tumors. Clin. Cancer Res. 6:3885-94.

94. Pelley, R., Ganapathi, R., Wood, L., Rybicki, L., McLain, D., Budd, GT., Peereboom, D., Olencki, T., Bukowski, RM. 2000. A phase II pharmacodynamic study of pyrazoloacridine in patients with metastatic colorectal cancer. Cancer Chemother. Pharmacol. 46:251-4.

95. Perrin, LC., Prenzler, PD., Cullinane, C., Phillips, DR., Denny, WA., McFadyen, WD. 2000. DNA targeted platinum complexes: synthesis, cytotoxicity and DNA interactions of cis-dichloroplatinum(II) complexes tethered to phenazine-1-carboxamides. J. Inorg. Biochem. 81:111-7.

96. Mazerski, J., Muchewicz, K. 2000. The intercalation of imidazoacridinones into DNA induces conformational changes in their side chain. Acta. Biochim. Pol. 47:65-78.

97. Sajewicz, W., Dlugosz, A. 2000. Cytotoxicity of some potential DNA intercalators (carbazole, acridine and anthracene derivatives) evaluated through neutrophil chemiluminescence. J. Appl. Toxicol. 20:305-12.

98. Osiadacz, J., Majka, J., Czarneski, K., Pecynska-Czoch, W., Zakrzewska-Czerwinska, J., Kaczmarek, L., Sokalski, WA. 2000. Sequence-selectivity of 5,11-dimethyl-5H-indolo[2,3-b]quinoline binding to DNA. Footprinting and molecular modeling studies. Bioorg. Med. Chem. 8:937-43.

99. Kirschstein, O., Sip, M., Kitter, L. 2000. Quantitative and sequence-specific analysis of DNA-ligand interaction by means of fluorescent intercalator probes. J. Mol. Recognit. 13:157-63.

100. Numez, ME., Noyes, KT., Gianolio, DA., McLaughlin, LW., Barton, JK. 2000. Long-range guanine oxidation in DNA restriction fragments by a triplex-directed naphathalene diimide intercalator. Biochemistry. 39:6190-9.

101. Jackson, BA., Barton, JK. 2000. Recognition of base mismatches in DNA by 5,6-chrysenequinone diimine complexes of rhodium(III): a proposed mechanism for preferential binding in destabilized regions of the double helix. Biochemistry. 39:6176-82.

102. Chen, QY., Li, DH., Zhao, Y., Yang, HH., Zhu, QZ., Xu, JG. 1999. Interaction of a novel red-region fluorescent probe, Nile blue, with DNA and its application to nucleic acids assay. Analyst. 124:901-6.

103. Dassonneville, L., Wattez, N., Mahieu, C., Colson, P., Houssier, C., Frederich, M., Tits, M., Angenot, L., Bailly, C. 1999. The plant alkaloid usambarensine intercalates into DNA and induces apoptosis in human HL60 leukemia cells. Anticancer Res. 19:5245-50.

104. Toshima, K., Takano, R., Maeda, Y., Suzuki, M., Asai, A., Matsumura, S. 1999. 2-Phenylquinoline-Carbohydrate Hybrids: Molecular Design, Chemical Synthesis, and Evaluation of a New Family of Light-Activatable DNA-Cleaving Agents. Angew. Chem. Int. Ed. Engl. 38:3733-3735.

105. Yang, X., Liu, WH., Jin, WJ., Shen, GL., Yu, RQ. 1999. DNA binding studies of a solvatochromic fluorescence probe 3-methoxybenzanthrone. Spectochim. Acta. A Mol. Biomol. Spectrosc. 55A:2719-27.

106. Mueller, SO., Stopper, H. 1999. Characterization of the genotoxicity of anthraquinones in mammalian cells. Biochim. Biophys. Acta. 1428:406-14.

107. Inoue, T., Sugiura, Y., Saitoh, J., Ishiguro, T., Otsuka, M. 1999. Flurorescence property of oxazole yellow-linked oligonucleotide. Triple helix formation and photocleavage of double-stranded DNA in the presence of spermine. Bioorg. Med. Chem. 7:1207-11.

108. Dempcy, RO., Kutyavin, IV., Mills, AG., Lukhtanov, EA., Meyer, RB. 1999. Linkers designed to intercalate the double helix greatly facilitate DNA alkylation by triplex-forming oligonucleotides carrying a cyclopropapyroloindole reactive moiety. Nucleic Acids Res. 27:2931-7.

109. Keppler, M., Zegrocka, O., Strekowski, L., Fox, KR. 1999. DNA triple helix stabilization by a naphthylquinoline dimmer. FEBS Lett. 447:223-6.

110. Ostaszewki, R., Wilczynska, E., Wolszczak, M. 1998. The synthesis of a new type of anthracene DNA intercalator. Bioorg. Med. Chem. Lett. 8:2995-6.

111. Atwell, GJ., Fan, JY., Tan, K., Denny, WA. 1998. DNA-Directed alkylating agents.7. Synthesis, DNA interaction, and antitumor activity of bis(hydroxymethyl)- and bis(carbamate)-substituted pyrrolizines and imidazoles. J. Med. Chem. 41:4744-54.

112. Milano, MT., Hu, GG., Williams LD., Bernhard, WA. 1998. Migration of electrons and holes in crystalline d(CGATCG)-anthracycline complexes X-irradiated at 4K. Radiat. Re. 150:101-14.

113. Benson, SC., Mathies, RA., Glazer, AN. 1993. Heterodimeric DNA-binding dyes designed for energy transfer: stability and applications of the DNA complexes. Nucleic Acids Research. 21:5720-5726.

114. Benson, SC., Singh, P., Glazer, AN. 1993. Heterodimeric DNA-binding dyes designed for energy transfer: synthesis and spectroscopic properties. Nucleic Acids Research. 21:5727-5735.

115. Smith, JO., Olson, DA., Armitage, BA. 1999. Molecular recognition of PNA-containing hybrids: Spontaneous assembly of helical cyanine dye aggregates on PNA templates. J. Am. Chem. Soc. 121:2686-2695.

## Claims

1. A method of detecting the presence or amount of a target polynucleotide having a target nucleic acid sequence in a sample, comprising:
a) combining the sample, a nucleic acid analog that is complementary to a target nucleic acid sequence of said target polynucleotide, and a cyanine dye for which the rate of change in an optical property selected from color, absorbance and fluorescence when exposed to light stimulus is different in the presence and absence of a target polynucleotide/nucleic acid analog hybrid, thereby producing a mixture;
b) exposing the mixture to light stimulus;
c) observing the rate of change in the optical property of the dye, comparing the rate of change in said optical property of the dye to a reference value characteristic of the rate of change in said optical property of the dye in a similar mixture containing a known amount of a polynucleotide/nucleic acid analog hybrid, and determining whether exposing the mixture to light stimulus causes a difference in the rate of change, or a difference in the change at a set time, in the optical property of the dye, wherein a difference in the rate of change, or a difference in the change at a set time, in the optical property of the dye caused by exposing the mixture to light stimulus correlates with the presence or amount of the target polynucleotide in the sample.

2. The method of claim 1, wherein step c) comprises the steps:
(i) comparing the rate of change in said optical property of the dye in the mixture to a reference value characteristic of the rate of change in the optical property of the dye in a similar mixture containing a known amount of a polynucleotide/nucleic acid analog hybrid to determine a relative rate of change in the optical property; and
(ii) correlating the relative rate of change in the optical property of the dye in the mixture with the presence or amount of the specified target polynucleotide in a sample.

3. The method of claim 1 or claim 2, wherein the light stimulus is white light.

4. The method of claim 1 or claim 2, wherein the light stimulus is light of a specific wavelength, or range or wavelengths.

5. The method of claim 4, wherein the light stimulus is blue light.

6. The method of claim 4, wherein the light stimulus is green light.

7. The method of any preceding claim, wherein the nucleic acid analog is a peptide nucleic acid (PNA) or a locked nucleic acid (LNA).

8. The method of any preceding claim, wherein the target polynucleotide is DNA.

9. The method of any preceding claim, wherein the target polynucleotide is RNA.

10. The method of any preceding claim, wherein the nucleic acid analog includes a complementary region that is greater than 4 nucleic acid bases in length and less than 24 nucleic acid bases in length.

11. The method of any preceding claim, wherein the nucleic acid analog or the target polynucleotide is immobilized on a solid substrate.

12. The method of any preceding claim, wherein the cyanine dye has a higher rate of change in the optical property when exposed to light stimulus in the presence of nucleic acid analog/target polynucleotide hybrid than in the absence of a nucleic acid analog/target polynucleotide hybrid.

13. The method of any preceding claim, wherein the dye is a carbocyanine dye.

14. The method of any of claims 1-12, wherein the dye is a thiacyanine dye.

15. The method of any of claims 1-12, wherein the cyanine dye is selected from the group consisting of 3,3'-diethylthiacyanine iodide, 3,3'-diethylthiacarbocyanine iodide, 3,3'-diethylthiadicarbocyanine iodide, and 3,3'-diethylthiatricarbocyanine iodide.

16. The method of any of claims 1-12, wherein the cyanine dye is 3,3'-diethylthiacarbocyanine iodide.

17. The method of any preceding claim, wherein the optical property is color.

18. The method of any preceding claim, wherein the optical property of the dye is measured at a single time.

19. The method of any of claims 1 to 17, wherein the optical property of the dye is measured at multiple times.

20. The method of claim 2, wherein the relative rate of change in the optical property is approximate or qualitative.

21. The method of claim 2, wherein the relative rate of change in the optical property is quantitative.

22. The method of claim 2, wherein the reference value is calculated or inferred and not measured.

23. The method of claim 2, wherein the reference value is empirically determined.

24. A method for detecting a single nucleotide polymorphism (SNP), comprising:
detecting the presence or amount of a target polynucleotide according to the method of any of claims 1 to 23, wherein the target nucleic acid sequence is a sequence specific to an SNP, and wherein the presence or amount of said target polynucleotide identifies said SNP.

25. A method of detecting an organism in a sample, comprising:
detecting the presence or amount of a target polynucleotide in the sample according to the method of any of claims 1 to 23, wherein the target nucleic acid sequence is a sequence specific to the organism, and wherein the presence or amount of said target polynucleotide identifies the presence or amount of the organism.

## Patentansprüche

1. Verfahren zum Nachweisen der Gegenwart oder der Menge eines Zielpolynukleotids mit einer Nukleinsäuresequenz in einer Probe, umfassend:
a) Kombinieren der Probe, eines Nukleinsäureanalogons, das komplementär zu einer Zielnukleinsäuresequenz des Zielpolynukleotids ist, und eines Cyaninfarbstoffs, für den die Änderungsrate einer optischen Eigenschaft, ausgewählt aus Farbe, Absorbanz und Fluoreszenz, wenn er einem Lichtreiz ausgesetzt ist, in Gegenwart und Abwesenheit eines Zielpolynukleotids/Nukleinsäureanalogonhybrids verschieden ist, wodurch ein Gemisch produziert wird;
b) Aussetzen des Gemischs einem Lichtreiz;
c) Beobachten der Änderungsrate der optischen Eigenschaft des Farbstoffs, Vergleichen der Änderungsrate der optischen Eigenschaft des Farbstoffs mit einem Referenzwert, der für die Änderungsrate der optischen Eigenschaft des Farbstoffs in einem ähnlichen Gemisch charakteristisch ist, das eine bekannte Menge eines Polynukleotids/Nukleinsäureanalogonhybrids enthält, und Bestimmen, ob das Aussetzen des Gemischs an den Lichtreiz eine Differenz in der Änderungsrate, oder eine Differenz in der Änderung der optischen Eigenschaft des Farbstoffs zu einem festgesetzten Zeitpunkt herbeiführt,
wobei eine Differenz der Änderungsrate oder eine Differenz der Veränderung der optischen Eigenschaft des Farbstoffs zu einem festgesetzten Zeitpunkt, die durch das Aussetzen des Gemischs an den Lichtreiz herbeigeführt werden, mit der Gegenwart oder der Menge des Zielpolynukleotids in der Probe korreliert.

2. Verfahren nach Anspruch 1, wobei Schritt c) die folgenden Schritte umfasst:
(i) Vergleichen der Veränderungsrate der optischen Eigenschaft des Farbstoffs in dem Gemisch mit einem Referenzwert, der für die Änderungsrate der optischen Eigenschaft des Farbstoffs in einem ähnlichen Gemisch charakteristisch ist, das eine bekannte Menge eines Polynukleotids/Nukleinsäureanalogonhybrids enthält, um eine relative Änderungsrate der optischen Eigenschaft zu bestimmen; und
(ii) Korrelieren der relativen Änderungsrate der optischen Eigenschaft des Farbstoffs in dem Gemisch mit der Gegenwart oder der Menge des spezifizierten Zielpolynukleotids in einer Probe.

3. Verfahren nach Anspruch 1 oder Anspruch 2, wobei der Lichtreiz weißes Licht ist.

4. Verfahren nach Anspruch 1 oder Anspruch 2, wobei der Lichtreiz Licht mit einer spezifischen Wellenlänge oder einem Wellenlängenbereich ist.

5. Verfahren nach Anspruch 4, wobei der Lichtreiz blaues Licht ist.

6. Verfahren nach Anspruch 4, wobei der Lichtreiz grünes Licht ist.

7. Verfahren nach einem der vorhergehenden Ansprüche, wobei das Nukleinsäureanalogon eine Peptidnukleinsäure (PNA) oder eine verbrückte Nukleinsäure (LNA) ist.

8. Verfahren nach einem der vorhergehenden Ansprüche, wobei das Zielpolynukleotid DNA ist.

9. Verfahren nach einem der vorhergehenden Ansprüche, wobei das Zielpolynukleotid RNA ist.

10. Verfahren nach einem der vorhergehenden Ansprüche, wobei das Nukleinsäureanalogon eine komplementäre Region beinhaltet, deren Länge größer als 4 Nukleinsäurebasen und kleiner als 24 Nukleinsäurebasen ist.

11. Verfahren nach einem der vorhergehenden Ansprüche, wobei das Nukleinsäureanalogon oder das Zielpolynukleotid auf einem festen Substrat immobilisiert ist.

12. Verfahren nach einem der vorhergehenden Ansprüche, wobei der Farbstoff eine höhere Änderungsrate der optischen Eigenschaft hat, wenn er einem Lichtreiz in Gegenwart eines Nukleinsäureanalogons/Zielpolynukleotidhybrids ausgesetzt wird als in Abwesenheit eines Nukleinsäureanalogons/Zielpolynukleotidhybrids.

13. Verfahren nach einem der vorhergehenden Ansprüche, wobei der Farbstoff ein Carbocyaninfarbstoff ist.

14. Verfahren nach einem der Ansprüche 1 bis 12, wobei der Farbstoff ein Thiacyaninfarbstoff ist.

15. Verfahren nach einem der Ansprüche 1 bis 12, wobei der Cyaninfarbstoff ausgewählt ist aus der Gruppe bestehend aus 3,3'-Diethylthiacyaniniodid, 3,3'-Diethylthiacarbocyaniniodid, Diethylthiadicarbocyaniniodid und 3,3'- Diethylthiatricarbocyaniniodid.

16. Verfahren nach einem der Ansprüche 1 bis 12, wobei der Cyaninfarbstoff 3,3'-Diethylthiacarbocyaniniodid ist.

17. Verfahren nach einem der vorhergehenden Ansprüche, wobei die optische Eigenschaft Farbe ist.

18. Verfahren nach einem der vorhergehenden Ansprüche, wobei die optische Eigenschaft des Farbstoffs zu einer Zeit gemessen wird.

19. Verfahren nach einem der Ansprüche 1 bis 17, wobei die optische Eigenschaft des Farbstoffs zu mehreren Zeiten gemessen wird.

20. Verfahren nach Anspruch 2, wobei die relative Änderungsrate der optischen Eigenschaft approximativ oder qualitativ ist.

21. Verfahren nach Anspruch 2, wobei die relative Änderungsrate der optischen Eigenschaft qualitativ ist.

22. Verfahren nach Anspruch 2, wobei der Referenzwert berechnet oder abgeleitet und nicht gemessen wird.

23. Verfahren nach Anspruch 2, wobei der Referenzwert empirisch bestimmt wird.

24. Verfahren zum Nachweisen eines Einzelnukleotidpolymorphismus (SNP), umfassend:
Nachweisen der Gegenwart oder der Menge eines Zielpolynukleotids gemäß dem Verfahren nach Anspruch 1 bis 23, wobei die Zielnukleinsäuresequenz eine Sequenz ist, die spezifisch für einen SNP ist, und wobei die Gegenwart oder die Menge des Zielpolynukleotids den SNP identifiziert.

25. Verfahren zum Nachweisen eines Organismus in einer Probe, umfassend:
Nachweisen der Gegenwart oder der Menge eines Zielpolynukleotids in der Probe gemäß dem Verfahren nach Anspruch 1 bis 23, wobei die Zielnukleinsäuresequenz eine Sequenz ist, die spezifisch für einen Organismus ist, und wobei die Gegenwart oder die Menge des Zielpolynukleotids die Gegenwart oder die Menge des Organismus identifiziert.

## Revendications

1. Procédé de détection de la présence ou de la quantité d'un polynucléotide cible présentant une séquence d'acide nucléique cible dans un échantillon, comprenant les étapes suivantes :
a) combiner l'échantillon, un analogue d'acide nucléique qui est complémentaire d'une séquence cible d'acide nucléique dudit polynucléotide cible, et un colorant cyanine pour lequel la vitesse de modification d'une propriété optique, sélectionnée parmi la couleur, l'absorbance et la fluorescence, lorsqu'il est exposé à un stimulus lumineux, est différente en présence et en absence d'un hybride polynucléotide cible/analogue d'acide nucléique, produisant de ce fait un mélange ;
b) exposer le mélange à un stimulus lumineux ;
c) observer la vitesse de modification de la propriété optique du colorant, comparer la vitesse de modification de ladite propriété optique du colorant à une valeur de référence caractéristique de la vitesse de modification de ladite propriété optique du colorant dans un mélange similaire contenant une quantité connue d'un hybride de polynucléotide/analogue d'acide nucléique, et déterminer si l'exposition du mélange au stimulus lumineux provoque une différence de la vitesse de modification, ou une différence de modification à un moment donné, de la propriété optique du colorant,
dans lequel une différence dans la vitesse de modification ou une différence de modification à un moment donné de la propriété optique du colorant provoquée par l'exposition du mélange au stimulus lumineux est en corrélation avec la présence ou la quantité du polynucléotide cible dans l'échantillon.

2. Procédé selon la revendication 1, dans lequel l'étape c) comprend les étapes suivantes :
(i) comparer la vitesse de modification de ladite propriété optique du colorant dans le mélange à une valeur de référence caractéristique de la vitesse de modification de la propriété optique du colorant dans un mélange similaire contenant une quantité connue d'un hybride polynucléotide/analogue d'acide nucléique pour déterminer une vitesse relative de modification de la propriété optique ; et
(ii) mettre en corrélation la vitesse relative de la modification de la propriété optique du colorant dans le mélange avec la présence ou la quantité du polynucléotide cible spécifié dans un échantillon.

3. Procédé selon la revendication 1 ou la revendication 2, dans lequel le stimulus lumineux est de la lumière blanche.

4. Procédé selon la revendication 1 ou la revendication 2, dans lequel le stimulus lumineux est une lumière d'une longueur d'onde spécifique, ou d'une plage de longueurs d'ondes.

5. Procédé selon la revendication 4, dans lequel le stimulus lumineux est de la lumière bleue.

6. Procédé selon la revendication 4, dans lequel le stimulus lumineux est de la lumière verte.

7. Procédé selon l'une quelconque des revendications précédentes, dans lequel l'analogue d'acide nucléique est un acide nucléique peptidique (PNA) ou un acide nucléique verrouillé (LNA).

8. Procédé selon l'une quelconque des revendications précédentes, dans lequel le polynucléotide cible est de l'ADN.

9. Procédé selon l'une quelconque des revendications précédentes, dans lequel le polynucléotide cible est de l'ARN.

10. Procédé selon l'une quelconque des revendications précédentes, dans lequel l'analogue d'acide nucléique comprend une région complémentaire dont la longueur est supérieure à 4 bases d'acide nucléique et dont la longueur est inférieure à 24 bases d'acide nucléique.

11. Procédé selon l'une quelconque des revendications précédentes, dans lequel l'analogue d'acide nucléique ou le polynucléotide cible est immobilisé sur un substrat solide.

12. Procédé selon l'une quelconque des revendications précédentes, dans lequel le colorant cyanine a une vitesse de modification de la propriété optique lorsqu'il est exposé à un stimulus lumineux en présence d'un hybride d'analogue d'acide nucléique/polynucléotide cible supérieure à celle en absence d'un hybride d'analogue d'acide nucléique/polynucléotide cible.

13. Procédé selon l'une quelconque des revendications précédentes, dans lequel le colorant est un colorant carbocyanine.

14. Procédé selon l'une quelconque des revendications 1 à 12, dans lequel le colorant est un colorant thiacyanine.

15. Procédé selon l'une quelconque des revendications 1 à 12, dans lequel le colorant cyanine est sélectionné dans le groupe composé de l'iodure de 3,3'-diéthylthiacyanine, de l'iodure de 3,3'-diéthylthiacarbocyanine, de l'iodure de 3,3'-diéthylthiadicarbocyanine et de l'iodure de 3,3'-diéthylthiatricarbocyanine.

16. Procédé selon l'une quelconque des revendications 1 à 12, dans lequel le colorant cyanine est l'iodure de 3,3'-diéthylthiacarbocyanine.

17. Procédé selon l'une quelconque des revendications précédentes, dans lequel la propriété optique est la couleur.

18. Procédé selon l'une quelconque des revendications précédentes, dans lequel la propriété optique du colorant est mesurée à un moment unique.

19. Procédé selon l'une quelconque des revendications 1 à 17, dans lequel la propriété optique du colorant est mesurée à plusieurs moments.

20. Procédé selon la revendication 2, dans lequel la vitesse relative de modification de la propriété optique est approximative ou qualitative.

21. Procédé selon la revendication 2, dans lequel la vitesse relative de modification de la propriété optique est quantitative.

22. Procédé selon la revendication 2, dans lequel la valeur de référence est calculée ou supposée et non mesurée.

23. Procédé selon la revendication 2, dans lequel la valeur de référence est déterminée de manière empirique.

24. Procédé de détection d'un polymorphisme pour un nucléotide unique (SNP), comprenant les étapes suivantes :
détecter la présence ou la quantité d'un polynucléotide cible selon le procédé de l'une quelconque des revendications 1 à 23, où la séquence cible d'acide nucléique est une séquence spécifique d'un SNP, et où la présence ou la quantité dudit polynucléotide cible identifie ledit SNP.

25. Procédé de détection d'un organisme dans un échantillon, comprenant les étapes suivantes :
détecter la présence ou la quantité d'un polynucléotide cible dans l'échantillon selon le procédé de l'une quelconque des revendications 1 à 23, où la séquence cible d'acide nucléique est une séquence spécifique de l'organisme, et où la présence ou la quantité dudit polynucléotide cible identifie la présence ou la quantité de cet organisme.
